# EUROPEAN PATENT APPLICATION

(11) **EP 4 748 854 A2**
(43) Date of publication of application: **27.05.2026**
(21) Application number: 26154282.3
(22) Date of filing: 04.12.2023
(51) Int. Cl.: C07K 14/55

(54) **MUTANT IL-2 POLYPEPTIDES AND IL-2 PRODRUGS**

(30) Priority: 02.12.2022 US 202263429940 P
(62) Divisional of application: 23841128.4
(71) Applicant: AskGene Pharma, Inc., Camarillo, CA 93012 (US)
(72) Inventor: YU, Chunxiao, Camarillo 93012 (US); LU, Yuefeng, Camarillo 93012 (US); SHANEBECK, Kurt, Camarillo 93012 (US); RUIZ, Jeanine, Camarillo 93012 (US); TUMANUT, Christine, Camarillo 93012 (US); FAN, Xiaomin, Camarillo 93012 (US); SHI, Donghui, Camarillo 93012 (US); CHUANG, Jui Chang, Camarillo 93012 (US)
(74) Representative: Cohausz & Florack

(57) **Abstract**

Provided herein are IL-2 mutants, IL-2 prodrugs, and IL-2 antibody fusion molecules, as well as methods of using the same to modulate the immune system in a subject.

## Description

### CROSS REFERENCE TO RELATED APPLICATION

The present application claims priority from U.S. Provisional Application No. 63/429,940 filed on December 2, 2022, the contents of which are incorporated herein by reference in their entirety.

### SEQUENCE LISTING

The application contains a Sequence Listing which has been submitted electronically in .XML format. Said .XML copy, created on November 30, 2023, is named "025471.WO015.xml" and is 525,382 bytes in size. The Sequence Listing contained in this .XML file is part of the specification and is hereby incorporated by reference herein in its entirety.

### BACKGROUND OF THE INVENTION

Interleukin-2 (IL-2) plays a central role in lymphocyte generation, survival, and homeostasis. It has 133 amino acids and consists of four antiparallel, amphipathic alpha-helices that form a quaternary structure essential for its function (Smith, Science (1988) 240:1169-76; Bazan, Science (1992) 257:410-13).

IL-2 exerts its activities by binding to IL-2 receptors (IL-2R), which consist of up to three individual subunits. Association of the α (CD25 or Tac antigen), β (CD122), and γ (yc, common γ chain, or CD132) subunits results in a trimeric, high-affinity receptor for IL-2 (KD ~ 0.01 nM). Dimeric IL-2 receptor consisting of the β and γ subunits is termed intermediate-affinity IL-2R (KD ~ 1 nM). The α subunit alone forms the monomeric low affinity IL-2 receptor (KD ~ 10 nM). *See, e.g.,* Kim et al., Cytokine Growth Factor Rev. (2006) 17:349-66. Although the dimeric intermediate-affinity IL-2 receptor binds IL-2 with approximately 100-fold lower affinity than the trimeric high-affinity receptor, both the dimeric and trimeric IL-2 receptors can transmit signal upon IL-2 binding (Minami et al., Annu Rev Immunol. (1993) 11:245-68). Thus, it appears that the α subunit, while conferring high-affinity binding of the receptor to IL-2, is not essential for IL-2 signaling. However, the β and γ subunits are essential for IL-2 signaling (Krieg et al., Proc Natl Acad Sci. (2010) 107:11906-11). The trimeric IL-2 receptor is expressed by CD4+FoxP3+ regulatory T (Treg) cells. Treg cells consistently express the highest level of IL-2Rα (CD25) *in vivo* (Fontenot et al., Nature Immunol. (2005) 6:1142-51). The trimeric IL-2 receptor is also transiently induced on conventional activated T cells, whereas in the resting state these cells express only the dimeric IL-2 receptor.

Mutated versions of IL-2 have been developed to optimize treatment of cancer and autoimmune diseases. However, immunogenicity of the mutated IL-2 molecules is a potential risk for clinical development of the molecules. Hence, there is a need to develop IL-2 based therapeutics with decreased immunogenicity.

### SUMMARY OF THE INVENTION

The present disclosure provides mutant human IL-2 polypeptides comprising a mutation at position L36 (e.g., L36I) according to SEQ ID NO:1. In one aspect, the present disclosure provides a mutant human IL-2 polypeptide comprising an amino acid sequence that is at least 90% identical to SEQ ID NO:1 and an L36I mutation relative to SEQ ID NO:1. In some embodiments, the mutant IL-2 polypeptides comprise additional mutations as further described herein.

In some embodiments, the additional one or more IL-2 polypeptide mutations reduce the polypeptide's binding affinity for CD25. In some embodiments, the IL-2 polypeptide mutation is a C125A mutation, relative to SEQ ID NO:1. In some embodiments, the additional one or more mutations are at positions selected from T3, wherein the mutation is an N3A mutation; D20, wherein the mutation is a D20H, D20K, D20L, D20M, D20N, D20Q, D20R, D20S, D20V, or D20Y mutation; R38, wherein the mutation is R38A, R38K, or R38S; F42, wherein the mutation is F42A, F42G, F42I, F42S, F42T, F42Q, F42E, F42N, F42D, F42R, or F42K; Y45, wherein the mutation is Y45A, Y45G, Y45I, Y45S, Y45T, Y45Q, Y45E, Y45N, Y45D, Y45R, or Y45K; E62, wherein the mutation is E62L, E62A, or E62I; E68, wherein the mutation is E68V; L72, wherein the mutation is L72G; A73, wherein the mutation is A73T; N88, wherein the mutation is N88A, N88E, N88F, N88H, N88K, N88T, N88L, N88M, N88S, N88V, N88W, or N88Y; N90, wherein the mutation is N90T; V91, wherein the mutation is V91K, V91A, V91H, or V91R; I92; and Q126, wherein the mutation is Q126A, Q126D, Q126F, Q126G, Q126H, Q126I, Q126K, Q126L, Q126P, Q126S, Q126T, Q126W, or Q126Y (numbering according to SEQ ID NO:1).

In some embodiments, the mutant IL-2 polypeptide comprises an amino acid sequence selected from SEQ ID NOs:3 and 5-33, or an amino acid sequence at least 95% identical thereto. In other embodiments, the mutant IL-2 polypeptide comprises an amino acid sequence that is at least 95% identical to SEQ ID NO:2 or 4.

In another aspect, the present disclosure provides an anti-IL-2 antibody or an antigen-binding fragment thereof, comprising heavy chain CDR1-3 of SEQ ID NOs: 170-172, respectively, and light chain CDR1-3 of SEQ ID NOs: 173- 175, respectively. In some embodiments, an anti-IL-2 antibody or an antigen-binding fragment thereof herein comprises a light chain variable domain (V_{L}) comprising SEQ ID NO:190 or an amino acid sequence at least 95% identical thereto, and a heavy chain variable domain (V_{H}) comprising SEQ ID NO:191 or an amino acid sequence at least 95% identical thereto; a V_{L} comprising SEQ ID NO:192 or an amino acid sequence at least 95% identical thereto, and a V_{H} comprising SEQ ID NO:193 or an amino acid sequence at least 95% identical thereto; or a V_{L} comprising SEQ ID NO:194 or an amino acid sequence at least 95% identical thereto, and a V_{H} comprising SEQ ID NO: 195 or an amino acid sequence at least 95% identical thereto. In some embodiments, an anti-IL-2 antigen-binding fragment herein comprises an amino acid sequence selected from SEQ ID NOs:34, 35, 36, and 250-258, or an amino acid sequence at least 90% identical thereto. In some embodiments, the antibody or antigen-binding fragment, when bound to an IL-2 polypeptide, reduces the IL-2 polypeptide's binding to IL-2Rβ (CD122) or to the complex of IL-2β and IL-2Rγ (CD132). In some embodiments, the antibody or antigen-binding fragment, when in a complex with a human IL-2 polypeptide, enhances the IL-2 polypeptide's thermal stability. In some embodiments, the complex has higher thermal stability or Tagg temperature than a complex formed by said IL-2 polypeptide and an IL-2Rβ; and/or the IL-2 polypeptide in the complex has increased Tagg temperature. In some embodiments, Tagg is increased by more than 2°C, more than 5°C, approximately 10 °C, or more than 10°C.

Also provided are anti-IL-2 antibodies or antigen-binding fragments thereof that compete for binding to human IL-2 with, or bind to the same epitope as, the antibodies or antigen-binding fragments specifically exemplified herein.

In another aspect, the present disclosure provides a prodrug comprising an IL-2 cytokine moiety, a masking moiety, and optionally a carrier moiety, wherein the masking moiety comprises the antibody or antigen-binding fragment herein, and the cytokine moiety comprises SEQ ID NO: 1, or an amino acid at least 90% identical thereto (e.g., the mutant IL-2 polypeptides herein). In some embodiments, the cytokine moiety comprises a mutant IL-2 polypeptide described herein. In some embodiments, the IL-2 cytokine moiety comprises an amino acid sequence selected from SEQ ID NOs: 1-33. In some embodiments, the masking moiety is an antibody comprising a VH of SEQ ID NO:191 or an amino acid sequence at least 95% identical thereto, and a VL of SEQ ID NO:190 or an amino acid sequence at least 95% identical thereto. In some embodiments, the IL-2 cytokine moiety comprises SEQ ID NO:1, wherein and in other embodiments, one or more mutations are selected from T3A, L36I, V69A, Q74P, and C125A.

In a related aspect, the present disclosure also provides a prodrug comprising an IL-2 cytokine moiety, a masking moiety, and optionally a carrier moiety, wherein the masking moiety binds to the cytokine moiety and inhibits a biological activity of the cytokine moiety, and the IL-2 cytokine moiety comprises the mutant IL-2 polypeptide described herein. In some embodiments, the prodrug comprises an IL-2 cytokine moiety, a masking moiety, and optionally a carrier moiety, wherein the masking moiety binds to the cytokine moiety and inhibits a biological activity of the cytokine moiety, and the IL-2 cytokine moiety comprises the mutant IL-2 polypeptide. In some embodiments, the masking moiety comprises an extracellular domain (ECD) of IL-2Rβ or a functional fragment thereof, or a single chain variable fragment (scFv) or a Fab. In some embodiments, the masking moiety comprises an IL-2Rβ ECD comprising SEQ ID NO:37 or an amino acid sequence at least 90% identical thereto. In some embodiments, the mutant IL-2 polypeptide comprises an L36I mutation, and may additionally comprise a C125A mutation, and wherein the masking moiety may comprise SEQ ID NO:34 or an amino acid sequence at least 90% identical thereto. In some embodiments, the mutant IL-2 polypeptide comprises an L36I mutation, a C125A mutation, and the masking moiety may comprise SEQ ID NO:34 or an amino acid sequence at least 90% identical thereto. In some embodiments, the masking moiety comprises the anti-IL-2 antibody or an antigen-binding fragment thereof. In some embodiments, the prodrug comprises a carrier moiety selected from an antigen-binding moiety, an Fc domain, albumin or a fragment thereof, and PEG.

In some embodiments, the carrier moiety comprises an antigen-binding moiety that targets an antigen presented on an immune or a cancer cell, wherein the antigen-binding moiety may be a bispecific antibody, a single domain antibody, a Fab, or an scFv. In some embodiments, the antigen-binding moiety targets an antigen presented on a T cell, an NK cell, a macrophage, or a cell in a tumor microenvironment (TME), wherein the antigen may be selected from PD-1, CD3, CD4, CD8, Tim-3, LAG-3, TIGIT, HER2, signal-regulatory protein alpha (SIRPα), CTLA-4, CSF1R, NKG2A, NKG2D, CD16A, NKp30, NKp46, ILT2, ILT4, CD40, CD163, LRRC15, fibroblast activation protein (FAP), an A1 domain of tenascin C (TNC A1), an A2 domain of tenascin C (TNC A2), an extra domain B of fibronectin (EDB), fibronectin (α5β1), vitronectin (αvβ3 and αvβ5 integrins), carcinoembryonic antigen (CEA), prostate specific antigen (PSA), 5T4, BCMA, PD-L1, CD47, epidermal growth factor receptor (EGFR), c-MET, Claudin 18.2, Claudin 6, CD20, CD24, CD38, CD47, GPC3, mesothelin, ROR1, and melanoma-associated chondroitin sulfate proteoglycan (MCSP). In some embodiments, the antigen-binding moiety comprises an anti-PD-1 antibody or an antigen-binding fragment thereof, wherein the anti-PD-1 antibody may be selected from nivolumab and pembrolizumab. In some embodiments, the antigen-binding moiety comprises an anti-CD8 antibody or an antigen-binding fragment thereof, wherein the antigen-binding moiety may comprise OKT8 or humanized OKT8, the heavy and light chain CDR1-3 derived from OKT8, and/or a light chain variable domain comprising SEQ ID NO:52 or an amino acid sequence at least 90% identical thereto, and a heavy chain variable domain comprising SEQ ID NO:53 or 54 or an amino acid sequence at least 90% identical thereto. In some embodiments, the carrier moiety comprises an IgG Fc domain or an IgG antibody comprising L234A and L235A ("LALA") mutations (Eu numbering), and/or knobs-into-holes mutations wherein the IL-2 cytokine moiety and the masking moiety are fused to different polypeptide chains of the Fc domain, to the different heavy chains of the IgG antibody, or to the light chain and the heavy chain, respectively, of the IgG antibody.

In some embodiments, the prodrug comprises one or more cleavable and/or non-cleavable peptide linkers. In some embodiments, the masking moiety is fused to the carrier moiety through a cleavable or non-cleavable peptide linker. In some embodiments, the cytokine moiety is fused to the carrier moiety through a cleavable or non-cleavable peptide linker, or to the masking moiety through a cleavable peptide linker. In some embodiments, the cleavable peptide linker is cleavable by one or more proteases located in a tumor microenvironment (TME), and the cleavage leads to activation of the prodrug in the TME, wherein the cleavable peptide linker may comprise a substrate sequence of urokinase-type plasminogen activator (uPA), matrix, metallopeptidase 2 (MMP2), MMP7, MMP9, MMP14, legumain, or matriptase, and/or substrate sequences of two, three, four, or more proteases that are preferentially expressed in the TME. In some embodiments, the cleavable peptide linker comprises an amino acid sequence selected from SEQ ID NOs:55-124, 268, and 269.

In some embodiments, the present disclosure provides a prodrug comprising the mutant IL-2 polypeptide described herein, wherein the prodrug comprises a first heavy chain polypeptide chain, a second heavy chain polypeptide chain, and one or two light chains, wherein: a. the first heavy chain polypeptide chain comprises SEQ ID NO: 275 or an amino acid sequence at least 95% identical thereto, the second heavy chain polypeptide chain comprises SEQ ID NO: 277 or an amino acid sequence at least 95% (e.g., at least 96, 97, 98, or 99%) identical thereto, and the two identical light chains comprise SEQ ID NO: 276 or an amino acid sequence at least 95% identical thereto; b. the first heavy chain polypeptide chain comprises SEQ ID NO: 278 or an amino acid sequence at least 95% (e.g., at least 96, 97, 98, or 99%) identical thereto, the second heavy chain polypeptide chain comprises SEQ ID NO: 279 or an amino acid sequence at least 95% (e.g., at least 96, 97, 98, or 99%) identical thereto, and the two identical light chains comprise SEQ ID NO: 189 or an amino acid sequence at least 95% identical thereto; c. the first and second heavy chain polypeptide chains comprise SEQ ID NO: 286 or an amino acid sequence at least 95% (e.g., at least 96, 97, 98, or 99%) identical thereto, and the two identical light chains comprise SEQ ID NO: 276 or an amino acid sequence at least 95% identical thereto; d. the first and second heavy chain polypeptide chains comprise SEQ ID NO: 187 or an amino acid sequence at least 95% (e.g., at least 96, 97, 98, or 99%) identical thereto, and the two identical light chains comprise SEQ ID NO: 189 or an amino acid sequence at least 95% identical thereto; or e. the first heavy chain polypeptide chain comprises SEQ ID NO: 283 or an amino acid sequence at least 95% (e.g., at least 96, 97, 98, or 99%) identical thereto, the second heavy chain polypeptide chain comprises SEQ ID NO: 284 or an amino acid sequence at least 95% (e.g., at least 96, 97, 98, or 99%) identical thereto, and the one light chain comprises SEQ ID NO: 189 or an amino acid sequence at least 95% identical thereto.

In another aspect, the present disclosure provides an IL-2 antibody fusion molecule comprising two identical antibody light chains, a first antibody heavy chain, and a second antibody heavy chain, wherein a. the light chains each comprise SEQ ID NO:38 or an amino acid sequence at least 90% identical thereto, the first heavy chain comprises SEQ ID NO:40, 41, 45, 46, or an amino acid sequence at least 95% identical thereto, and the second heavy chain comprises SEQ ID NO:42, 43, 44, or 47 or an amino acid sequence at least 95% identical thereto; b. the light chains each comprise SEQ ID NO:38 or an amino acid sequence at least 90% identical thereto, and the first and second heavy chains each comprise SEQ ID NO:40, 41, 48, 49, 50, or 51 or an amino acid sequence at least 95% identical thereto; or c. the light chains each comprise SEQ ID NO:189 or an amino acid sequence at least 90% identical thereto, the first heavy chain comprises SEQ ID NO:185 or an amino acid sequence at least 95% identical thereto, and the second heavy chain comprises SEQ ID NO:186 or an amino acid sequence at least 95% identical thereto.

In yet another aspect, the present disclosure provides an IL-2 antibody fusion molecule comprising: a. two identical light chains, and two identical heavy chains, wherein the light chains and heavy chains respectively comprise (i) SEQ ID NO:207 or an amino acid sequence at least 95% identical thereto, and SEQ ID NO:205, 206, 211, 212, 213, or 214 or an amino acid sequence at least 95% identical thereto, or (ii) SEQ ID NO:208 or an amino acid sequence at least 95% identical thereto, and SEQ ID NO:215 or an amino acid sequence at least 95% identical thereto; or b. a first polypeptide chain and a second polypeptide chain, wherein the first and second polypeptide chains respectively comprise (i) SEQ ID NO:196 or 197 or an amino acid sequence at least 95% identical thereto, and SEQ ID NO:209 or 210 or an amino acid sequence at least 95% identical thereto, or (ii) SEQ ID NO:198 or an amino acid sequence at least 95% identical thereto, and SEQ ID NO:199, 202, 203 or 204 or an amino acid sequence at least 95% identical thereto.

Also provided are pharmaceutical compositions comprising the mutant human IL-2 polypeptides, prodrugs, or IL-2 antibody fusion molecules herein and a pharmaceutically acceptable excipient.

Also provided are one or more polynucleotides encoding the new proteins (i.e., mutant human IL-2 polypeptides, prodrugs, antibodies or antigen-binding fragments thereof, or IL-2 antibody fusion molecules) herein; expression vectors comprising the polynucleotides; host cells comprising the expression vectors; and methods of producing the new proteins by culturing mammalian host cells that allow expression of the new proteins and isolating the expressed new protein from the culture.

In yet another aspect, the present disclosure provides a method of treating a cancer or an infectious disease or modulating (e.g., stimulating) the immune system in a patient in need thereof, comprising administering to the patient a therapeutically effective amount of the present mutant IL-2 polypeptide, prodrug, IL-2 antibody fusion molecule, or pharmaceutical composition. Also provided are mutant IL-2 polypeptides, prodrugs, IL-2 antibody fusion molecules, and pharmaceutical compositions for use in treating a cancer or an infectious disease or modulating (e.g., stimulating) the immune system in a patient (e.g., human patients) in need thereof; and use of these mutant IL-2 polypeptides, prodrugs, and IL-2 antibody fusion molecules for the manufacture of a medicament for treating a cancer or an infectious disease or modulating (e.g., stimulating) the immune system in a patient in need thereof. In some embodiments, the patient has a viral infection (e.g., HIV infection); has a cancer selected from the group consisting of leukemia, lymphoma, kidney cancer, bladder cancer, urinary tract cancer, cervical cancer, brain cancer, head and neck cancer, skin cancer, uterine cancer, testicular cancer, esophageal cancer, liver cancer, colorectal cancer, stomach cancer, squamous cell carcinoma, prostate cancer, pancreatic cancer, lung cancer such as non-small cell lung cancer, cholangiocarcinoma, breast cancer, and ovarian cancer, and medullary thyroid cancer; or has an inflammatory or an autoimmune disease, for example one selected from asthma, Type I diabetes, rheumatoid arthritis, allergy, systemic lupus erythematosus, organ graft rejection, and graft-versus-host disease. Articles of manufacture (e.g., kits) comprising one or more dosing units of the present new proteins are also provided.

Other features, objects, and advantages of the invention are apparent in the detailed description that follows. It should be understood, however, that the detailed description, while indicating embodiments and aspects of the invention, is given by way of illustration only, not limitation. Various changes and modification within the scope of the invention will become apparent to those skilled in the art from the detailed description.

### BRIEF DESCRIPTIONS OF THE DRAWINGS

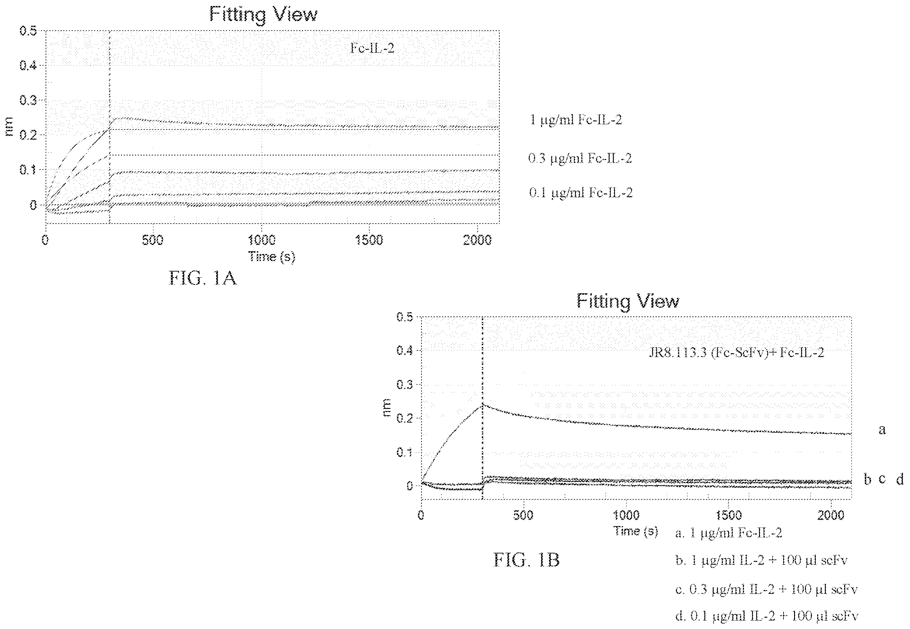

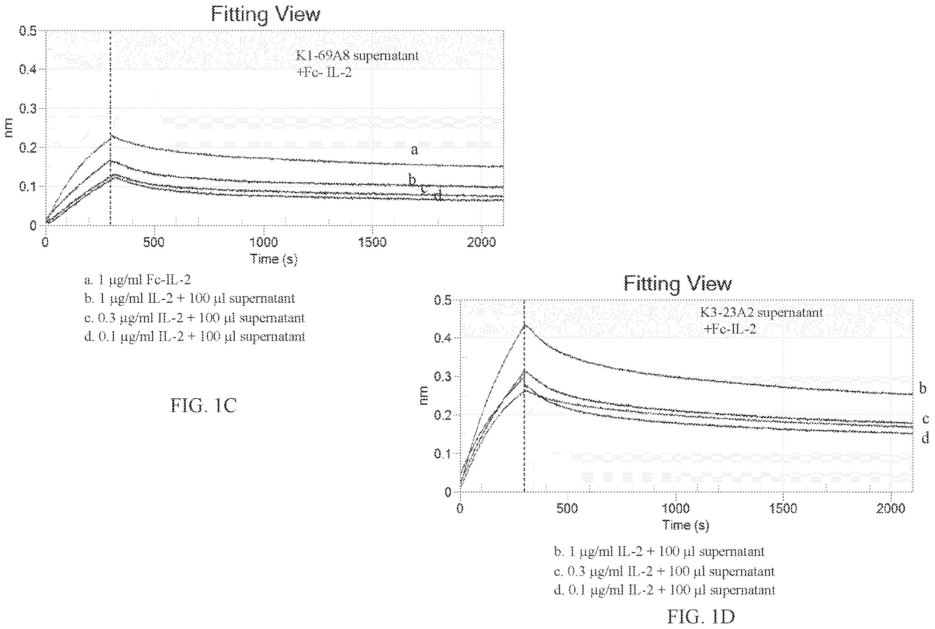

**FIGs. 1A-D** show the blocking assay results. The supernatants of the candidate scFv clones were tested for their ability of blocking the binding of Fc-IL-2 to Fc-IL-2Rβγ. The blocking was tested using an Octet Red96e Kinetics binding assay. **FIG. 1A** is a graph showing the binding affinity of IL-2 (at 1 µg/ml, 0.3 µg/ml, and 0.1 µg/ml) to IL-2Rβγ. **FIG. 1B** is a graph showing the blocking of IL-2 binding to IL-2Rβγ. **FIG. 1C** is a graph showing the partial inhibition of IL-2 binding to IL-2Rβγ. **FIG. 1D** shows the blocking assay by 100 µl of the supernatants of the clone K3-23A2, which did not block the binding of IL-2 to IL-2Rβγ.
**FIG. 2** shows results from the CTLL2 cell-based IL-2 activity inhibition assay using different fusion (scFv-Fc) proteins.
**FIG. 3A** is a table showing the heavy chains and titer of expression of scFv clones.
**FIG. 3B** is a graph showing a CTLL2 cell-based IL-2 activity inhibition assay using various scFv clones.
**FIGs. 4A** and **4B** show the binding kinetics of selected scFv-Fc fusion molecules. The name, binding affinity, and response are shown for each clone.

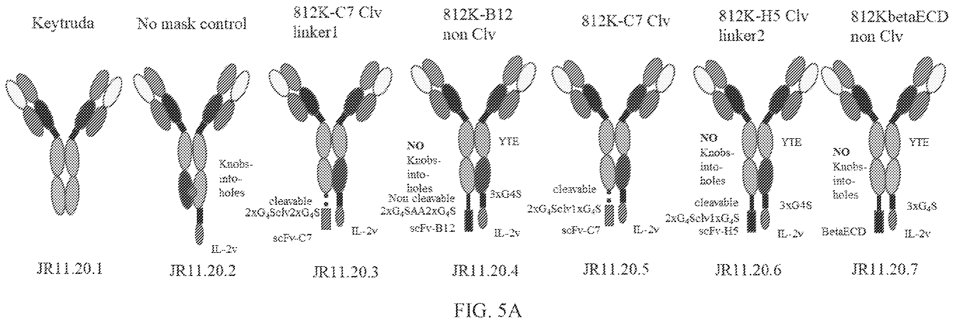

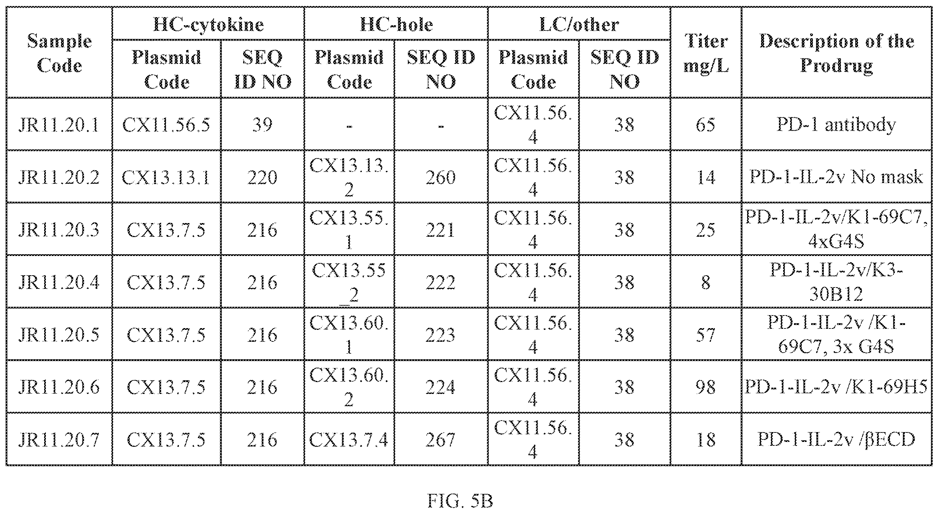

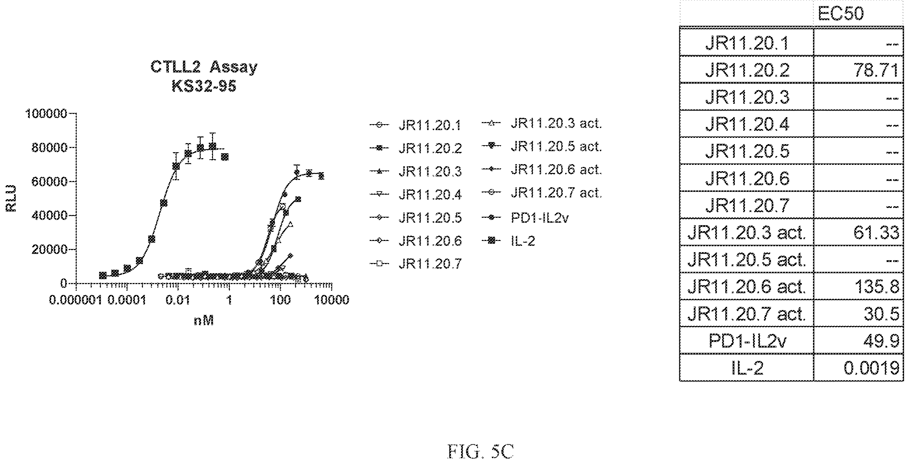

**FIGs. 5A-C** show the information of PD-1 antibody-IL-2 prodrugs and screening data of the prodrugs following a CTLL2-based activity assay. **FIG. 5A** shows the schematic illustrations of the anti-PD-1 antibody Keytruda^{®} (JR11.20.1), the PD-1 antibody-IL-2 fusion molecule (JR11.20.2), and the PD-1 antibody-IL-2 prodrug fusion molecules with theIL-2 moiety of each prodrug having a mask comprising a scFv against IL-2 (JR11.20.3 to JR11.20.6) or IL-2Rβ-ECD (JR11.20.7). **FIG. 5B** is a table showing the names of the samples/molecules, the plasmid codes and SEQ ID NO for each molecule. The titer of expression and a brief description of each molecule are also included. **FIG. 5C** shows the results from the CTLL2 cell-based activity assay of the prodrug samples prior to and after activation.

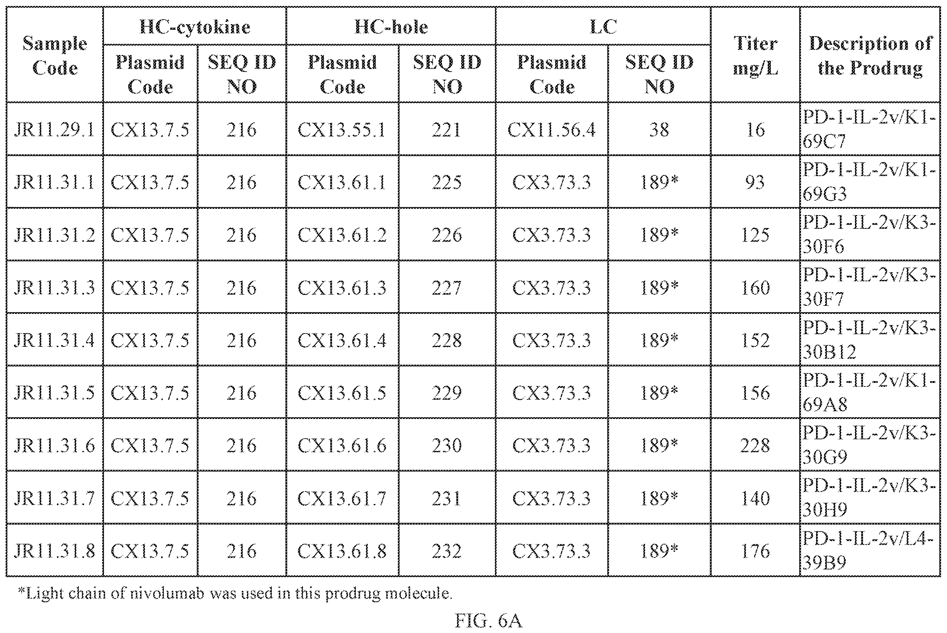

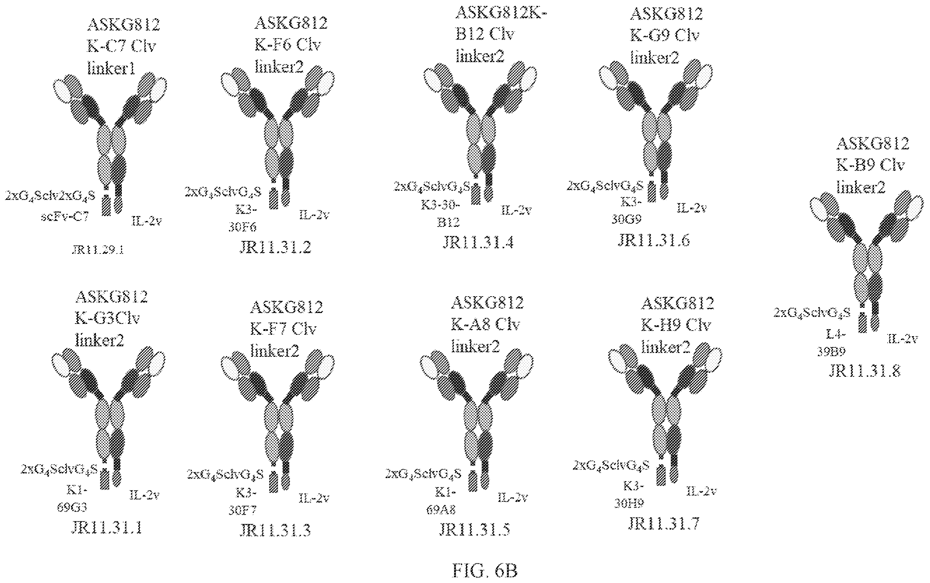

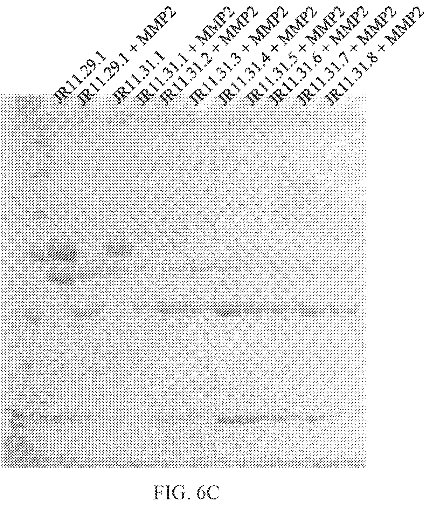

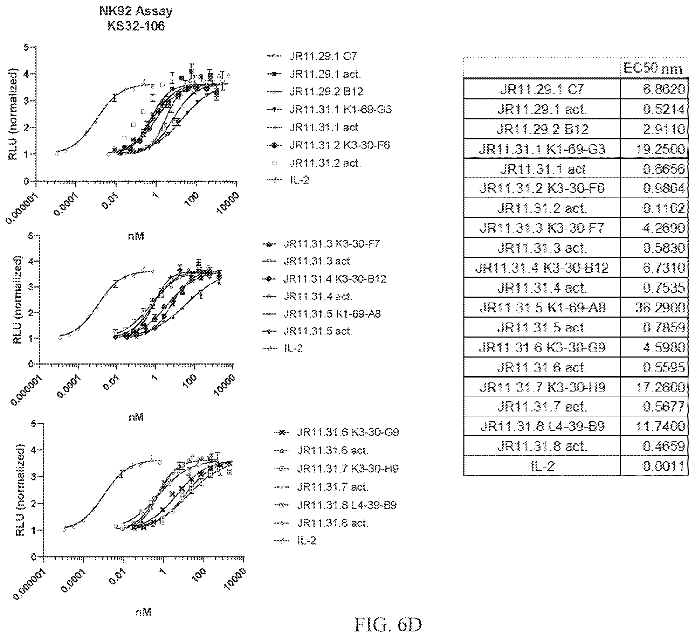

**FIGs. 6A-D** show the information and data from the screening of PD-1 antibody-IL-2 prodrugs based on NK92-based activity assay. **FIG. 6A** is a table showing the names, plasmid code, SEQ ID NO, and titers of transient expression of additional PD-1-antibody-IL-2 prodrug molecules masked by scFv's screened from a yeast library. **FIG. 6B** shows the schematic illustrations of the anti-PD-1 antibody-IL-2 prodrug fusion molecules wherein the IL-2 moiety of each prodrug is masked with a mask comprising a scFv against IL-2 (JR11.29.1, JR11.31.1-8). **FIG. 6C** shows a SDS-PAGE analysis of the prodrug samples JR11.29.1 and JR11.31.1 prior to activation and all the prodrug sample post protease MMP-2-based activation. All the samples were able to be digested by MMP-2. **FIG. 6D** are panels showing results of a NK92 cell-based activity assay (left panel), which measures the activities of the prodrug samples prior to and after the activation by MMP-2. The activities are shown on the right panel.
**FIGs. 7A-C** show the information and results from the peptide linker optimization experiment. **FIG. 7A** shows the plasmid and SEQ ID NO of each prodrug sample. **FIG. 7B** shows schematic illustrations of the anti-PD-1 antibody-IL-2 prodrug fusion molecules with the IL-2 moiety (IL-2v) linked to the heavy chain using different linkers (JR11.101.1-3) or without a linker (JR11.101.4). **FIG. 7C** shows results from the SEC-HPLC analysis.

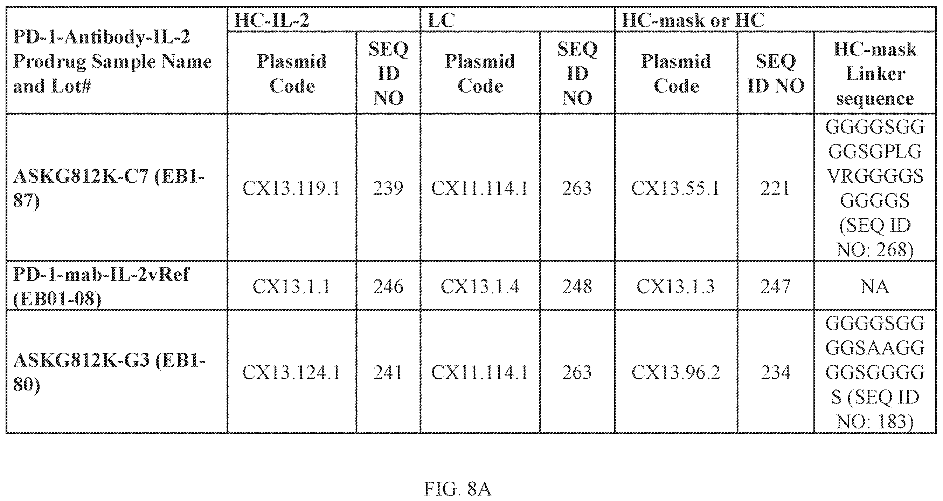

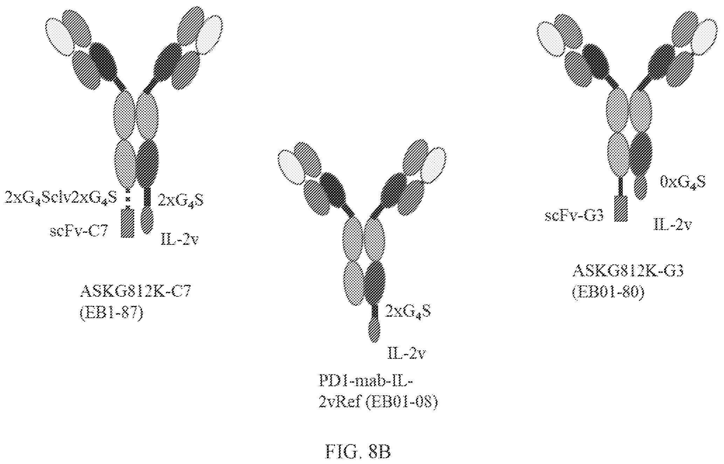

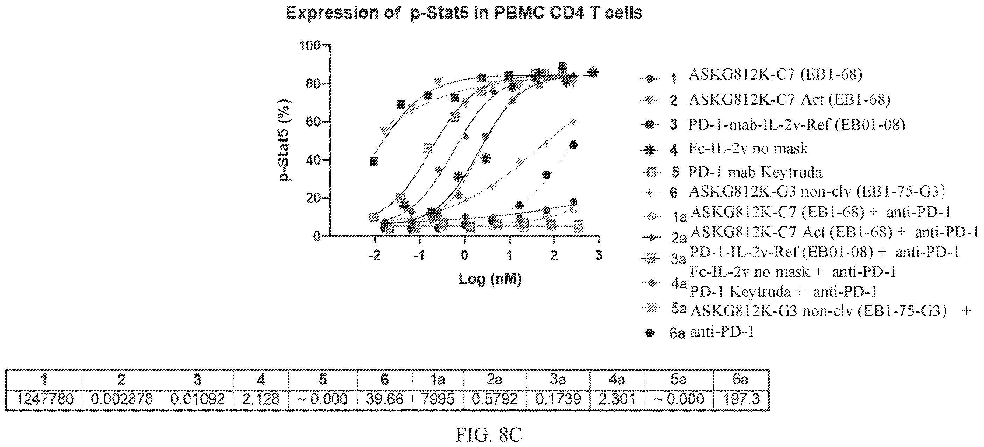

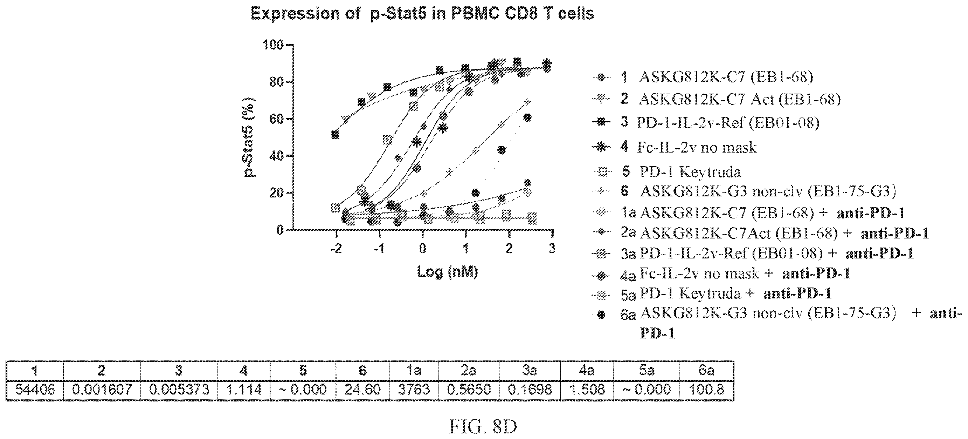

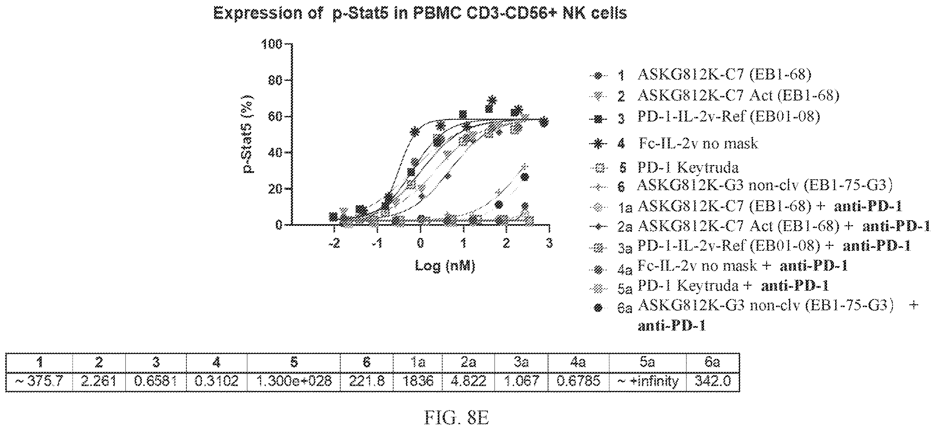

**FIGs. 8A-E** show the results for ex vivo assay of PD-1 antibody-IL-2 prodrugs with human PBMC. **FIG. 8A** is a table that shows the plasmid and SEQ ID NOs of the two prodrug molecules, ASKG812K-C7 and ASKG812K-G3, as well as the control molecule - a PD-1 antibody-IL-2v fusion molecule without a mask (PD1-mab-IL-2vRef). The prodrug ASKG812K-G3 does not have a cleavable linker. **FIG. 8B** shows the schematic illustrations of the prodrugs. IL-2 induces STAT5 Phosphorylation (pSTAT5) in immune cells. The inductions of pSTAT5 are shown in **FIG. 8C** for the CD4+ T cells, **FIG. 8D** for the CD8+ T cells, and **FIG. 8E** for the NK cells.
**FIG. 9A-D** shows the sample information and the enhancement of stability and thermal stability with scFv masks. **FIG. 9A** is a table that shows the plasmid and SEQ ID NOs of the three prodrug molecules ASKG812K-C7, ASKG812K-F7, and ASKG812K-G3 as well as the control molecule, which is an ASKG812-β-ECD prodrug masked with an IL-2Rβ extracellular domain (ECD). **FIG. 9B** is a table showing thermostability of the prodrugs. **FIG. 9C** shows the schematic illustrations of the prodrugs. **FIG. 9D** is a graph showing an accelerated stability study carried out for the prodrugs.

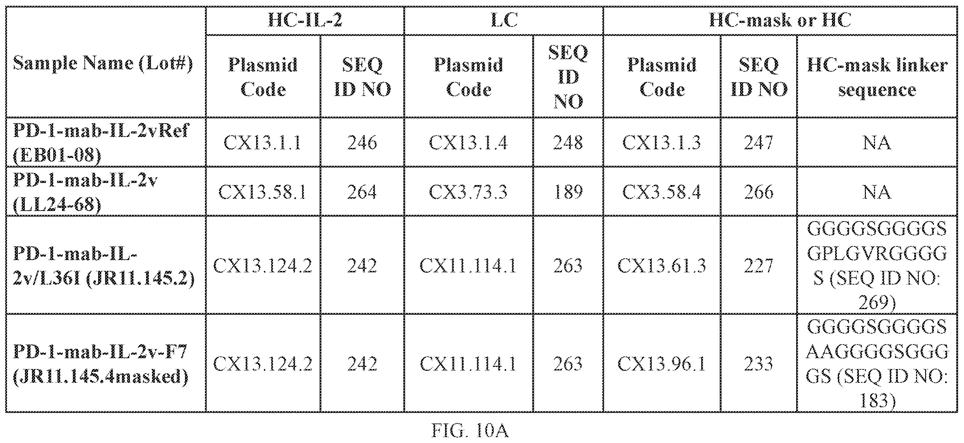

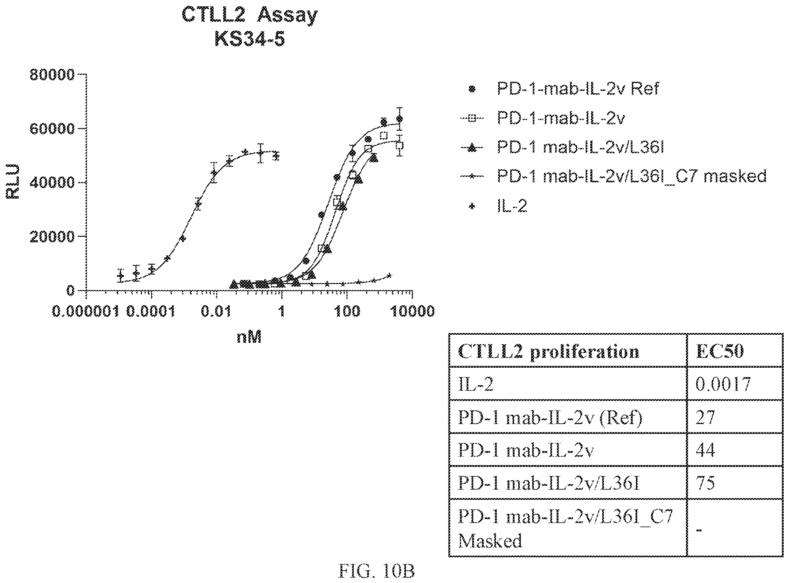

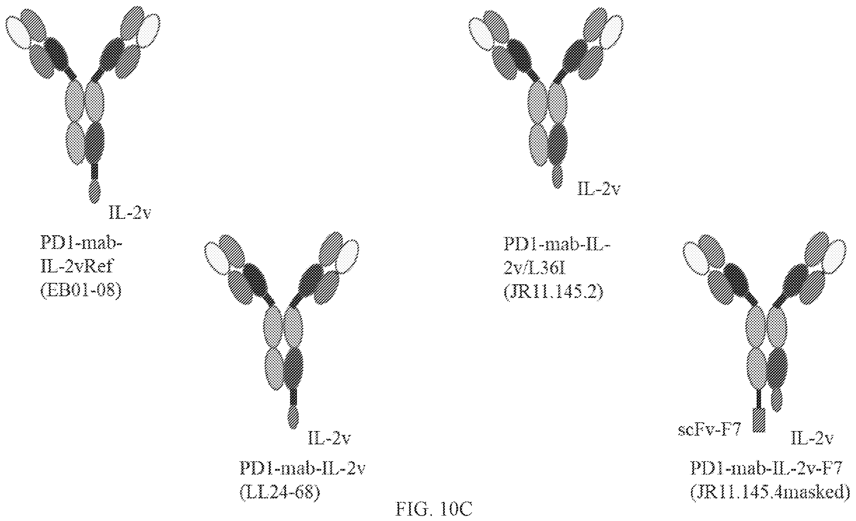

**FIG. 10A-C** show the sample information and results from a CTLL2 assay of several prodrug molecules. **FIG. 10A** is a table that shows the plasmid and SEQ ID NOs of the following molecules: PD-1 antibody-IL-2v reference molecule (EB01-08; PD1-mab-IL-2v Ref), PD-1 antibody-IL-2v (LL24-68; PD1-mab-IL-2v), PD-1 antibody-IL-2v/L36I (JR11.145.2; PD1-mab-IL-2v/L36I), and PD-1 antibody-IL-2v/L36I masked with F7 (JR11.145.4masked; PD-1 mab-IL-2v/L36I_C7 masked). All of the prodrugs were tested by the CTLL2 assay. The assay results are shown in **FIG. 10B** (left and right panels). **FIG. 10C** shows the schematic illustrations of the prodrugs.

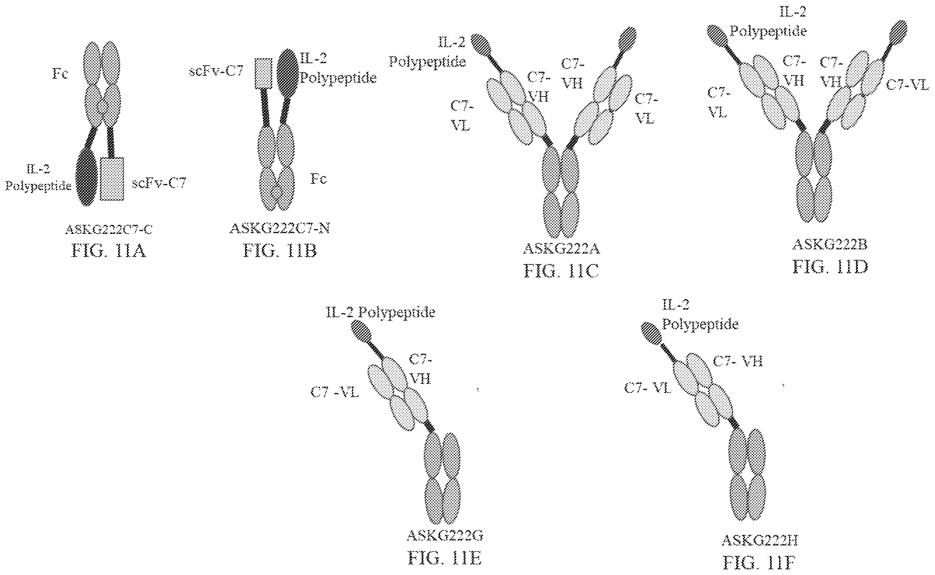

**FIGs. 11A-F** show the schematic illustrations of the structures of antibody-cytokine fusion molecules. **FIG. 11A** shows the structure of ASKG222C7-C, wherein the cytokine IL-2 and the scFv mask K1-69C7 or "C7" are fused to the C-termini of an Fc domain. **FIG. 11B** shows the structure of ASKG222C7-N, wherein the cytokine IL-2 and the mask C7 are fused to the N-termini of an Fc domain. **FIG. 11C** shows the structure of ASKG222A, wherein the antibody comprises the same VL and VH as that of C7; and wherein the IL-2 cytokine is fused to the N-terminus of each of the heavy chains. **FIG. 11D** shows the structure of ASKG222B, wherein the antibody comprises the same VL and VH as that of C7; and wherein the IL-2 cytokine is fused to the N-terminus of each of the light chains. **FIG. 11E** shows the structure of ASKG222G, which is essentially the same as that of ASKG222A (**FIG. 11C**) except that it has a single Fab. **FIG. 11F** shows the structure of ASKG222H, which is essentially the same as that of ASKG222B (**FIG. 11D**) except that it has a single Fab.
**FIG. 12A** shows the plasmid and sequence information of ASKG222C7-C and ASKG222C7-D.
**FIG. 12B** shows the DRC-HPLC purity of the molecules expressed in CHO cells and purified by Protein A affinity chromatography.
**FIG. 13A** shows the plasmid and sequence information of ASKG222A-C7-Ab and ASKG222B-C7-Ab.
**FIG. 13B** shows the DRC-HPLC purity of the molecules expressed in CHO cells and purified by Protein A affinity chromatography.
**FIGs. 14A** and **14B** show the results of an ex vivo assay using the antibody-cytokine fusion molecules. **FIG. 14A** is a graph showing the activity of the antibody-fusion molecules in stimulating the proliferation of Ki67+ regulatory T cells (Treg). **FIG. 14B** is a graph showing the activity of the antibody-fusion molecules in stimulating the proliferation of Ki67+ effector T cells (Teff).
**FIG. 15** is a graph showing the rat PK data for ASKG222A-C7-Ab. The results showed that ASKG222A-C7-Ab has a half-life of 66-88 hours.
**FIGs. 16A-16E** show schematic illustrations of the structures of antibody-cytokine fusion molecules and the sequences of the polypeptide chains, which form the fusion molecules.
**FIGs. 17A** and **17B** show the *in vivo* efficacy and safety results of the two fusion molecules (812mN-mut4 and 812mW5-mut4) in comparison with the reference molecule Ref3, and an anti-mouse PD-1 antibody.
**FIG. 18** shows sequence information for the antibody-cytokine fusion molecules.
**FIGs. 19A** and **19B** show the *in vivo* efficacy and safety results of the fusion molecules (678F3-Fab-B) at two different dosages.
**FIG. 20** shows the results of the *in vitro* cell based activity assay (HEK Blue Reporter Assay).
**FIG. 21** shows the schematic drawing of the structure of the fusion molecule 678F3-Fab-B.

### DETAILED DESCRIPTION OF THE INVENTION

As used herein and in the appended clauses, the singular forms "a," "or," and "the" include plural referents unless the context clearly dictates otherwise.

Reference to "about" a value or parameter herein includes (and describes) variations that are directed to that value or parameter *per se.* For example, description referring to "about X" includes description of "X." Additionally, use of "about" preceding any series of numbers includes "about" each of the recited numbers in that series. For example, description referring to "about X, Y, or Z" is intended to describe "about X, about Y, or about Z."

The term "antigen-binding moiety" refers to a polypeptide or a set of interacting polypeptides that specifically bind to an antigen, and includes, but is not limited to, an antibody (e.g., a monoclonal antibody, polyclonal antibody, a multi-specific antibody, a dual specific or bispecific antibody, an anti-idiotypic antibody, or a bifunctional hybrid antibody) or an antigen-binding fragment thereof (e.g., a Fab, a Fab', a F(ab')₂, a Fv, a disulfide linked Fv, a scFv, a single domain antibody (dAb), or a diabody), a single chain antibody, and an Fc-containing polypeptide such as an immunoadhesin or an scFv-Fc. In some embodiments, the antibody may be of any heavy chain isotype (e.g., IgG, IgA, IgM, IgE, or IgD) or subtype (e.g., IgG₁, IgG₂, IgG₃, or IgG₄). In some embodiments, the antibody may be of any light chain isotype (e.g., kappa or lambda). The antibody may be human, non-human (e.g., from mouse, rat, rabbit, goat, or another non-human animal), chimeric (e.g., with a non-human variable region and a human constant region), or humanized (e.g., with non-human CDRs and human framework and constant regions). In some embodiments, the antibody is a derivatized antibody.

The term "cytokine agonist polypeptide" refers to a wildtype cytokine, or an analog thereof. An analog of a wildtype cytokine has the same biological specificity (e.g., binding to the same receptor(s) and activating the same target cells) as the wildtype cytokine, although the activity level of the analog may be different from that of the wildtype cytokine. The analog may be, for example, a mutein (i.e., mutated polypeptide) of the wildtype cytokine, and may comprise at least one, at least two, at least three, at least four, at least five, at least six, at least seven, at least eight, at least nine, or at least ten mutations relative to the wildtype cytokine.

The term "prodrug" herein refers to a cytokine fusion protein that comprises a cytokine moiety bound by a masking moiety and has not yet been activated to remove the mask from the cytokine moiety. Once the cytokine moiety is unbound, the fusion molecule becomes "activated."

The terms "cytokine antagonist," "cytokine mask," and "masking moiety" refer to a moiety (e.g., a polypeptide) that binds to a cytokine and thereby inhibits the cytokine from binding to its receptor on the surface of a target cell and/or exerting its biological functions while being bound by the antagonist or mask. Examples of a cytokine antagonist or mask include, without limitations, a polypeptide derived from an extracellular domain of the cytokine's natural receptor that makes contact with the cytokine and an antibody that binds to the cytokine or an antigen-binding fragment thereof (e.g., scFv).

The term "effective amount" or "therapeutically effective amount" refers to an amount of a compound or composition sufficient to treat a specified disorder, condition, or disease, such as ameliorate, palliate, lessen, and/or delay one or more of its symptoms. In reference to a disease such as cancer, an effective amount may be an amount sufficient to delay cancer development or progression (e.g., decrease tumor growth rate, and/or delay or prevent tumor angiogenesis, metastasis, or infiltration of cancer cells into peripheral organs), reduce the number of epithelioid cells, cause cancer regression (e.g., shrink or eradicate a tumor), and/or prevent or delay cancer occurrence or recurrence. An effective amount can be administered in one or more administrations.

The term "functional analog" refers to a molecule that has the same biological specificity (e.g., binding to the same ligand) and/or activity (e.g., activating or inhibiting a target cell) as a reference molecule.

The term "fused" or "fusion" in reference to two polypeptide sequences refers to the joining of the two polypeptide sequences through a backbone peptide bond. Two polypeptides may be fused directly or through a peptide linker that is one or more amino acids long. A fusion polypeptide may be made by recombinant technology from a coding sequence containing the respective coding sequences for the two fusion partners, with or without a coding sequence for a peptide linker in between. In some embodiments, fusion encompasses chemical conjugation.

The term "pharmaceutically acceptable excipient" when used to refer to an ingredient in a composition means that the excipient is suitable for administration to a treatment subject, including a human subject, without undue deleterious side effects to the subject and without affecting the biological activity of the active pharmaceutical ingredient (API).

The term "subject" refers to a mammal and includes, but is not limited to, a human, a pet (e.g., a canine or a feline), a farm animal (e.g., cattle or horse), a rodent, or a primate.

As used herein, "treatment" or "treating" is an approach for obtaining beneficial or desired clinical results. Beneficial or desired clinical results include, but are not limited to, one or more of the following: alleviating one or more symptoms resulting from a disease, diminishing the extent of a disease, ameliorating a disease state, stabilizing a disease (e.g., preventing or delaying the worsening or progression of the disease), preventing or delaying the spread (e.g., metastasis) of a disease, preventing or delaying the recurrence of a disease, providing partial or total remission of a disease, decreasing the dose of one or more other medications required to treat a disease, increasing the patient's quality of life, and/or prolonging survival. The methods of the present disclosure contemplate any one or more of these aspects of treatment.

It is to be understood that one, some, or all of the properties of the various embodiments described herein may be combined to form other embodiments of the present invention. The section headings used herein are for organizational purposes only and are not to be construed as limiting the subject matter described thereunder.

### I. Mutant IL-2 Prodrugs

The present disclosure further provides cytokine prodrugs that are metabolized *in vivo* at a target site to become active cytokine therapeutics. The cytokine prodrugs have fewer side effects (including less immunogenicity risk from the cytokine as compared to the wildtype cytokine), better *in vivo* PK profiles (e.g., longer half-life) and better target specificity, and are more efficacious as compared to prior cytokine therapeutics. The present prodrugs comprise a cytokine moiety comprising a cytokine agonist polypeptide linked to a carrier moiety and masked (bound) by a cytokine antagonist (masking moiety). The cytokine antagonist may be, for example, an extracellular domain of a receptor for the cytokine, and is linked to the cytokine moiety or to the carrier moiety through a peptide linker (e.g., a cleavable or non-cleavable peptide linker). The mask inhibits the cytokine moiety's biological functions while the mask is binding to it. The prodrugs may be activated at a target site (e.g., at a tumor site or the surrounding environment) in the patient by cleavage of the linker and the consequent release of the cytokine mask from the prodrug, exposing the previously masked cytokine moiety and allowing the cytokine moiety to bind to its receptor on a target cell and exert its biological functions on the target cell. In other embodiments, when the masking moiety is linked to the carrier moiety through a non-cleavable peptide linker, the prodrugs disclosed herein can engage the target cell via "cis-binding" of a cytokine receptor and an antigen expressed on the cell surface, leading to increased activity of the prodrug without cleavage and removal of the masking moiety. The cytokine moiety of the prodrugs may increase in activity at a target site (e.g., at a tumor site or the surrounding environment), where both the antigen targeted by the carrier and a receptor of the cytokine are expressed on the same cell.

In some embodiments, the carriers for the prodrugs are antigen-binding moieties, such as antibodies or antigen-binding fragments thereof, that bind an antigen at the target site.

In some embodiments, the present prodrugs are pro-inflammatory cytokine prodrugs that are metabolized to become pro-inflammatory cytokines at a target site in the body targeted by the carrier moiety. In further embodiments, the carrier moiety in the prodrug is an antibody or antigen-binding fragment thereof targeting a tumor antigen such that the prodrug is delivered to a tumor site in a patient and is metabolized locally (e.g., inside or in the vicinity of the tumor microenvironment) through cleavage of the linker linking the cytokine mask to the carrier moiety or the cytokine moiety, making the pro-inflammatory cytokine moiety available to interact with its receptor on a target cell and stimulating the target immune cells locally.

An IL-2 prodrug may comprise a cytokine moiety comprising an IL-2 agonist polypeptide, a carrier moiety, and a masking moiety (an IL-2 antagonist), wherein the cytokine moiety is fused to the carrier moiety directly or through a linker (e.g., cleavable or non-cleavable peptide linker), and the IL-2 antagonist is linked to the IL-2 agonist polypeptide or to the carrier moiety through a cleavable peptide linker. In the present IL-2 prodrugs, the IL-2 agonist polypeptide may be an IL-2 mutein such as an IL-2 mutein described herein derived from a human IL-2. The IL-2 mutein may have significantly reduced affinity for CD25 or the trimeric high-affinity IL-2R, as compared to wildtype IL-2. In some embodiments, the IL-2 mutein has binding affinity for the high-affinity IL-2R that is 100 times, 300 times, 500 times, 1,000 times, or 10,000 times lower as compared to wildtype IL-2. Unless otherwise indicated, all residue numbers in IL-2 and IL-2 muteins described herein are in accordance with the numbering in SEQ ID NO: 1.

The present disclosure further provides a prodrug that comprises a mutant IL-2 polypeptide as described above and a carrier moiety. In some embodiments, the carrier moiety comprises an antigen-binding moiety, wherein the antigen-binding moiety binds to an antigen expressed on an immune cell. In some embodiments, the prodrug comprises an antigen-binding moiety, wherein the antigen-binding moiety binds to an antigen expressed on a tumor cell or in the tumor microenvironment. In some embodiments, the carrier moiety comprises two or more antigen-binding moieties, wherein the antigen-binding moieties bind to two different antigens, wherein one of the antigens is expressed on an immune cell, and the other antigen is expressed on a tumor cell or in the tumor microenvironment.

In some embodiments, the prodrug further comprises a masking moiety, wherein the masking moiety binds to the mutant IL-2 polypeptide and inhibits a biological activity of the mutant IL-2 polypeptide. In some embodiments, the masking moiety comprises the extracellular domain of an IL-2 receptor subunit. In some embodiments, the masking moiety comprises the extracellular domain (ECD) of IL-2 receptor beta (IL-2Rβ) or a functional analog thereof. In some embodiments, the IL-2Rβ-ECD comprises an amino acid sequence of SEQ ID NO:37. In some embodiments, the masking moiety comprises an antibody or the antigen-binding fragment thereof, wherein the antibody binds to IL-2. In some embodiments, the masking moiety is an scFv. In some embodiments, the scFv comprises an amino acid sequence selected from SEQ ID NOs:34, 35, and 36. In some embodiments, the IL-2 prodrug further comprises a cleavable peptide linker. In some embodiments, the cleavable linker links the masking moiety to the carrier. In some embodiments, the cleavable linker links the mutant IL-2 polypeptide to the carrier.

### A. Mutant IL-2 Polypeptides

In the present IL-2 prodrugs, the IL-2 agonist polypeptide is an IL-2 mutein derived from a human IL-2. IL-2 with mutations to decrease or eliminate the interaction between IL-2 and CD25 have been disclosed previously. For example, WO 2008/0034473 refers to mutations R38W and F42K, while WO 2012/107417 refers to mutation at position 72. U.S. Pat. Pub. 2003/0124678 refers to introducing the R38W mutation to eliminate IL-2's vasopermeability activity. Heaton et al. (Cancer Res. (1993) 53:2597-2602; U.S. Pat. 5,229,109) describe introducing two mutations, R38A and F42K, to obtain an IL-2 mutein with reduced ability to induce secretion of pro-inflammatory cytokines from natural killer (NK) cells. EP2639241B1 refers to IL-2 muteins that are at least 1,000 times less effective than native IL-2 in stimulating T_{reg} cells and refers to IL-2 muteins having the mutations selected from 1) R38K, F42I, Y45N, E62L, and E68V; 2) R38A, F42I, Y45N, E62L, and E68V; 3) R38K, F42K, Y45R, E62L, and E68V; or 4) R38A, F42A, Y45A, and E62A. U.S. Pat. Pub. 2014/0328791 refers to pegylated IL-2 with reduced affinity for CD25. These IL-2 mutants were called "non-alpha" IL-2 agonist polypeptides and were shown to preferentially activate T effector cells and natural killer (NK) cells over regulatory T cells. Such IL-2 mutants may comprise one or more mutations selected from T3A, R38A, R38K, R38S, F42A, F42G, F42S, F42T, F42Q, F42E, F42N, F42D, F42R, F42K, Y45A, Y45G, Y45S, Y45T, Y45Q, Y45E, Y45N, Y45D, Y45R, Y45K, E62A, E62L, E62I, E68A, E68V, L72G, L72A, L72S, L72T, L72Q, L72E, L72N, L72D, L72R, L72K, and C125S (numbering according to SEQ ID NO:1). Unfortunately, such mutations may introduce potential immunogenicity risks. For example, IL-2 mutants comprising mutations T3A/R38S/F42A/Y45A/E62A/C125S (numbering according to SEQ ID NO: 1) showed additional hot spots based on MHC Class II binding predictions using the T Cell Epitope Prediction Tools. **Table 1** shows the results of the prediction of the wildtype IL-2. Table 2 shows the results of the IL-2 mutant with T3A/R38S/F42A/Y45A/E62A/C125S (numbering according to SEQ ID NO:1). This mutant is named IL-2V1, which comprises an amino acid sequence of SEQ ID NO:176.

**Table 1. MHC Class II Molecules Binding Prediction of WT IL-2 Using T Cell Epitope Prediction Tools**

| MHC Class II Molecule | WT smm_align_core | smm_align_IC₅₀ | SEQ ID NO |
|---|---|---|---|
| HLA-DRB1*13:02 | LISNINVIV | 9 | 289 |
| HLA-DRB1*13:02 | LISNINVIV | 9 | 289 |
| HLA-DRB1*13:02 | LISNINVIV | 10 | 289 |
| HLA-DRB1*13:02 | LISNINVIV | 10 | 289 |
| HLA-DRB1*13:02 | HLRPRDLIS | 10 | 290 |
| HLA-DRB1*07:01 | LNLAQSKNF | 21 | 291 |
| HLA-DRB1*07:01 | LAQSKNFHL | 21 | 292 |
| HLA-DRB1*07:01 | LAQSKNFHL | 21 | 292 |
| HLA-DRB1*03:01 | LLLDLQMIL | 22 | 293 |
| HLA-DRB1*03:01 | LLLDLQMIL | 22 | 293 |
| HLA-DRB1*03:01 | QLQLEHLLL | 22 | 294 |
| HLA-DRB1*03:01 | LLLDLQMIL | 23 | 293 |
| HLA-DRB1*03:01 | LLLDLQMIL | 23 | 293 |
| HLA-DRB1*07:01 | LAQSKNFHL | 24 | 292 |
| HLA-DRB1*07:01 | LAQSKNFHL | 25 | 292 |
| HLA-DRB1*13:02 | LISNINVIV | 27 | 289 |
| HLA-DRB1*13:02 | LISNINVIV | 27 | 289 |
| HLA-DRB1*07:01 | FCQSIISTL | 44 | 296 |
| HLA-DRB1*07:01 | LEEVLNLAQ | 51 | 295 |
| HLA-DRB1*07:01 | LNLAQSKNF | 52 | 292 |
| HLA-DRB1*07:01 | WITFCQSII | 54 | 297 |

**Table 2. MHC Class II Molecules Binding Prediction of IL-2 Mutants with "Non-Alpha" Mutations, T3A, and C125S (IL-2V1, SEQ ID NO:176) Using T Cell Epitope Prediction Tools**

| MHC Class II Molecule | IL-2V1 | smm_align_IC₅₀ | SEQ ID NO |
|---|---|---|---|
| | smm_align_core | | |
| HLA-DRB1*13:02 | LISNINVIV | 9 | 289 |
| HLA-DRB1*13:02 | LISNINVIV | 9 | 289 |
| HLA-DRB1*13:02 | LISNINVIV | 10 | 289 |
| HLA-DRB1*13:02 | LISNINVIV | 10 | 289 |
| HLA-DRB1*13:02 | HLRPRDLIS | 10 | 290 |
| HLA-DRB1*07:01 | LNLAQSKNF | 21 | 291 |
| HLA-DRB1*07:01 | LAQSKNFHL | 21 | 292 |
| HLA-DRB1*07:01 | LAQSKNFHL | 21 | 292 |
| HLA-DRB1*03:01 | LLLDLQMIL | 22 | 293 |
| HLA-DRB1*03:01 | LLLDLQMIL | 22 | 293 |
| HLA-DRB1*03:01 | QLQLEHLLL | 22 | 294 |
| HLA-DRB1*03:01 | LLLDLQMIL | 23 | 293 |
| HLA-DRB1*03:01 | LLLDLQMIL | 23 | 293 |
| HLA-DRB1*07:01 | LAQSKNFHL | 24 | 292 |
| HLA-DRB1*07:01 | LAQSKNFHL | 25 | 292 |
| HLA-DRB1*13:02 | LISNINVIV | 27 | 289 |
| HLA-DRB1*13:02 | LISNINVIV | 27 | 289 |
| HLA-DRB1*07:01 | RWITFSQSI | 35 | 287 |
| HLA-DRB1*07:01 | FSQSIISTL | 38 | 299 |
| HLA-DRB1*01:01 | LTSMLTAKF | 42 | 298 |
| HLA-DRB1*01:01 | LTSMLTAKF | 45 | 298 |
| HLA-DRB1*01:01 | LTSMLTAKF | 45 | 298 |
| HLA-DRB1*01:01 | LTSMLTAKF | 45 | 298 |
| HLA-DRB1*07:01 | LEEVLNLAQ | 51 | 295 |
| HLA-DRB1*07:01 | LNLAQSKNF | 52 | 291 |
| HLA-DRB1*01:01 | NPKLTSMLT | 52 | 288 |

In some embodiments, the present invention provides novel IL-2 muteins (mutant IL-2 polypeptides) with lower immunogenicity risk. In some embodiments, the mutant IL-2 polypeptide comprises a mutation in position L36 (numbering according to SEQ ID NO: 1). In particular embodiments, the mutant IL-2 polypeptide comprises an L36I mutation (numbering according to SEQ ID NO: 1). In some embodiments, the mutant IL-2 polypeptide comprises an amino acid sequence selected from SEQ ID NOs:2-33, or at least 95%, at least 98%, or at least 99% to an amino acid sequence selected from SEQ ID NOs:2-33.

In some embodiments, the mutant IL-2 polypeptide disclosed herein may further comprise a mutation at A73 (e.g., a mutation to T or another amino acid residue) and/or the K35N mutation. Without being bound by theory, the present inventors contemplate that A73 and K35 are potential glycosylation sites on IL-2, and the mutation of these glycosylation sites modulates the IL-2 mutein's affinity for the IL-2 receptors. The mutant IL-2 polypeptides will have safer clinical profiles and can be used in patients in need of IL-2 activity, such as patients in need of a stimulated immune system (e.g., cancer patients and AIDS patients). The mutant IL-2 polypeptides can be used as a separate entity or in a conjugate (e.g., fused to a carrier such as in the present prodrugs).

In some embodiments, the mutant IL-2 polypeptides of the present invention may comprise a mutation at L36 (e.g., L36I) and one or more mutations at position(s) selected from T3, D20, K35, R38, F42, F44, Y45, E62, E68, L72, A73, N88, N90, C125, and Q126 (numbering according to SEQ ID NO:1). In certain embodiments, the mutant IL-2 polypeptide comprises mutations at L36, R38, F42, Y45, and A73 (numbering according to SEQ ID NO: 1).

In some embodiments, the mutant human IL-2 polypeptides of the present invention may comprise a K35N mutation and one or more mutations at position(s) selected from T3, D20, R38, F42, F44, Y45, E62, E68, L72, A73, N88, N90, C125, and Q126 (numbering according to SEQ ID NO: 1). In certain embodiments, the mutant human IL-2 polypide comprises the mutation K35N and additional mutations at R38, F42, and Y45, with or without a mutation at A73. In some embodiments, in addition to a mutation at L36, the mutant human IL-2 polypeptides of the present invention may comprise a K35N mutation and one or more mutations at position(s) selected from T3, D20, R38, F42, F44, Y45, E62, E68, L72, A73, N88, N90, C125, and Q126 (numbering according to SEQ ID NO:1).

In some embodiments, the mutant human IL-2 polypeptides may comprise one or more mutations at K35, R38, F42, F44, Y45, E62, E68, L72, and A73 (numbering according to SEQ ID NO: 1). In some embodiments, the mutant human IL-2 polypeptides further comprise one or more mutations at D20, N88, N90, and Q126 (numbering according to SEQ ID NO:1). Additional mutations at T3 and/or C125 may also be included.

### B. Masking Moieties of the Prodrugs

The cytokine antagonist, i.e., the masking moiety, in the present immunoconjugate may comprise a peptide, an antibody, or antibody fragment that binds to the cytokine moiety in the prodrug, thereby masking the cytokine moiety and inhibiting its biological functions. In some embodiments, the prodrug comprises a masking moiety, wherein the masking moiety binds to a mutant IL-2 polypeptide disclosed herein and inhibits a biological activity of the mutant IL-2 polypeptide.

By way of example, IL-2 antagonists may comprise peptides and antibodies that bind IL-2 and interfere with the binding of the IL-2 moiety to its receptors, leading to the reduced biological activities of the IL-2 moiety while masked. In some embodiments, the IL-2 antagonist comprises an IL-2Rβ or IL-2Rγ extracellular domain or its functional analog such as one derived from human IL-2Rβ or IL-2Rγ. In some embodiments, the IL-2Rβ-ECD comprises an amino acid sequence of SEQ ID NO:37 or an amino acid sequence at least 90% identical thereto. In some embodiments, the IL-2 antagonist comprises a peptide identified from the screening of a peptide library. In some embodiments, the masking moiety comprises an antibody or an antigen-binding fragment thereof. In particular embodiments, the IL-2 antagonist comprises an antibody or fragment thereof that blocks the binding of IL-2 or IL-2 muteins to an IL-2 receptor. In particular embodiments, the IL-2 antagonist comprises a scFv, a Fab, or a single chain Fab having the same CDR sequences as an scFv comprising an amino acid sequence selected from SEQ ID NOs:34, 35, and 36.

In some other embodiments, the prodrug further comprises a peptide linker (e.g., cleavable or non-cleavable), wherein the peptide linker links the masking moiety to the carrier. In some other embodiments, the peptide linker links the mutant IL-2 polypeptide to the carrier moiety.

### C. Carrier Moieties of the Prodrugs

The carrier moiety of the present prodrugs may be an antigen-binding moiety, or a moiety that does not bind an antigen. The carrier moiety may improve the PK profiles such as serum half-life of the cytokine agonist polypeptide, and may also target the cytokine agonist polypeptide to a target site in the body, such as a tumor site.

### 1. Antigen-Binding Carrier Moieties

The carrier moiety may be an antibody or an antigen-binding fragment thereof, or an immunoadhesin. In some embodiments, the antigen-binding moiety is a full-length antibody with two heavy chains and two light chains, a Fab fragment, a Fab' fragment, a F(ab')₂ fragment, a Fv fragment, a disulfide linked Fv fragment, a single domain antibody, a nanobody, or a single-chain variable fragment (scFv). In some embodiments, the antigen-binding moiety is a bispecific antigen-binding moiety and can bind to two different antigens or two different epitopes on the same antigen. The antigen-binding moiety may provide additional and potentially synergetic therapeutic efficacy to the cytokine agonist polypeptide.

The cytokine agonist polypeptide and its mask may be fused to the N-terminus or C-terminus of the light chains and/or heavy chains of the antigen-binding moiety. By way of example, the cytokine agonist polypeptide and its mask may be fused to a heavy chain of an antibody or an antigen-binding fragment thereof. In some embodiments, the cytokine agonist polypeptide and its mask may be fused to the light chain of an antibody or an antigen-binding fragment thereof. In some embodiments, the cytokine agonist polypeptide is fused to the C-terminus of one or both of the heavy chains of an antibody, and the cytokine's mask (masking moiety) is fused to the C-terminus of the cytokine agonist polypeptide through a cleavable or non-cleavable peptide linker. In some embodiments, the cytokine agonist polypeptide is fused to the C-terminus of one of the heavy chains of an antibody, and the cytokine's mask is fused to the C-terminus of the other heavy chain of the antibody through a cleavable or non-cleavable peptide linker, wherein the two heavy chains contain mutations that allow their specific pairing.

Strategies of forming heterodimers are well known *(see, e.g.,* Spies et al., Mol Imm. (2015) 67(2)(A):95-106). For example, the two heavy chain polypeptides in the prodrug may form stable heterodimers through "knobs-into-holes" mutations. "Knobs-into-holes" mutations are made to promote the formation of the heterodimers of the antibody heavy chains and are commonly used to make bispecific antibodies *(see, e.g.,* U.S. Pat. 8,642,745). For example, the Fc domain of the antibody may comprise a T366W mutation in the CH3 domain of the "knob chain" and T366S, L368A, and/or Y407V mutations in the CH3 domain of the "hole chain." An additional interchain disulfide bridge between the CH3 domains can also be used, e.g., by introducing a Y349C mutation into the CH3 domain of the "knobs chain" and an E356C or S354C mutation into the CH3 domain of the "hole chain" *(see, e.g.,* Merchant et al., Nature Biotech. (1998) 16:677-81). In other embodiments, the antibody moiety may comprise Y349C and/or T366W mutations in one of the two CH3 domains, and E356C, T366S, L368A, and/or Y407V mutations in the other CH3 domain. In certain embodiments, the antibody moiety may comprise Y349C and/or T366W mutations in one of the two CH3 domains, and S354C (or E356C), T366S, L368A, and/or Y407V mutations in the other CH3 domain, with the additional Y349C mutation in one CH3 domain and the additional E356C or S354C mutation in the other CH3 domain, forming an interchain disulfide bridge (numbering always according to EU index of Kabat; Kabat et al., "Sequences of Proteins of Immunological Interest," 5th ed., Public Health Service, National Institutes of Health, Bethesda, Md. (1991)). Other knobs-into-holes technologies, such as those described in EP1870459A1, can be used alternatively or additionally. Thus, another example of knobs-into-holes mutations for an antibody moiety is having R409D/K370E mutations in the CH3 domain of the "knob chain" and D399K/E357K mutations in the CH3 domain of the "hole chain" (Eu numbering).

In some embodiments, the antibody moiety in the prodrug comprises L234A and L235A ("LALA") mutations in its Fc domain. The LALA mutations eliminate complement binding and fixation as well as Fcy dependent ADCC (*see, e.g.,* Hezareh et al., J. Virol. (2001) 75(24):12161-8). In further embodiments, the LALA mutations are present in the antibody moiety in addition to the knobs-into-holes mutations.

In some embodiments, the antibody moiety comprises the M252Y/S254T/T256E ("YTE") mutations in the Fc domain. The YTE mutations allow the simultaneous modulation of serum half-life, tissue distribution and activity of IgG₁ (*see* Dall' Acqua et al., J Biol Chem. (2006) 281:23514-24; and Robbie et al., Antimicrob Agents Chemother. (2013) 57(12):6147-53). In further embodiments, the YTE mutations are present in the antibody moiety in addition to the knobs-into-holes mutations. In particular embodiments, the antibody moiety has YTE, LALA and knobs-into-holes mutations or any combination thereof.

The antigen-binding moiety may bind to an antigen on the surface of a cell, such as an immune cell, for example, T cells, NK cells, and macrophages. In other instances, the antigen-binding moiety may bind to a cytokine. For example, the antigen-binding moiety may be an antibody or antigen-binding fragment thereof that binds to PD-1, LAG-3, TIM-3, TIGIT, SIRPalpha, CTLA-4, CSF1R, NKG2A, NKG2D, CD16A, NKp30, NKp46, Fibroblast Activation Protein (FAP), the A1 domain of Tenascin-C (TNC A1), the A2 domain of Tenascin-C (TNC A2), the Extra Domain B of Fibronectin (EDB), PSA, 5T4, CD47, CMET, Claudin 6, CD24, or the Melanoma-associated Chondroitin Sulfate Proteoglycan (MCSP), or TGF-beta. The antibody may have the ability to activate the immune cell and enhance its anti-cancer activity.

The antigen-binding moiety may be an antibody or antigen-binding fragment thereof that binds to an antigen on the surface of a tumor cell. For example, the antigen-binding moiety may bind to FAP alpha, 5T4, Trop-2, PD-L1, HER-2, EGFR, Claudin 18.2, or carcinoembryonic antigen (CEA). The antibody may or may not have antibody-dependent cellular cytotoxicity (ADCC) activity. The antibody may also be conjugated to a cytotoxic drug.

In some embodiments, the antigen-binding moiety is an anti-PD1 antibody. In some embodiments, the anti-PD-1 antibody is pembrolizumab or nivolumab. In some embodiments, the antigen-binding moiety is an anti-CD8 antibody or a CD8 binding fragment thereof. In some embodiments, the antigen-binding moiety is an anti-NKG2A, NKG2D, CD16A, NKp30, NKP44, or NKP46 antibody or a binding fragment thereof.

In some embodiments, the PD-1-binding moiety includes an antibody or fragment thereof known in the art that binds to PD-1 and disrupts the interaction between the PD-1 and its ligand (PD-L1) to stimulate an anti-tumor immune response. In some embodiments, the antibody or antigen-binding portion thereof binds specifically to PD-1. For example, antibodies that target PD-1 and which can find use in the present invention include, but are not limited to, nivolumab (BMS-936558, Bristol-Myers Squibb), pembrolizumab (lambrolizumab, MK03475 or MK-3475, Merck), humanized anti-PD-1 antibody JS001 (ShangHai JunShi), monoclonal anti-PD-1 antibody TSR-042 (Tesaro, Inc.), pidilizumab (anti-PD-1 mAb CT-011, Medivation), anti-PD-1 monoclonal Antibody BGB-A317 (BeiGene), and/or anti-PD-1 antibody SHR-1210 (ShangHai HengRui), human monoclonal antibody REGN2810 (Regeneron), human monoclonal antibody MDX-1106 (Bristol-Myers Squibb), and/or humanized anti-PD-1 IgG4 antibody PDR001 (Novartis). In some embodiments, the PD-1 antibody is from clone: RMP1-14 (rat IgG)-BioXcell cat# BP0146. Other suitable anti-PD-1 antibodies include those disclosed in U.S. Pat. No. 8,008,449. In some embodiments, a PD-1 binding moiety comprises a single domain antibody or a nanobody. In some embodiments, said single domain antibody comprises the ones as disclosed in WO 2019/137541.

In some embodiments, the antigen-binding moiety binds to guanyl cyclase C (GCC), carbohydrate antigen 19-9 (CA19-9), glycoprotein A33 (gpA33), mucin 1 (MUC1), insulin-like growth factor 1 receptor (IGF1-R), human epidermal growth factor receptor 2 (HER2), human epidermal growth factor receptor 3 (HER3), delta-like protein 3 (DLL3), delta-like protein 4 (DLL4), epidermal growth factor receptor (EGFR), glypican-3 (GPC3), c-MET, vascular endothelial growth factor receptor 1 (VEGFR1), vascular endothelial growth factor receptor 2 (VEGFR2), Nectin-4, Liv-1, glycoprotein NMB (GPNMB), prostate specific membrane antigen (PSMA), Trop-2, carbonic anhydrase IX (CA9), endothelin B receptor (ETBR), six transmembrane epithelial antigen of the prostate 1 (STEAP1), folate receptor alpha (FR-α), SLIT and NTRK-like protein 6 (SLITRK6), carbonic anhydrase VI (CA6), ectonucleotide pyrophosphatase/phosphodiesterase family member 3 (ENPP3), mesothelin, trophoblast glycoprotein (TPBG), CD19, CD20, CD22, CD33, CD40, CD56, CD66e, CD70, CD74, CD79b, CD98, CD123, CD138, CD352, CD47, signal-regulatory protein alpha (SIRPα), Claudin 18.2, Claudin 6, BCMA, or EPCAM. In some embodiments, the antigen-binding moiety binds to an epidermal growth factor (EGF)-like domain of DLL3. In some embodiments, the antigen-binding moiety binds to a Delta/Serrate/Lag2 (DSL)-like domain of DLL3. In some embodiments, the antigen-binding moiety binds to an epitope located after the 374^{th} amino acid of GPC3. In some embodiments, the antigen-binding moiety binds to a heparin sulfate glycan of GPC3. In some embodiments, the antigen-binding moiety binds to Claudin 18.2 and does not bind to Claudin 18.1. In some embodiments, the antigen-binding moiety binds to Claudin 18.1 with at least 10 times weaker binding affinity than to Claudin 18.2.

Exemplary antigen-binding moieties include trastuzumab, rituximab, brentuximab, cetuximab, panitumumab, GC33 (or a humanized version thereof), anti-EGFR antibody mAb806 (or a humanized version thereof), anti-dPNAG antibody F598, and antigen-binding fragments thereof. In some embodiments, the antigen-binding moiety has at least 90%, 91%, 92%, 93%, 94%, 95%, 96%, 97%, 98%, or 99% identity to trastuzumab, rituximab, brentuximab, cetuximab, or panitumumab, GC33 (or a humanized version thereof), anti-EGFR antibody mAb806 (or a humanized version thereof), anti-dPNAG antibody F598, or a fragment thereof. In some embodiments, the antigen-binding moiety has an antibody heavy chain with at least 90%, 91%, 92%, 93%, 94%, 95%, 96%, 97%, 98%, or 99% identity to the antibody heavy chain of trastuzumab, rituximab, brentuximab, cetuximab, panitumumab, GC33 (or a humanized version thereof), anti-EGFR antibody mAb806 (or a humanized version thereof), anti-dPNAG antibody F598, or a fragment thereof. In some embodiments, the antigen-binding moiety has an antibody light chain with at least 90%, 91%, 92%, 93%, 94%, 95%, 96%, 97%, 98%, or 99% identity to the antibody light chain of trastuzumab, rituximab, brentuximab, cetuximab, panitumumab, GC33 (or a humanized version thereof), anti-EGFR antibody mAb806 (or a humanized version thereof), anti-dPNAG antibody F598, or a fragment thereof. In some embodiments, the antigen-binding moiety is fused to an IL-2 agonist polypeptide. In some embodiments, the antigen-binding moiety comprises the six complementarity-determining regions (CDRs) of trastuzumab, rituximab, brentuximab, cetuximab, panitumumab, GC33, anti-EGFR antibody mAb806, or anti-dPNAG antibody F598.

A number of CDR delineations are known in the art and are encompassed herein. A person of skill in the art can readily determine a CDR for a given delineation based on the sequence of the heavy or light chain variable region. The "Kabat" CDRs are based on sequence variability and are the most commonly used (Kabat et al., Sequences of Proteins of Immunological Interest, 5th Ed. Public Health Service, National Institutes of Health, Bethesda, Md. (1991)). "Chothia" CDRs refer to the location of the structural loops (Chothia & Lesk, Canonical structures for the hypervariable regions of immunoglobulins, J. Mol. Biol., vol. 196, pp. 901-917 (1987)). The "AbM" CDRs represent a compromise between the Kabat CDRs and Chothia structural loops, and are used by Oxford Molecular's AbM antibody modeling software. The "Contact" CDRs are based on an analysis of the available complex crystal structures. The residues from each of these CDRs are noted below in Table 3, in reference to common antibody numbering schemes. Unless otherwise specified herein, amino acid numbers in antibodies refer to the Kabat numbering scheme as described in Kabat et al., *supra,* including when CDR delineations are made in reference to Kabat, Chothia, AbM, or Contact schemes. Using this numbering system, the actual linear amino acid sequence may contain fewer or additional amino acids corresponding to a shortening of, or insertion into, a framework region (FR) or CDR of the variable domain. For example, a heavy chain variable domain may include a single amino acid insert (residue 52a according to Kabat) after residue 52 of H2 and inserted residues (e.g., residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

**Table 3. CDR Delineations According to Various Schemes**

| **CDR** | **Kabat** | **AbM** | **Chothia** | **Contact** |
|---|---|---|---|---|
| VL-CDR1 | L24-L34 | L24-L34 | L26-L32 | L30-L36 |
| VL-CDR2 | L50-L56 | L50-L56 | L50-L52 | L46-L55 |
| VL-CDR3 | L89-L97 | L89-L97 | L91-L96 | L89-L96 |
| VH-CDR1 (Kabat nos.) | H31-H35B | H26-H35B | H26-H32 | H30-H35B |
| VH-CDR1 (Chothia nos.) | H3 1-H35 | H26-H35 | H26-H32 | H30-H35 |
| VH-CDR2 | H50-H65 | H50-H58 | H53-H55 | H47-H58 |
| VH-CDR3 | H95-H102 | H95-H102 | H95-H101 | H93-H101 |

In some embodiments, the CDRs are "extended CDRs," and encompass a region that begins or terminates according to a different scheme. For example, an extended CDR can be as follows: L24-L36, L26-L34, or L26-L36 (VL-CDR1); L46-L52, L46-L56, or L50-L55 (VL-CDR2); L91-L97 (VL-CDR3); H47-H55, H47-H65, H50-H55, H53-H58, or H53-H65 (VH-CDR2); and/or H93-H102 (VH-CDR3).

In some embodiments, the antigen-binding moiety binds to HER2, and comprises a light chain having an amino acid sequence with at least 90%, 91%, 92%, 93%, 94%, 95%, 96%, 97%, 98%, or 99% identity to SEQ ID NO:126, or a fragment thereof, and a heavy chain having an amino acid sequence with at least 90%, 91%, 92%, 93%, 94%, 95%, 96%, 97%, 98%, or 99% identity to SEQ ID NO:127, or a fragment thereof. In some embodiments, the antigen-binding domain comprises CDR1, CDR2, and CDR3 from SEQ ID NO:126, and CDR1, CDR2, and CDR3 from SEQ ID NO:127.

In some embodiments, the antigen-binding moiety binds to CD20, and comprises a light chain having an amino acid sequence with at least 90%, 91%, 92%, 93%, 94%, 95%, 96%, 97%, 98%, or 99% identity to SEQ ID NO:128, or a fragment thereof, and a heavy chain having an amino acid sequence with at least 90%, 91%, 92%, 93%, 94%, 95%, 96%, 97%, 98%, or 99% identity to SEQ ID NO:129, or a fragment thereof. In some embodiments, the antigen-binding domain comprises CDR1, CDR2, and CDR3 from SEQ ID NO:128, and CDR1, CDR2, and CDR3 from SEQ ID NO:129.

In some embodiments, the antigen-binding moiety binds to CD30, and comprises a light chain having an amino acid sequence with at least 90%, 91%, 92%, 93%, 94%, 95%, 96%, 97%, 98%, or 99% identity to SEQ ID NO:130, or a fragment thereof, and a heavy chain having an amino acid sequence with at least 90%, 91%, 92%, 93%, 94%, 95%, 96%, 97%, 98%, or 99% identity to SEQ ID NO:131, or a fragment thereof. In some embodiments, the antigen-binding domain comprises CDR1, CDR2, and CDR3 from SEQ ID NO:130, and CDR1, CDR2, and CDR3 from SEQ ID NO:131.

In some embodiments, the antigen-binding moiety binds to EGFR, and comprises a light chain having an amino acid sequence with at least 90%, 91%, 92%, 93%, 94%, 95%, 96%, 97%, 98%, or 99% identity to SEQ ID NO:132, or a fragment thereof, and a heavy chain having an amino acid sequence with at least 90%, 91%, 92%, 93%, 94%, 95%, 96%, 97%, 98%, or 99% identity to SEQ ID NO:133, or a fragment thereof. In some embodiments, the antigen-binding domain comprises CDR1, CDR2, and CDR3 from SEQ ID NO:132, and CDR1, CDR2, and CDR3 from SEQ ID NO:133.

In some embodiments, the antigen-binding moiety binds to EGFR, and comprises a light chain having an amino acid sequence with at least 90%, 91%, 92%, 93%, 94%, 95%, 96%, 97%, 98%, or 99% identity to SEQ ID NO:134, or a fragment thereof, and a heavy chain having an amino acid sequence with at least 90%, 91%, 92%, 93%, 94%, 95%, 96%, 97%, 98%, or 99% identity to SEQ ID NO:135, or a fragment thereof. In some embodiments, the antigen-binding domain comprises CDR1, CDR2, and CDR3 from SEQ ID NO:134, and CDR1, CDR2, and CDR3 from SEQ ID NO:135.

In some embodiments, the antigen-binding moiety binds to c-MET, and comprises a light chain having an amino acid sequence with at least 90%, 91%, 92%, 93%, 94%, 95%, 96%, 97%, 98%, or 99% identity to SEQ ID NO:136, or a fragment thereof, and a heavy chain having an amino acid sequence with at least 90%, 91%, 92%, 93%, 94%, 95%, 96%, 97%, 98%, or 99% identity to SEQ ID NO:137, or a fragment thereof. In some embodiments, the antigen-binding domain comprises CDR1, CDR2, and CDR3 from SEQ ID NO:136, and CDR1, CDR2, and CDR3 from SEQ ID NO:137.

In some embodiments, the antigen-binding moiety binds to GPC3, and comprises a light chain having an amino acid sequence with at least 90%, 91%, 92%, 93%, 94%, 95%, 96%, 97%, 98%, or 99% identity to SEQ ID NO:138, or a fragment thereof, and a heavy chain having an amino acid sequence with at least 90%, 91%, 92%, 93%, 94%, 95%, 96%, 97%, 98%, or 99% identity to SEQ ID NO:139, or a fragment thereof. In some embodiments, the antigen-binding domain comprises CDR1, CDR2, and CDR3 from SEQ ID NO:138, and CDR1, CDR2, and CDR3 from SEQ ID NO:139.

In some embodiments, the antigen-binding moiety binds to Claudin 18.2, and comprises a light chain having an amino acid sequence with at least 90%, 91%, 92%, 93%, 94%, 95%, 96%, 97%, 98%, or 99% identity to SEQ ID NO:140, or a fragment thereof, and a heavy chain having an amino acid sequence with at least 90%, 91%, 92%, 93%, 94%, 95%, 96%, 97%, 98%, or 99% identity to SEQ ID NO:141, or a fragment thereof. In some embodiments, the antigen-binding domain comprises CDR1, CDR2, and CDR3 from SEQ ID NO:140, and CDR1, CDR2, and CDR3 from SEQ ID NO:141.

In some embodiments, the antigen-binding moiety binds to FAP alpha, and comprises a light chain having an amino acid sequence with at least 90%, 91%, 92%, 93%, 94%, 95%, 96%, 97%, 98%, or 99% identity to SEQ ID NO:158 or 159, or a fragment thereof, and a heavy chain having an amino acid sequence with at least 90%, 91%, 92%, 93%, 94%, 95%, 96%, 97%, 98%, or 99% identity to SEQ ID NO:160, or a fragment thereof. In some embodiments, the antigen-binding domain comprises CDR1, CDR2, and CDR3 from SEQ ID NO:158 or 159, and CDR1, CDR2, and CDR3 from SEQ ID NO:160. In some embodiments, the antigen-binding moiety binds to FAP alpha, and comprises a light chain variable domain having an amino acid sequence with at least 90%, 91%, 92%, 93%, 94%, 95%, 96%, 97%, 98%, or 99% identity to SEQ ID NO:161, and a heavy chain variable domain having an amino acid sequence with at least 90%, 91%, 92%, 93%, 94%, 95%, 96%, 97%, 98%, or 99% identity to SEQ ID NO:162. In some embodiments, the antigen-binding domain comprises CDR1, CDR2, and CDR3 from SEQ ID NO:161, and CDR1, CDR2, and CDR3 from SEQ ID NO:162. In particular embodiments, the humanized FAP antibody comprises a light chain amino acid sequence shown in SEQ ID NO:158 or 159 and a heavy chain amino acid sequence shown in SEQ ID NO:160.

In some embodiments, the antigen-binding moiety binds to carcinoembryonic antigen (CEA) and may be derived from antibody PR1A3 (U.S. Pat. 8,642,742). The anti-CEA antibody, and comprises a light chain having an amino acid sequence with at least 90%, 91%, 92%, 93%, 94%, 95%, 96%, 97%, 98%, or 99% identity to SEQ ID NO:156, or a fragment thereof, and a heavy chain having an amino acid sequence with at least 90%, 91%, 92%, 93%, 94%, 95%, 96%, 97%, 98%, or 99% identity to SEQ ID NO:157, or a fragment thereof. In some embodiments, the antigen-binding domain comprises CDR1, CDR2, and CDR3 from SEQ ID NO:154, and CDR1, CDR2, and CDR3 from SEQ ID NO:155. In certain embodiments, the PR1A3 antibody is a humanized antibody comprising a light chain variable domain amino acid sequence shown in SEQ ID NO:156 and a heavy chain variable domain amino acid sequence shown in SEQ ID NO:157.

In some embodiments, the antigen-binding moiety binds to PDL1, and comprises a light chain having an amino acid sequence with at least 90%, 91%, 92%, 93%, 94%, 95%, 96%, 97%, 98%, or 99% identity to SEQ ID NO:167, or a fragment thereof, and a heavy chain having an amino acid sequence with at least 90%, 91%, 92%, 93%, 94%, 95%, 96%, 97%, 98%, or 99% identity to SEQ ID NO:168, or a fragment thereof. In some embodiments, the antigen-binding domain comprises CDR1, CDR2, and CDR3 from SEQ ID NO:167, and CDR1, CDR2, and CDR3 from SEQ ID NO:168.

In some embodiments, the antigen-binding moiety binds to 5T4, and comprises a light chain variable domain having an amino acid sequence with at least 90%, 91%, 92%, 93%, 94%, 95%, 96%, 97%, 98%, or 99% identity to SEQ ID NO: 165 or 166, and a heavy chain variable domain having an amino acid sequence with at least 90%, 91%, 92%, 93%, 94%, 95%, 96%, 97%, 98%, or 99% identity to SEQ ID NO:163 or 164, or a fragment thereof. In some embodiments, the antigen-binding domain comprises CDR1, CDR2, and CDR3 from SEQ ID NO:165 or 166, and CDR1, CDR2, and CDR3 from SEQ ID NO:163 or 164.

In some embodiments, the antigen-binding moiety binds to Trop-2, and comprises a light chain variable region comprising a CDR1 comprising an amino acid sequence of KASQDVSIAVA (SEQ ID NO:142), a CDR2 comprising an amino acid sequence of SASYRYT (SEQ ID NO: 143), and a CDR3 comprising an amino acid sequence of QQHYITPLT (SEQ ID NO:144); and a heavy chain variable region comprising a CDR1 comprising an amino acid sequence of NYGMN (SEQ ID NO:145), a CDR2 comprising an amino acid sequence of WINTYTGEPTYTDDFKG (SEQ ID NO:146), and a CDR3 comprising an amino acid sequence of GGFGSSYWYFDV (SEQ ID NO:147).

In some embodiments, the antigen-binding moiety binds to mesothelin, and comprises light chain variable region comprising a CDR1 comprising an amino acid sequence of SASSSVSYMH (SEQ ID NO:148), a CDR2 comprising an amino acid sequence of DTSKLAS (SEQ ID NO: 149), and a CDR3 comprising an amino acid sequence of QQWSGYPLT (SEQ ID NO:150); and a heavy chain variable region comprising a CDR1 comprising an amino acid sequence of GYTMN (SEQ ID NO: 151), a CDR2 comprising an amino acid sequence of LITPYNGASSYNQKFRG (SEQ ID NO:152), and a CDR3 comprising an amino acid sequence of GGYDGRGFDY (SEQ ID NO:153).

In some embodiments, the antigen-binding moiety comprises one, two, or three antigen-binding domains. For example, the antigen-binding moiety is bispecific and binds to two different antigens selected from the group consisting of HER2, HER3, EGFR, 5T4, FAP alpha, Trop-2, GPC3, VEGFR2, Claudin 18.2 and PD-L1. In some embodiments, said bispecific antigen-binding moiety binds to two different epitopes of HER2.

### 2. Other Carrier Moieties

Other non-antigen-binding carrier moieties may be used for the present prodrugs. For example, an antibody Fc domain (e.g., a human IgG₁, IgG₂, IgG₃, or IgG₄ Fc), a polymer (e.g., PEG), an albumin (e.g., a human albumin) or a fragment thereof, or a nanoparticle can be used.

By way of example, the cytokine agonist polypeptide and its antagonist may be fused to an antibody Fc domain, forming an Fc fusion protein. In some embodiments, the cytokine agonist polypeptide is fused (directly or through a peptide linker) to the C-terminus or N-terminus of one of the Fc domain polypeptide chains, and the cytokine mask is fused to the C-terminus or N-terminus of the other Fc domain polypeptide chain through a cleavable or non-cleavable peptide linker, wherein the two Fc domain polypeptide chains contain mutations that allow their specific pairing. In some embodiments, the Fc domain comprises the knobs-into-holes mutations described above. In further embodiments, the Fc domain may comprise also the YTE and/or LALA mutations described above.

The carrier moiety of the prodrug may comprise an albumin (e.g., human serum albumin) or a fragment thereof. An exemplary sequence of albumin is shown in SEQ ID NO: 124. In some embodiments, the albumin or albumin fragment is about 85% or more, about 90% or more, about 91% or more, about 92% or more, about 93% or more, about 94% or more, about 95% or more, about 96% or more, about 97% or more, about 98% or more, about 99% or more, about 99.5% or more, or about 99.8% or more identical to human serum albumin or a fragment thereof.

In some embodiments, the carrier moiety comprises an albumin fragment (e.g., a human serum albumin fragment) that is about 10 or more, 20 or more, 30 or more 40 or more, 50 or more, 60 or more, 70 or more, 80 or more, 90 or more, 100 or more, 120 or more, 140 or more, 160 or more, 180 or more, 200 or more, 250 or more, 300 or more, 350 or more, 400 or more, 450 or more, 500 or more, or 550 or more amino acids in length. In some embodiments, the albumin fragment is between about 10 amino acids and about 584 amino acids in length (such as between about 10 and about 20, about 20 and about 40, about 40 and about 80, about 80 and about 160, about 160 and about 250, about 250 and about 350, about 350 and about 450, or about 450 and about 550 amino acids in length). In some embodiments, the albumin fragment includes the Sudlow I domain or a fragment thereof, or the Sudlow II domain or the fragment thereof.

### D. Linker Components of the Prodrugs

The IL-2 agonist polypeptide may be fused to the carrier moiety with or without a peptide linker. The peptide linker may be non-cleavable. In particular embodiments, the peptide linker comprises the amino acid sequence GGS (SEQ ID NO: 177), GGGGS (SEQ ID NO: 178), GGSGGS (SEQ ID NO: 179), GGGSGGGGS (SEQ ID NO: 180), GGGGSGGGGSGGGGS (SEQ ID NO:181), GGGGSAAGGGGAGGGGA (SEQ ID NO:182), or GGGGSGGGGSAAGGGGSGGGGS (SEQ ID NO:183).

The IL-2 mask may be fused to the cytokine moiety or to the carrier through a cleavable linker. The cleavable linker may contain one or more (e.g., two or three) cleavable moieties (CM). Each CM may be a substrate for an enzyme or protease selected from legumain, plasmin, TMPRSS-3/4, MMP-2, MMP-9, MT1-MMP, cathepsin, caspase, human neutrophil elastase, beta-secretase, uPA, and PSA. Examples of cleavable linkers include, without limitation, those comprising an amino acid sequence selected from SEQ ID NOs:55-124.

Specific, nonlimiting examples of cytokine agonist polypeptides, cytokine masks, carriers, peptide linkers, and prodrugs are shown in the Sequences section below. Further, the prodrugs and novel IL-2 muteins of the present disclosure may be made by well-known recombinant technology. For example, one or more expression vectors comprising the coding sequences for the polypeptide chains of the prodrugs may be transfected into mammalian host cells (e.g., CHO cells), and cells are cultured under conditions that allow the expression of the coding sequences and the assembly of the expressed polypeptides into the prodrug complex. In order for the prodrug to remain inactive, the host cells that express no or little uPA, MMP-2 and/or MMP-9 may be used. In some embodiments, the host cells may contain null mutations (knockout) of the genes for these proteases.

### II. Examples of the Prodrugs

In some embodiments, the prodrugs presented here comprise an antibody fused with one or two of the above said mutant IL-2 polypeptides.

In some embodiments, the prodrug comprises one mutant IL-2 polypeptide. By way of example, the prodrug comprises two identical light chains with an amino acid sequence of SEQ ID NO:38 or an amino acid sequence at least 90% identical thereto, a first heavy chain polypeptide comprising an amino acid sequence of SEQ ID NO:39 or an amino acid sequence at least 90% identical thereto, and a second heavy chain polypeptide chain of amino acid sequence of SEQ ID NO:40 or 41, or an amino acid sequence at least 90% identical thereto.

In some embodiments, the prodrug comprises one mutant IL-2 polypeptide. By way of example, the prodrug comprises two identical light chains with an amino acid sequence of SEQ ID NO:38 or an amino acid sequence at least 90% identical thereto, a first heavy chain polypeptide comprising an amino acid sequence of SEQ ID NO:47 or an amino acid sequence at least 90% identical thereto, and a second heavy chain polypeptide chain of amino acid sequence of SEQ ID NO:45 or 46, or an amino acid sequence at least 90% identical thereto.

In some embodiments, the prodrug comprises two mutant IL-2 polypeptides. By way of example, the prodrug comprises two identical light chains with an amino acid sequence of SEQ ID NO:38 or an amino acid sequence at least 90% identical thereto, and two identical heavy chain polypeptide chains of amino acid sequence of SEQ ID NO:40 or 41, or an amino acid sequence at least 90% identical thereto.

By way of example, the prodrug comprises two identical light chains with an amino acid sequence of SEQ ID NO:38 or an amino acid sequence at least 90% identical thereto, a first heavy chain polypeptide comprising an amino acid sequence of SEQ ID NO:42, 43 or 44, or an amino acid sequence at least 90% identical thereto, and a second heavy chain polypeptide chain of amino acid sequence of SEQ ID NO:40, 41, or 125, or an amino acid sequence at least 90% identical thereto.

By way of example, the prodrug comprises two identical light chains with an amino acid sequence of SEQ ID NO:38 or an amino acid sequence at least 90% identical thereto, and two identical heavy chain polypeptide chains of amino acid sequence of SEQ ID NO:48, 49, 50 or 51 or an amino acid sequence at least 90% identical thereto.

### III. Pharmaceutical Compositions

Pharmaceutical compositions comprising the prodrugs and muteins (i.e., the active pharmaceutical ingredient or API) of the present disclosure may be prepared by mixing the API having the desired degree of purity with one or more optional pharmaceutically acceptable excipients (*see, e.g.,* Remington's Pharmaceutical Sciences, 16th Edition., Osol, A. Ed. (1980)) in the form of lyophilized formulations or aqueous solutions. Pharmaceutically acceptable excipients (or carriers) are generally nontoxic to recipients at the dosages and concentrations employed, and include, but are not limited to: buffers containing, for example, phosphate, citrate, succinate, histidine, acetate, or another inorganic or organic acid or salt thereof; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including sucrose, glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffers are used to control the pH in a range which optimizes the therapeutic effectiveness, especially if stability is pH dependent. Buffers are preferably present at concentrations ranging from about 50 mM to about 250 mM. Suitable buffering agents for use with the present invention include both organic and inorganic acids and salts thereof, such as citrate, phosphate, succinate, tartrate, fumarate, gluconate, oxalate, lactate, and acetate. Additionally, buffers may comprise histidine and trimethylamine salts such as Tris.

Preservatives are added to retard microbial growth, and are typically present in a range from 0.2% - 1.0% (w/v). Suitable preservatives for use with the present invention include octadecyldimethylbenzyl ammonium chloride; hexamethonium chloride; benzalkonium halides (e.g., chloride, bromide, iodide), benzethonium chloride; thimerosal, phenol, butyl or benzyl alcohol; alkyl parabens such as methyl or propyl paraben; catechol; resorcinol; cyclohexanol, 3-pentanol, and m-cresol.

Tonicity agents, sometimes known as "stabilizers" are present to adjust or maintain the tonicity of liquid in a composition. When used with large, charged biomolecules such as proteins and antibodies, they are often termed "stabilizers" because they can interact with the charged groups of the amino acid side chains, thereby lessening the potential for inter- and intra-molecular interactions. Tonicity agents can be present in any amount between 0.1% to 25% by weight, or more preferably between 1% to 5% by weight, considering the relative amounts of the other ingredients. Preferred tonicity agents include polyhydric sugar alcohols, preferably trihydric or higher sugar alcohols, such as glycerin, erythritol, arabitol, xylitol, sorbitol and mannitol.

Non-ionic surfactants or detergents (also known as "wetting agents") are present to help solubilize the therapeutic agent as well as to protect the therapeutic protein against agitation-induced aggregation, which also permits the formulation to be exposed to shear surface stress without causing denaturation of the active therapeutic protein or antibody. Non-ionic surfactants are present in a range of about 0.05 mg/ml to about 1.0 mg/ml, preferably about 0.07 mg/ml to about 0.2 mg/ml.

Suitable non-ionic surfactants include polysorbates (20, 40, 60, 65, 80, etc.), polyoxamers (184, 188, etc.), PLURONIC^{®} polyols, TRITON^{®}, polyoxyethylene sorbitan monoethers (TWEEN^{®}-20, TWEEN^{®}-80, etc.), lauromacrogol 400, polyoxyl 40 stearate, polyoxyethylene hydrogenated castor oil 10, 50 and 60, glycerol monostearate, sucrose fatty acid ester, methyl cellulose and carboxymethyl cellulose. Anionic detergents that can be used include sodium lauryl sulfate, dioctyle sodium sulfosuccinate and dioctyl sodium sulfonate. Cationic detergents include benzalkonium chloride or benzethonium chloride.

The choice of pharmaceutical carrier, excipient or diluent may be selected with regard to the intended route of administration and standard pharmaceutical practice. Pharmaceutical compositions may additionally comprise any suitable binder(s), lubricant(s), suspending agent(s), coating agent(s) or solubilizing agent(s).

There may be different composition/formulation requirements dependent on the different delivery systems. By way of example, pharmaceutical compositions useful in the present invention may be formulated to be administered using a mini-pump or by a mucosal route, for example, as a nasal spray or aerosol for inhalation or ingestible solution, or parenterally in which the composition is formulated by an injectable form, for delivery, by, for example, an intravenous, intramuscular or subcutaneous route.

In some embodiments, the pharmaceutical composition of the present disclosure is a lyophilized protein formulation. In other embodiments, the pharmaceutical composition may be an aqueous liquid formulation.

### IV. Methods of Treatment

The prodrugs and novel IL-2 muteins (mutant IL-2 polypeptides) of the present invention can be used to treat a disease. In some embodiments, the prodrugs or mutant IL-2 polypeptides are used to treat cancer. In some embodiments, the prodrugs or mutant IL-2 polypeptides are used to treat an infection, for example, when the drug molecule is an antibacterial agent or an antiviral agent.

In some embodiments, the method of treating a disease (such as cancer, a viral infection, or a bacterial infection) in a subject comprises administering to the subject an effective amount of the prodrugs or mutant IL-2 polypeptides disclosed herein.

In some embodiments, the cancer is a solid cancer. In some embodiments, the cancer is a blood cancer or a solid tumor. Exemplary cancers that may be treated include, but are not limited to, leukemia, lymphoma, kidney cancer, bladder cancer, urinary tract cancer, cervical cancer, brain cancer, head and neck cancer, skin cancer, uterine cancer, testicular cancer, esophageal cancer, liver cancer, colorectal cancer, stomach cancer, squamous cell carcinoma, prostate cancer, pancreatic cancer, lung cancer such as non-small cell lung cancer, cholangiocarcinoma, breast cancer, and ovarian cancer, and medullary thyroid cancer.

In some embodiments, the prodrugs or mutant IL-2 polypeptides are used to treat a bacterial infection such as sepsis. In some embodiments, the bacteria causing the bacterial infection are drug-resistant bacteria. In some embodiments, the antigen-binding moiety (carrier moiety) disclosed herein binds to a bacterial antigen.

In some embodiments, the prodrugs or mutant IL-2 polypeptides are used to treat a viral infection. In some embodiments, the virus causing the viral infection is hepatitis C (HCV), hepatitis B (HBV), human immunodeficiency virus (HIV), a human papilloma virus (HPV). In some embodiments, the antigen-binding moiety disclosed herein binds to a viral antigen.

Generally, dosages and routes of administration of the present pharmaceutical compositions are determined according to the weight and conditions of the subject, according to standard pharmaceutical practice. In some embodiments, the pharmaceutical composition is administered to a subject through any route, including orally, transdermally, by inhalation, intravenously, intra-arterially, intramuscularly, direct application to a wound site, application to a surgical site, intraperitoneally, by suppository, subcutaneously, intradermally, transcutaneously, by nebulization, intrapleurally, intraventricularly, intra-articularly, intraocularly, intracranially, or intraspinally. In some embodiments, the composition is administered to a subject intravenously.

In some embodiments, the dosage of the pharmaceutical composition is a single dose or a repeated dose. In some embodiments, the doses are given to a subject once per day, twice per day, three times per day, or four or more times per day. In some embodiments, about 1 or more (such as about 2, 3, 4, 5, 6, or 7 or more) doses are given in a week. In some embodiments, the pharmaceutical composition is administered weekly, once every 2 weeks, once every 3 weeks, once every 4 weeks, weekly for two weeks out of 3 weeks, or weekly for 3 weeks out of 4 weeks. In some embodiments, multiple doses are given over the course of days, weeks, months, or years. In some embodiments, a course of treatment is about 1 or more doses (such as about 2, 3, 4, 5, 7, 10, 15, or 20 or more doses).

Unless otherwise defined herein, scientific and technical terms used in connection with the present disclosure shall have the meanings that are commonly understood by those of ordinary skill in the art. Exemplary methods and materials are described below, although methods and materials similar or equivalent to those described herein can also be used in the practice or testing of the present disclosure. In case of conflict, the present specification, including definitions, will control. Further, unless otherwise required by context, singular terms shall include pluralities and plural terms shall include the singular. Throughout this specification and embodiments, the words "have" and "comprise," or variations such as "has," "having," "comprises," or "comprising," will be understood to imply the inclusion of a stated integer or group of integers but not the exclusion of any other integer or group of integers. All publications and other references mentioned herein are incorporated by reference in their entirety. Although a number of documents are cited herein, this citation does not constitute an admission that any of these documents forms part of the common general knowledge in the art. As used herein, the term "approximately" or "about" as applied to one or more values of interest refers to a value that is similar to a stated reference value. In certain embodiments, the term refers to a range of values that fall within 10%, 9%, 8%, 7%, 6%, 5%, 4%, 3%, 2%, 1%, or less in either direction (greater than or less than) of the stated reference value unless otherwise stated or otherwise evident from the context.

According to the present disclosure, back-references in the dependent claims are meant as short-hand writing for a direct and unambiguous disclosure of each and every combination of claims that is indicated by the back-reference. Further, headers herein are created for ease of organization and are not intended to limit the scope of the claimed invention in any manner.

### V. Exemplary Embodiments

Further particular embodiments of the present disclosure are described as follows. These embodiments are intended to illustrate the compositions and methods described in the present disclosure and are not intended to limit the scope of the present disclosure.
1. A mutant human interleukin-2 (IL-2) polypeptide comprising a mutation L36I (numbering according to SEQ ID NO: 1); wherein said IL-2 polypeptide comprises an amino acid sequence at least 90% identical as the sequence set forth in SEQ ID NO: 1.
2. The mutant human interleukin-2 polypeptide of embodiment 1, wherein the mutant IL-2 further comprises a second mutation C125A (numbering according SEQ ID NO:1).
3. The mutant human interleukin-2 polypeptide of embodiment 1 or 2, wherein the mutant IL-2 comprises one or more additional amino acid mutations that abolish or reduce affinity of the mutant IL-2 polypeptide to the high-affinity IL-2 receptor and preserves affinity of the mutant IL-2 polypeptide to the intermediate-affinity IL-2 receptor, each compared to a wildtype IL-2 polypeptide.
4. The mutant interleukin-2 polypeptide of embodiment 3, wherein said additional amino acid mutation(s) is at a position selected from the positions corresponding to residue 35, 38, 42, 43, 45, 62, 68, and 72 (numbering according to SEQ ID NO:1).
5. The mutant interleukin-2 polypeptide of embodiment 1, 2, 3 or 4, wherein the mutant IL-2 further comprises one or more mutations at a position or positions selected from residue 88, 91, 92, and 126 (numbering according to SEQ ID NO:1).
6. The mutant interleukin-2 polypeptide of embodiment 3, 4, or 5, wherein said additional amino acid mutation or mutations are selected from the group of R38A, R38K, R38S, F42A, F42G, F42S, F42T, F42Q, F42E, F42N, F42D, F42R, F42K, Y45A, Y45G, Y45S, Y45T, Y45Q, Y45E, Y45N, Y45D, Y45R, Y45K, E62A, E62L, E62I, E68A, E68V, L72G, L72A, L72S, L72T, L72Q, L72E, L72N, L72D, L72R, L72K, N88D, N88E, N88F, N88H, N88K, N88L, N88M, N88S, N88T, N88V, N88W, N88Y, N88A, V91K, Q126E, Q126A, Q126D, Q126F, Q126G, Q126H, Q126I, Q126K, Q126L, Q126P, Q126S, Q126T, Q126W and Q126Y (numbering according to SEQ ID NO:1).
7. The mutant interleukin-2 polypeptide of any one of embodiments 1 to 6, wherein the mutant IL-2 further comprises an amino acid mutation which eliminates the O-glycosylation site of IL-2 at a position corresponding to residue 3 of human IL-2 (numbering according to SEQ ID NO:1).
8. The mutant interleukin-2 polypeptide of embodiment 1, wherein said mutant IL-2 polypeptide comprises an amino acid sequence selected from SEQ ID NOs:2-33.
9. The mutant interleukin-2 polypeptide of any one of embodiments 1 to 8, wherein said mutant IL-2 polypeptide is linked to a carrier.
10. The mutant interleukin-2 polypeptide of embodiment 9, wherein said carrier comprises an antigen binding moiety.
11. An immunoconjugate comprising a mutant IL-2 polypeptide according to any one of embodiments 1 to 8 and an antigen binding moiety.
12. The immunoconjugate of embodiment 11, wherein said immunoconjugate comprises a first and a second antigen binding moiety.
13. The immunoconjugate of any one of embodiments 11 and 12, wherein said antigen binding moiety is an antibody or an antibody fragment.
14. The immunoconjugate of embodiment 11 or 12, wherein said antigen binding moiety is selected from a Fab molecule and a scFv molecule.
15. The immunoconjugate of any one of embodiments 11 and 12, wherein said antigen binding moiety is an immunoglobulin molecule, particularly an IgG molecule.
16. The immunoconjugate of any one of embodiments 11 to 15, wherein said antigen binding moiety is directed to an antigen presented on a tumor cell or in a tumor cell environment.
17. The immunoconjugate of any one of embodiments 11 to 15, wherein said antigen binding moiety is directed to an antigen presented on an immune cell.
18. The immunoconjugate of any one of embodiments 11 to 15, wherein said antigen binding moiety is directed to an antigen presented on a T cell, an NK cell, or a macrophage.
19. The immunoconjugate of embodiment 16, wherein said antigen is selected from the group of Fibroblast Activation Protein (FAP), the A1 domain of Tenascin-C (TNC A1), the A2 domain of Tenascin-C (TNC A2), the Extra Domain B of Fibronectin (EDB), Carcinoembryonic Antigen (CEA), PSA, 5T4, PDL1, CD47, HER2, CD20, CD38, BCMA, EGFR, CMET, Claudin 18.2, Claudin 6, CD24, and the Melanoma-associated Chondroitin Sulfate Proteoglycan (MCSP).
20. The immunoconjugate of embodiment 17 or 18, wherein said antigen is selected from the group of PD-1, PDL1, CD8, Tim-3, LAG-3, TIGIT, SIRPalpha, CTLA-4, CSF1R, NKG2A, NKG2D, CD16A, NKp30, and NKp46.
21. The immunoconjugate of embodiment 12, wherein said antigens are selected from the group of PD-1, CD8, Tim-3, LAG-3, TIGIT, SIRPalpha, CTLA-4, CSF1R, NKG2A, NKG2D, CD16A, NKp30, NKp46, Fibroblast Activation Protein (FAP), the A1 domain of Tenascin-C (TNC A1), the A2 domain of Tenascin-C (TNC A2), the Extra Domain B of Fibronectin (EDB), Carcinoembryonic Antigen (CEA), PSA, 5T4, PDL1, CD47, EGFR, CMET, Claudin 18.2, Claudin 6, CD24, and the Melanoma-associated Chondroitin Sulfate Proteoglycan (MCSP).
22. The mutant IL-2 polypeptide or immunoconjugate of any one of embodiments 1 to 21, wherein said IL-2 polypeptide is masked.
23. The mutant IL-2 polypeptide or immunoconjugate of any one of embodiments 1 to 21, wherein said IL-2 polypeptide is masked by a masking moiety, wherein said masking moiety comprises an extracellular domain (ECD) of IL-2 receptor beta (IL-2Rβ) or a functional fragment thereof.
24. The mutant IL-2 polypeptide or immunoconjugate of embodiment 23, wherein said IL-2Rβ-ECD comprises an amino acid sequence of SEQ ID NO:37 or at least 90% identical as that of SEQ ID NO:37.
25. The mutant IL-2 polypeptide or immunoconjugate of any one of embodiments 1 to 21, wherein said IL-2 polypeptide is masked by a masking moiety, wherein said masking moiety comprises a scFv or a Fab, wherein said scFv or Fab binds to said IL-2 polypeptide and inhibits a biological activity of said IL-2 polypeptide.
26. The mutant IL-2 polypeptide or immunoconjugate of embodiment 23, wherein said masking moiety is an scFv, which comprises an amino acid sequence of SEQ ID NO:34, 35, or 36 or at least 90% identical as that of SEQ ID NO:34, 35, or 36.
27. The mutant IL-2 polypeptide or immunoconjugate of embodiment 23, wherein said masking moiety comprises an scFv or Fab, which comprises the same heavy chain CDRs and the same light chain CDRs as that of the scFv with an amino acid sequence of SEQ ID NO:34, 35, or 36.
28. The mutant IL-2 polypeptide or immunoconjugate of any one of embodiments 23 to 27, wherein the masking moiety further comprises a cleavable peptide linker.
29. The immunoconjugate of embodiment 17 or 28, wherein said antigen-binding moiety is an antibody against PD-1 or the binding fragment thereof; and wherein said anti-PD-1 antibody is selected from nivolumab and pembrolizumab.
30. An immune conjugate comprising two identical light chains and a first heavy chain polypeptide chain and a second heavy chain polypeptide chain; wherein said light chain comprises an amino acid sequence of SEQ ID NO:38 or at least 90% identical as that of SEQ ID NO:38; said first heavy chain polypeptide chain comprises an amino acid sequence selected from SEQ ID NOs:40, 41, 44, and 45 or at least 95% identical as that of SEQ ID NO:40, 41, 44, or 45; and said second heavy chain polypeptide chain comprises an amino acid sequence of SEQ ID NOs:42, 43, 46 and 47 or at least 95% identical as that of SEQ ID NO:42, 43, 46, or 47.
31. An immune conjugate comprising two identical light chains and two identical heavy chain polypeptide chains; wherein said light chain comprises an amino acid sequence of SEQ ID NO:38 or at least 90% identical as that of SEQ ID NO:38; said heavy chain polypeptide chain comprises an amino acid sequence selected from SEQ ID NOs:40, 41, 48, 49, 50, and 51 or at least 95% identical as that of SEQ ID NO:40, 41, 48, 49, 50 or 51.
32. The immunoconjugate of embodiment 17 or 28, wherein said antigen-binding moiety is an antibody against CD8 or the binding fragment thereof; and wherein said anti-CD8 antibody is OKT8 or humanized OKT8, or comprises the same heavy chain CDRs and the same light chain CDRs as derived from OKT8.
33. The immunoconjugate of embodiment 32, wherein the antigen-binding moiety further comprises an additional antigen-binding moiety that binds an antigen on a tumor cell or in a tumor cell environment.
34. The immunoconjugate of embodiment 33, wherein said tumor associated antigen is selected from the group of Fibroblast Activation Protein (FAP), the A1 domain of Tenascin-C (TNC A1), the A2 domain of Tenascin-C (TNC A2), the Extra Domain B of Fibronectin (EDB), Carcinoembryonic Antigen (CEA), PSA, 5T4, PDL1, CD47, HER2, CD20, CD38, BCMA, EGFR, CMET, Claudin 18.2, Claudin 6, CD24, and the Melanoma-associated Chondroitin Sulfate Proteoglycan (MCSP).
35. An isolated polynucleotide encoding the mutant IL-2 polypeptide or immunoconjugate of any one of embodiments 1 to 34.
36. An expression vector comprising the polynucleotide of embodiment 35.
37. A host cell comprising the polynucleotide of embodiment 35 or the expression vector of embodiment 36.
38. A method of producing a mutant IL-2 polypeptide or an immunoconjugate thereof, comprising culturing the host cell of embodiment 37 under conditions suitable for the expression of the mutant IL-2 polypeptide or the immunoconjugate.
39. A mutant IL-2 polypeptide or immunoconjugate produced by the method of embodiment 38.
40. A pharmaceutical composition comprising the mutant IL-2 polypeptide or immunoconjugate of any one of embodiments 1 to 34 or 39 and a pharmaceutically acceptable carrier.
41. The mutant IL-2 polypeptide or immunoconjugate of any one of embodiments 1 to 34 or 39 for use in the treatment of a disease in an individual in need thereof.
42. The mutant IL-2 polypeptide or immunoconjugate of embodiment 41, wherein said disease is cancer.
43. Use of the mutant IL-2 polypeptide or immunoconjugate of any one of embodiments 1 to 34 or 39 for manufacture of a medicament for treating a disease in an individual in need thereof.
44. A method of treating disease in an individual, comprising administering to said individual a therapeutically effective amount of a composition comprising the mutant IL-2 polypeptide or immunoconjugate of any one of embodiments 1 to 34 or 39 in a pharmaceutically acceptable form.
45. The method of embodiment 44, wherein said disease is cancer.
46. A method of stimulating the immune system of an individual, comprising administering to said individual an effective amount of a composition comprising the mutant IL-2 polypeptide or immunoconjugate of any one of embodiments 1 to 34 or 39 in a pharmaceutically acceptable form.
47. The mutant IL-2 polypeptide or immunoconjugate of any one of embodiments 1 to 34 or 39, which has reduced risk of immunogenicity when dosed in an animal or a patient comparing to that of the corresponding IL-2 polypeptide or immunoconjugate which comprises amino acid L at position 36 (numbering according to SEQ ID NO:1).
48. The immunoconjugate of embodiment 32, wherein said CD8 antibody comprises a light chain variable domain of SEQ ID NO:52, or at least 90% identical as that of SEQ ID NO:52, and a heavy chain variable domain of SEQ ID NO:53 or 54 or at least 90% identical to a sequence set forth in SEQ ID NO:53 or 54.
49. The immunoconjugate of embodiment 28, 29, 32, 33, 34, or 48, wherein said immunoconjugate comprises one or more cleavable peptide linkers; wherein said cleavable linker comprises an amino acid sequence selected from SEQ ID NOs:55-124.

In order that this invention may be better understood, the following examples are set forth. These examples are for purposes of illustration only and are not to be construed as limiting the scope of the invention in any manner.

### EXAMPLES

### Example 1: Discovery of IL-2 Mutant Polypeptides with Reduced Immunogenicity

Several human IL-2 variants were made, including IL-2V1/L53I, IL-2V1/L56I, IL-2V1/Y45G, IL-2V1/Y45G/L53I, IL-2V1/Y45G/L56I, IL-2V1/L70I, IL-2V1/L72I, IL-2V1/Y45G/L80I, and IL-2V1/Y45G/L118I (all mutations relative to SEQ ID NO:1). None of these mutations removed the immunogenic "hot spots" introduced by the "non-alpha" mutations (data not shown). Surprisingly, introduction of mutation L36I removed most of the "hot spots" introduced by the "non-alpha" mutations (Table 4).

**Table 4. MHC Class II Molecules Binding Prediction of IL-2V1 (SEQ ID NO: 176) with Mutation L36I (IL-2V1/L36I) Using T cell Epitope Prediction Tools**

| MHC Class II Molecule | IL-2V1/ L36I | smm_align_IC₅₀ | SEQ ID NO |
|---|---|---|---|
| | smm_align_core | | |
| HLA-DRB1*13:02 | LISNINVIV | 9 | 289 |
| HLA-DRB1*13:02 | LISNINVIV | 9 | 289 |
| HLA-DRB1*13:02 | LISNINVIV | 10 | 289 |
| HLA-DRB1*13:02 | LISNINVIV | 10 | 289 |
| HLA-DRB1*13:02 | HLRPRDLIS | 10 | 290 |
| HLA-DRB1*07:01 | LNLAQSKNF | 21 | 291 |
| HLA-DRB1*07:01 | LAQSKNFHL | 21 | 292 |
| HLA-DRB1*07:01 | LAQSKNFHL | 21 | 292 |
| HLA-DRB1*03:01 | LLLDLQMIL | 22 | 293 |
| HLA-DRB1*03:01 | LLLDLQMIL | 22 | 293 |
| HLA-DRB1*03:01 | QLQLEHLLL | 22 | 294 |
| HLA-DRB1*03:01 | LLLDLQMIL | 23 | 293 |
| HLA-DRB1*03:01 | LLLDLQMIL | 23 | 293 |
| HLA-DRB1*07:01 | LAQSKNFHL | 24 | 292 |
| HLA-DRB1*07:01 | LAQSKNFHL | 25 | 292 |
| HLA-DRB1*13:02 | LISNINVIV | 27 | 289 |
| HLA-DRB1*13:02 | LISNINVIV | 27 | 289 |
| HLA-DRB1*07:01 | RWITFSQSI | 35 | 287 |
| HLA-DRB1*07:01 | FSQSIISTL | 38 | 299 |
| HLA-DRB1*07:01 | LEEVLNLAQ | 51 | 295 |
| HLA-DRB1*07:01 | LNLAQSKNF | 52 | 291 |

The additional "hot spot" introduced by the C125S mutation was removed by instead using a C125A mutation (Table 5). Sequence numbering/ amino acid residue positions are according to SEQ ID NO:1.

**Table 5. MHC Cass II Molecules Binding Prediction of IL-2 Mutant with L36I, C125A and "Non-alpha" Mutations (IL-2V1/L36I/C125A) Using T Cell Epitope Prediction Tools**

| IL-2V1-C125A-L36I | smm_align_core | smm_align_IC₅₀ | SEQ ID NO |
|---|---|---|---|
| MHC Class II Allele | | | |
| HLA-DRB1*13:02 | LISNINVIV | 9 | 289 |
| HLA-DRB1*13:02 | LISNINVIV | 9 | 289 |
| HLA-DRB1*13:02 | LISNINVIV | 10 | 289 |
| HLA-DRB1*13:02 | LISNINVIV | 10 | 289 |
| HLA-DRB1*13:02 | HLRPRDLIS | 10 | 290 |
| HLA-DRB1*07:01 | LNLAQSKNF | 21 | 291 |
| HLA-DRB1*07:01 | LAQSKNFHL | 21 | 292 |
| HLA-DRB1*07:01 | LAQSKNFHL | 21 | 292 |
| HLA-DRB1*03:01 | LLLDLQMIL | 22 | 293 |
| HLA-DRB1*03:01 | LLLDLQMIL | 22 | 293 |
| HLA-DRB1*03:01 | QLQLEHLLL | 22 | 294 |
| HLA-DRB1*03:01 | LLLDLQMIL | 23 | 293 |
| HLA-DRB1*03:01 | LLLDLQMIL | 23 | 293 |
| HLA-DRB1*07:01 | LAQSKNFHL | 24 | 292 |
| HLA-DRB1*07:01 | LAQSKNFHL | 25 | 292 |
| HLA-DRB1*13:02 | LISNINVIV | 27 | 289 |
| HLA-DRB1*13:02 | LISNINVIV | 27 | 289 |
| HLA-DRB1*07:01 | LEEVLNLAQ | 51 | 295 |

It was surprising that IL-2V1/L36I/C125A (SEQ ID NO:184) was shown to have lower immunogenicity risk than the wildtype IL-2. A side-by-side comparison showed that this IL-2 mutant had fewer "hot spots" than wildtype IL-2 (Table 6; the additional "hot spot" binding peptides in the wildtype IL-2 are bolded; SEQ: SEQ ID NO).

**Table 6. Comparison of "Hot Spots" in IL-2 Mutant IL-2V1/L36I/C125A and WT IL-2**

| IL-2V1/L36I/C125A | | | SEQ | WT | | | SEQ |
|---|---|---|---|---|---|---|---|
| allele | smm_align_ core | smm_ align_IC₅₀ | | allele | smm_align _core | smm_ align_IC₅₀ | |
| HLA-DRB1*13:02 | LISNINVIV | 9 | 289 | HLA-DRB1*13:02 | LISNINVI V | 9 | 289 |
| HLA-DRB1*13:02 | LISNINVIV | 9 | 289 | HLA-DRB1*13:02 | LISNINVI V | 9 | 289 |
| HLA-DRB1*13:02 | LISNINVIV | 10 | 289 | HLA-DRB1*13:02 | LISNINVI V | 10 | 289 |
| HLA-DRB1*13:02 | LISNINVIV | 10 | 289 | HLA-DRB1*13:02 | LISNINVI V | 10 | 289 |
| HLA-DRB1*13:02 | HLRPRDLI S | 10 | 290 | HLA-DRB1*13:02 | HLRPRDL IS | 10 | 290 |
| HLA-DRB1*07:01 | LNLAQSK NF | 21 | 291 | HLA-DRB1*07:01 | LNLAQS KNF | 21 | 291 |
| HLA-DRB1*07:01 | LAQSKNF HL | 21 | 292 | HLA-DRB1*07:01 | LAQSKN FHL | 21 | 292 |
| HLA-DRB1*07:01 | LAQSKNF HL | 21 | 292 | HLA-DRB1*07:01 | LAQSKN FHL | 21 | 292 |
| HLA-DRB1*03:01 | LLLDLQMI L | 22 | 293 | HLA-DRB1*03:01 | LLLDLQ MIL | 22 | 293 |
| HLA-DRB1*03:01 | LLLDLQMI L | 22 | 293 | HLA-DRB1*03:01 | LLLDLQ MIL | 22 | 293 |
| HLA-DRB1*03:01 | QLQLEHL LL | 22 | 294 | HLA-DRB1*03:01 | QLQLEH LLL | 22 | 294 |
| HLA-DRB1*03:01 | LLLDLQMI L | 23 | 293 | HLA-DRB1*03:01 | LLLDLQ MIL | 23 | 293 |
| HLA-DRB1*03:01 | LLLDLQMI L | 23 | 293 | HLA-DRB1*03:01 | LLLDLQ MIL | 23 | 293 |
| HLA-DRB1*07:01 | LAQSKNF HL | 24 | 292 | HLA-DRB1*07:01 | LAQSKN FHL | 24 | 292 |
| HLA-DRB1*07:01 | LAQSKNF HL | 25 | 292 | HLA-DRB1*07:01 | LAQSKN FHL | 25 | 292 |
| HLA-DRB1*13:02 | LISNINVIV | 27 | 289 | HLA-DRB1*13:02 | LISNINVI V | 27 | 289 |
| HLA-DRB1*13:02 | LISNINVIV | 27 | 289 | HLA-DRB1*13:02 | LISNINVI V | 27 | 289 |
| HLA-DRB1*07:01 | LEEVLNL AQ | 51 | 295 | HLA-DRB1*07:01 | FCQSIIS TL | 44 | 296 |
| | | | | HLA-DRB1*07:01 | LEEVLNL AQ | 51 | 295 |
| | | | | HLA-DRB1*07:01 | LNLAQS KNF | 52 | 292 |
| | | | | HLA-DRB1*07:01 | WITFCQ SII | 54 | 297 |

### Example 2: Discovery of scFv Molecules That Bind to IL-2 and Inhibit the Interactions Between IL-2 and IL-2Rβγ

### A. Screening scFvs from a Yeast Library

A human scFv yeast display library was screened for IL-2 binders. The IL-2 was labeled by biotin on the avi-tag (Acro Biosystems). After two initial rounds of magnetic-activated cell sorting (MACS) using 200 nM biotinylated IL-2 and streptavidin beads and two rounds of fluorescence-activated cell sorting (FACS) using 200 nM biotinylated IL-2 and APC-streptavidin, the enriched pool was further sorted for scFv binders that blocked the IL-2/IL-2Rβγ interacts. Then single clones were plated into six 96-plates. iQue^{®} screening were further performed using both 100 nM IL-2 and 100 nM biotinylated IL-2Rα for the scFv binders that do not block IL-2R alpha interaction. The second iQue^{®} screening were performed using both 100 nM biotinylated IL-2 and 100 nM Fc-IL-2βγ for the scFv binders that block IL-2Rβγ interaction. Sixty-eight yeast clones that possessed both characters were collected for further analyses. The supernatants of these clones' culture were collected and tested for the binding to IL-2 and blocking IL-2Rβγ functions using Fortebio. Thirty-six scFv candidates were cloned out fused to an Fc fragment and expressed in ExpiCHO cells. Purified scFv-Fc homodimers were further characterized using Fortebio and CTLL2 neutralization assays.

### B. Blocking Assay for Selected IL-2 Binders Screened from Human ScFv Yeast Library

The supernatants of the candidate clones were tested for their ability to block the binding of IL-2 to IL2Rβγ. The blocking was tested using an Octet^{®} Red96e Kinetics binding assay. Biotinylated IL-2Rβγ was loaded onto Streptavidin (SA) sensors at 1µg/ml. First, the binding affinity of IL-2 was confirmed by associating a three-fold serial dilution of Fc-IL-2 (JR8_48.3, comprising SEQ ID NOs:261 and 262, with a starting concentration of 1 µg/ml), onto the SA sensors loaded with IL-2Rβγ (**FIG. 1A**). Fc-IL-2 was added (JR8.48.3, comprising SEQ ID NOs:261 and 262,) at 1 µg/ml, 0.3 µg/ml, and 0.1 µg/ml, onto the SA sensors loaded with IL-2Rβγ.

A one-to-one fitting curve was fitted onto the kinetics results yielding the indicated KD with corresponding binding pattern. 100 µl of control Fc-scFv (JR8.113.3, SEQ ID NO:249) was added to each of the IL-2 dilutions, which resulted in complete blocking of IL-2 binding to IL-2Rβγ (**FIG. 1B**). 100 µl of the yeast culture supernatant of clone K1-69A8 was added to each dilution of Fc-IL-2, which partially blocked the binding of IL-2 to IL-2Rβγ (**FIG. 1C**). In contrast, the supernatants of the clone K3-23A2 did not block the binding of IL-2 to IL-2Rβγ (**FIG 1D**). The results of the blocking assay for the scFv clones from the yeast library are summarized in **Table 7.**

**Table 7. Results from the ForteBio Blocking Assay**

| **scFv-Fc Plasmid Code** | **ScFv Clone Name** | **Assay Results** |
|---|---|---|
| JR8.113.3 | Fc-scFv positive control | blocking |
| IL-2scFv#1 | K1-69A11 | not blocking |
| IL-2scFv#2 | K1-69C7 | NA |
| IL-2scFv#3 | K1-69G3 | blocking |
| IL-2scFv#4 | K1-69H5 | NA |
| IL-2scFv#5 | K3-15F7 | blocking |
| IL-2scFv#6 | K3-30F6 | blocking |
| IL-2scFv#7 | K3-30F7 | not blocking |
| IL-2scFv#8 | K3-30H7 | NA |
| IL-2scFv#9 | K3-30B12 | blocking |
| IL-2scFv#10 | K1-69A8 | blocking |
| IL-2scFv#11 | K1-69B4 | Partial blocking |
| IL-2scFv#12 | K3-23A2 | not blocking |
| IL-2scFv#13 | K3-30G9 | blocking |
| IL-2scFv#14 | K3-30H9 | blocking |
| IL-2scFv#15 | K4-39B3 | NA |
| IL-2scFv#16 | K4-39G4 | not blocking |
| IL-2scFv#17 | L4-39A5 | not blocking |
| IL-2scFv#18 | L4-39B9 | blocking |
| IL-2scFv#19 | L4-39D7 | blocking |

| | | |
|---|---|---|
| *NA: not applicable. | | |

### C. CTLL2 Cell-Based Activity Assays

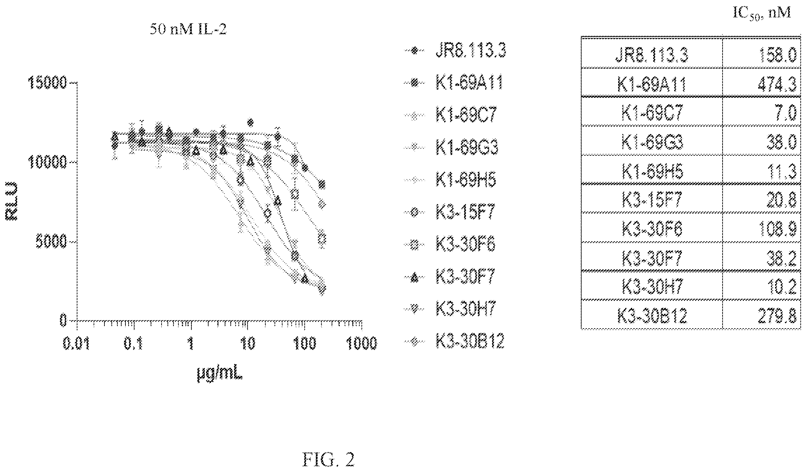

Nine scFvs (#1-#9 of Table 7) were cloned and their genes were fused to a human Fc domain. The fusion proteins were expressed in Expi293 cells as homodimers and purified by ProA chromatography. The purified proteins were tested in a cell-based inhibition assay using CTLL2 cell line (**FIG. 2**).

CTLL2 cells were grown in the RPMI 1640 medium supplemented with L-glutamine, 10% fetal bovine serum, 10% non-essential amino acids, 10% sodium pyruvate, and 55 µM beta-mercaptoethanol. CTLL2 cells were non-adherent and maintained at 5 x 10⁴ -1 x 10⁶ cells/ml in medium with 100 ng/ml of IL-2. Generally, cells were split twice per week. For bioassays, it was best to use cells no less than 48 hours after passage. Samples were diluted at 2x concentration in 50 µl/well in a 96 well plate. The IL-2 standards were titrated from 20 ng/ml (2x concentration) to 3x serial dilutions for 12 wells. Samples were titer tested as appropriate. CTLL2 cells were washed 5 times to remove IL-2, dispensed 5000 cells/well in 50 µl and cultured overnight or at least 18 hours with the samples. Subsequently, 100 µl/well Cell Titer Glo^{®} reagents (Promega) were added and luminescence were measured.

During the CTLL2 assay, 50 nM IL-2 was added to the CTLL2 cells. The purified scFv-Fc fusion proteins together with the positive control (JR8.113.3) were added to the cells. Dose-dependent inhibition of the IL-2 activities by the purified fusion proteins are shown in the Figure. The scFv-Fc homodimers K1-69-C7, K1-69-H5, and K3-30-H7 showed strong inhibitory effect on IL-2 and had an IC₅₀ of 7.0 nM, 11.3 nM, and 10.2 nM, respectively (**FIG. 2**).

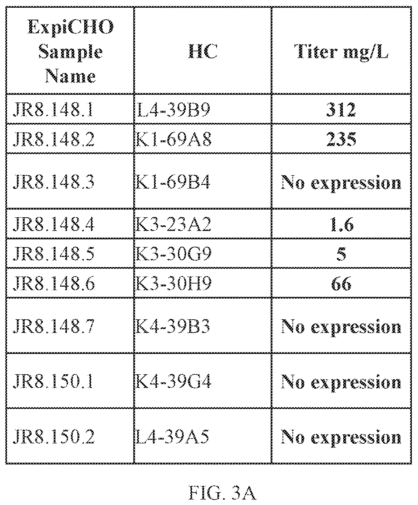

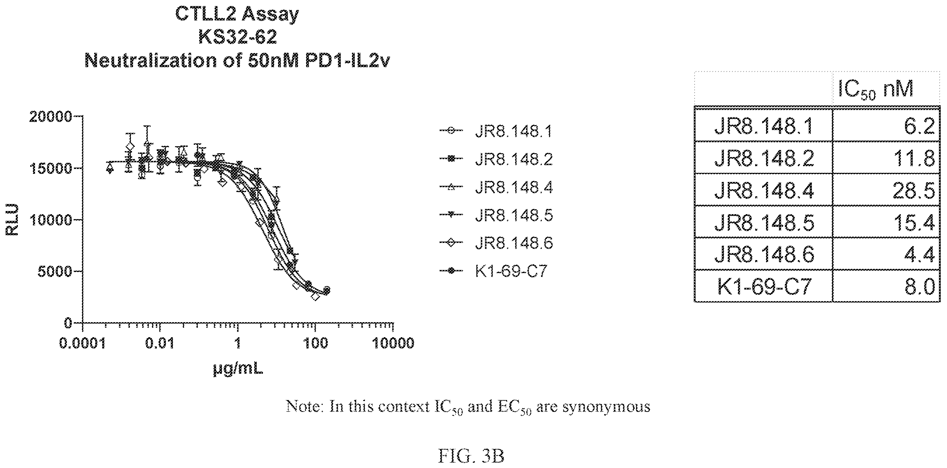

Nine additional scFvs-Fc (#10-#18 of Table 7) plasmids were constructed and expressed in ExpiCHO^{™} cells and purified using ProA chromatography. Four of the scFvs-Fc were not expressed. The purified scFv-Fc's were tested in the CTLL2 assays to neutralize 50 nM PD1-IL-2v (**FIGs. 3A** and **3B**). Their inhibition activities were compared with the clone K1-69-C7. Several additional clones showed strong inhibition of IL-2 activities. L4-39-B9, K1-69-A8, K3-23-A2, K3-30-G9 and K3-30-H9 showed strong neutralization effect and had IC₅₀=6.2 nM,11.8 nM, 28.5 nM, 15.4 nM, and 4.4 nM, respectively.

The sequences of the scFv clones selected from the CTLL2 cell-based activity assays are shown in SEQ ID NOs:34-36 and 250-270.

### Example 3: Binding Affinities of anti-IL-2 ScFv-Fc

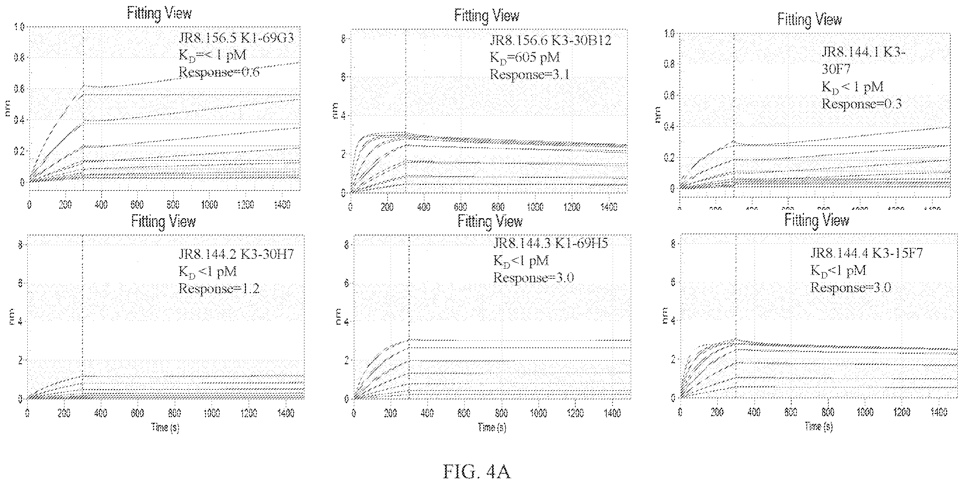

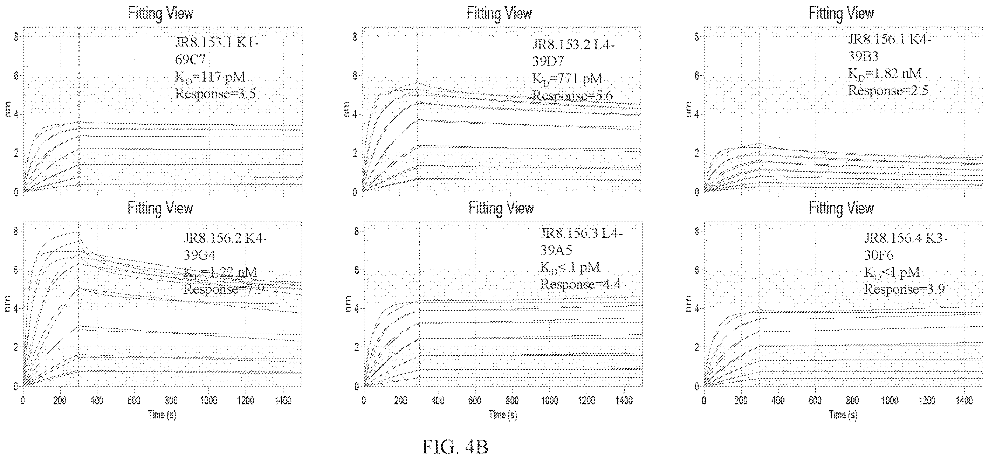

The binding affinities of the selected scFv-Fc's to IL-2 were tested using Fortebio (**FIGs. 4A** and **4B**). The results are summarized in **Table 8.**

**Table 8. Results from the ForteBio Blocking Assay**

| **ScFv Clone** | **Purified Sample** | **K_{D}KD** | **Response** |
|---|---|---|---|
| K1-69G3 | JR8.156.5 K1-69G3 | <1 pM | 0.6 |
| K3-30B 12 | JR8.156.6 K3-30B12 | 605 pM | 3.1 |
| K3-30F7 | JR8.144.1 K3-30F7 | <1 pM | 0.3 |
| K3-30H7 | JR8.144.2 K3-30H7 | <1 pM | 1.2 |
| K1-69H5 | JR8.144.3 K1-69H5 | <1 pM | 3.0 |
| K3-15F7 | JR8.144.4 K3-15F7 | <1 pM | 3.0 |
| K1-69C7 | JR8.153.1 K1-69C7 | 117 pM | 3.5 |
| L4-39D7 | JR8.153.2 L4-39D7 | 771 pM | 5.6 |
| K4-39B3 | JR8.156.1 K4-39B3 | 1.82 nM | 2.5 |
| K4-39G4 | JR8.156.2 K4-39G4 | 1.22 nM | 7.9 |
| L4-39A5 | JR8.156.3 L4-39A5 | <1 pM | 4.4 |
| K3-30F6 | JR8.156.4 K3-30F6 | < 1pM | 3.9 |

### Example 4: Expression and Activity Tests of PD-1 Antibody-IL-2 Prodrug Molecules

**CTLL2 Assay.** The scFvs C7, H5, and B12 were fused to the anti-PD1-antibody HC to mask the mutant human IL-2 (IL-2v) as shown in the drawings in **FIG. 5C****.** The molecules were transiently expressed in ExpiCHO^{™} system as shown in **FIG. 5A** and purified by ProA Affinity Chromatography. The purified proteins were activated by hMMP2 digestion, and their IL-2 activities were tested in the CTLL2 assays (**FIG. 5B**). C7, H5 and B12 scFvs were all able to mask the IL-2v activity as the β-ECD did, and IL-2v activity for each prodrug was restored after activation by hMMP2 (**FIG. 5C**). Specifically, the prodrug molecules JR11.20.3, JR11.20.6, and JR11.20.7 with masks C7 (or K1-69C7), H5, and β-ECD showed little activity prior to activation and significantly improved activities after protease-based activation (**FIG. 5C**).

**NK92 Assay.** In another experiment, a total of eight prodrug molecules (**FIG. 6A**) were expressed and purified by Protein A Affinity Chromatography. The prodrugs prior to and after the protease digestion were tested by the NK92 cell-based activity assay. Briefly, NK92 cells were grown in the RPMI 1640 medium supplemented with L-glutamine, 10% fetal bovine serum, 10% non-essential amino acids, 10% sodium pyruvate, and 55 pM β-mercaptoethanol. NK92 cells were non-adherent and maintained at 1 x 10⁵ - 1 x 10⁶ cells/ml in medium with 100 ng/ml of IL-2. Generally, cells were split twice per week. For bioassays, it was best to use cells no less than 48 hours after passage. The purified proteins were activated by digesting with hMMP2 and analyzed by SDS-PAGE (**FIG. 6B**), and the IL-2 activities were tested in the NK92 assays (**FIG. 6C**). K1-69C7, K3-30F7, K1-69A8, K3-30H9 and L4-39B9 masked IL-2v strongly. K3-30G9 and K3-30-B12 were able to mask IL-2 activity partially. K3-30F6 did not mask well.

### Example 5: Linker Optimization

In Example 4, the linkers between the anti-PD1 antibody HC and the scFv moiety of the prodrug with an anti-PD-1 antibody as the carrier were tested. Both linkers GGGGSGGGGSGPLGVRGGGGSGGGGS (SEQ ID NO:268) (JR11.20.3) and GGGGSGGGGSGPLGVRGGGGS (SEQ ID NO:269) (JR11.20.5) worked well for the molecule assembly and function.

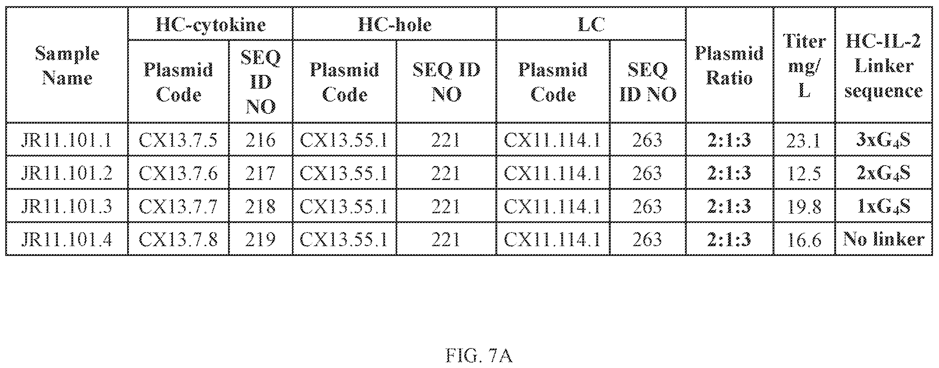

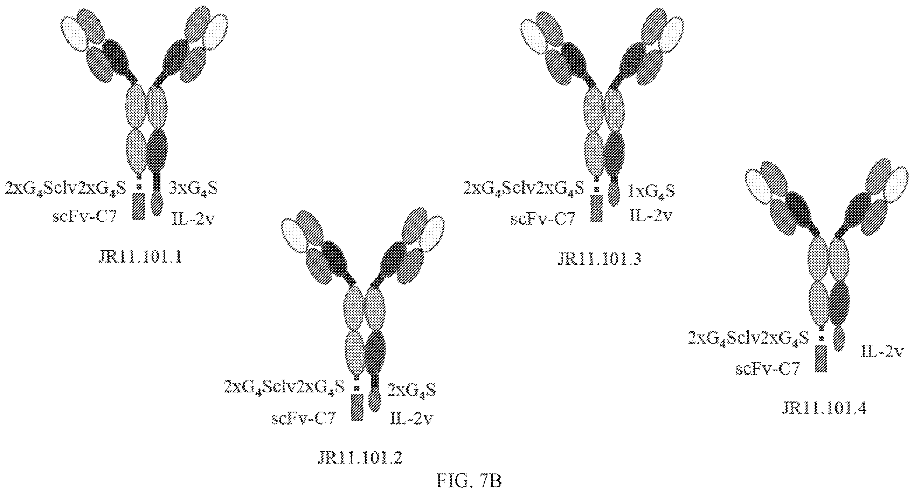

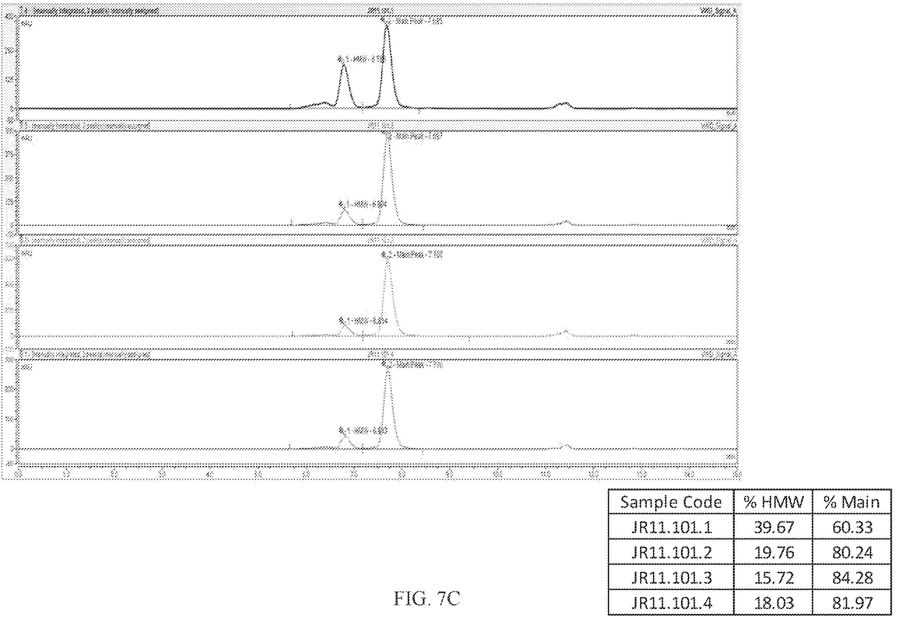

In order to optimize the production and purities of the prodrug molecules, prodrug molecules with linkers of various lengths between the anti-PD1 antibody HC and the IL-2v moiety (**FIG. 7A**) were tested. The linker between the heavy chain (HC) and the cytokine moiety was a GGGGSGGGGSGGGGS (SEQ ID NO:181) or 3x G₄S linker, a GGGGSGGGGS (SEQ ID NO:180) or 2x G₄S linker, or a GGGGS (SEQ ID NO:178) or 1x G₄S linker; or there was no peptide linker. The molecules were transiently expressed in ExpiCHO^{™} systems and purified by ProA affinity chromatography. The purified proteins were analyzed by SDS-PAGE gel and SEC-HPLC (**FIG. 7B**). The data showed that the molecules with shorter (2xG₄S or 1xG₄S) or no peptide linker had higher main peak purity (improved purity) and reduced aggregation. The 2xG₄S linker was selected for subsequent prodrug designs.

### Example 6: Ex vivo Assays with Human PBMC

Human PBMCs were first cultured in 6-well plates with anti-CD3 antibody (1 µg/ml) (pre-coated) and anti-CD28 antibody (1 µg/ml) for 72 hrs to induce the expression of PD-1 on the T cells. The PBMCs were collected, washed, and resuspended in RPMI1640 complete medium resting on ice for 4 hrs. The rested cells were split into two parts. The PBMCs used in the experiments/curves 1a-6a were further pretreated with 40 ng/ml of an PD-1 antibody, which led to the internalization of the PD-1 expressed on the T cells. One part of the cells was incubated for 45 minutes on ice with an anti-PD1 antibody (56 µg/ml) to mask PD-1 expression. After incubation with the anti-PD1 antibody, the cells were then washed to remove free anti-PD1 antibodies. The activated PBMCs with or without anti-PD1 antibody pretreatment were incubated with serially diluted test articles in 96-well plates for 15 minutes in a 37°C incubator. The cells were then immediately fixed with BD Cytofix^{™} fixation buffer and permeabilized with BD Phosflow^{™} Perm Buffer IV (0.5x). The cells were then incubated with a PE-conjugated anti-p-Stat5 antibody.

IL-2 induces STATS Phosphorylation (pSTAT5) in immune cells. The phosphorylation of Stat5 in CD4+ T cells, CD8+ T cells, and CD3-CD56+ NK cells were measured by flow cytometry. Data were graphed with GraphPad Prism 9 software. The PBMC were then treated with anti-CD3 and anti-CD8 antibodies to induce the expression of PD-1 on the T cells. The results are shown in **FIG. 8B** for the CD4+ T cells, **FIG. 8C** for the CD8+ T cells, and **FIG. 8D** for the NK cells. PD-1 antibody-IL-2 prodrug masked by C7 (ASKG812K-C7) showed very low activity with cells with or without prior treatment with the PD-1 antibody. However, PD-1 antibody-IL-2 prodrug masked by C7 showed strong activities post activation (ASKG812K-C7 Act; Tests 2 and 2a in **FIGs. 8B****,** **8C****,** and **8D**) for all the cell types, though the activities were significantly higher with the T cells than the NK cells. In addition, T cells without prior treatment with the PD-1 antibody showed significantly higher activities than the cells with the prior treatment with the PD-1 antibody. The treatment with anti-CD3 and anti-CD8 induced the expression of PD-1 on the T cells. The pretreatment with PD-1 antibody led to the internalization of the PD-1 expressed on the T cells. The results showed that the PD-1 antibody-IL-2 prodrug post activation selectively activated the T cells expressing PD-1. This targeted stimulation of the T cells was achieved presumably through the mechanism of "cis-activation", i.e. the activated prodrug bound to the T cells through both the PD-1-binding domains as well as the cytokine domain. Similar observations were made with the reference PD-1 antibody-IL-2 fusion reference molecule (Tests 3 and 3a in **FIGs. 8B** and **8C**). This was not observed with the NK cells as they typically do not express high levels of PD-1 (Tests 3 and 5a in **FIGs. 8B** and **8C**). "Cis-activation" was not observed with the PD-1 antibody (Tests 5 and 5a in **FIGs. 8B** and **8C**) or the Fc-IL-2v molecule (Tests 4 and 4a in **FIGs. 8B** and **8C**). It was observed that the prodrug masked by scFv G3 (Tests 6 and 6a in **FIGs. 8B** and **8C**) showed "cis-activation" for both CD4+ and CD8+ T cells even though this prodrug comprises no cleavable linker.

### Example 7: Enhanced Thermostability of the Prodrug Molecules

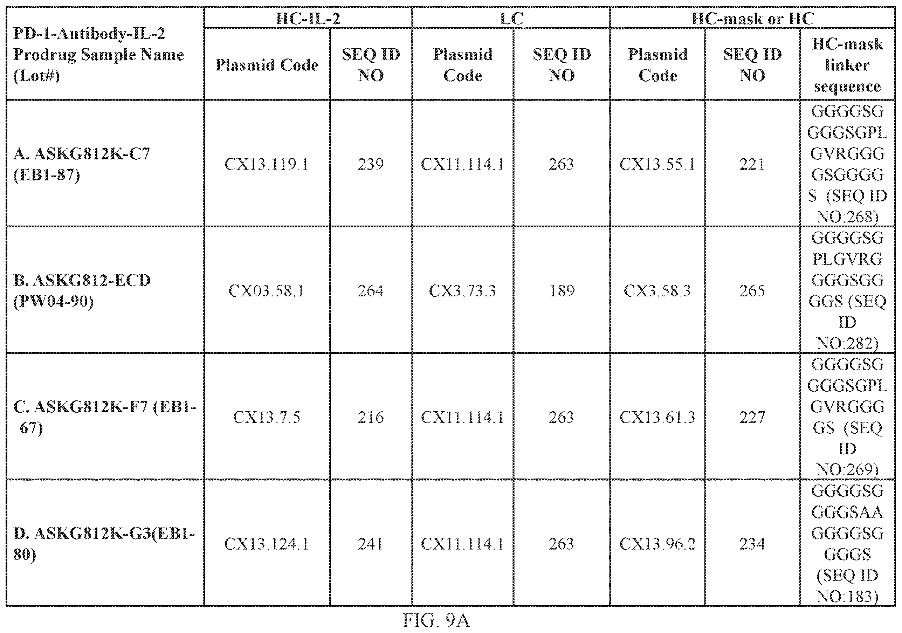

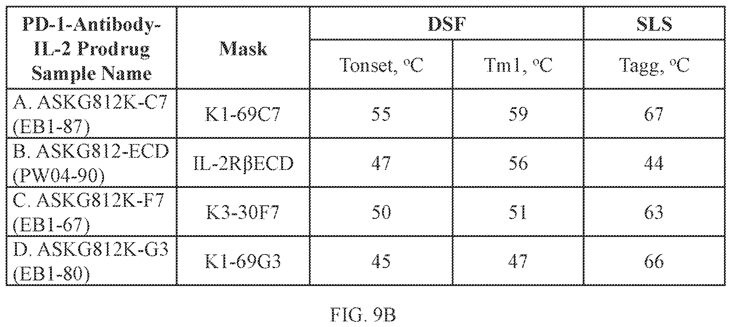

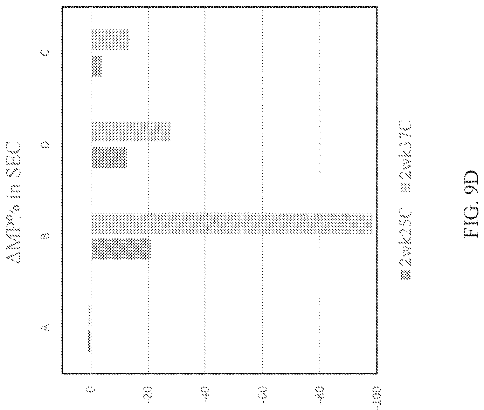

Thermal stability studies were conducted for the prodrug molecules on an Unchained Labs UNcle instrument. Briefly, 8.8 µL of protein samples were loaded into microcuvette (the "Uni") in triplicates. A temperature scan from 25°C to 95°C at a rate of 1°C/min was conducted, and intrinsic fluorescence as well as static light scattering (SLS) was measured. Tm (melting point), Tonset (onset of unfolding), Tagg-266 nm (aggregation onset detected at 266 nm), and Tagg-473nm (aggregation onset detected at 473 nm) results of the triplicate runs (analyzed by UNcle Analysis 4.01) were manually inspected and averaged after outliers were removed. Surprisingly, the results showed that the prodrug masked with the mask C7 (ASKG812K-C7) showed significantly higher thermostability than the prodrugs masked with other masks (**FIG. 9B**). The Tonset and Tm1 were 10°C and 12°C higher than the one masked with IL-2Rβ-ECD, while Tagg was 22°C higher than the one masked with IL-2Rβ-ECD. Both prodrugs masked with F7 and G3 also showed higher Tagg's than the one masked with IL-2Rβ-ECD.

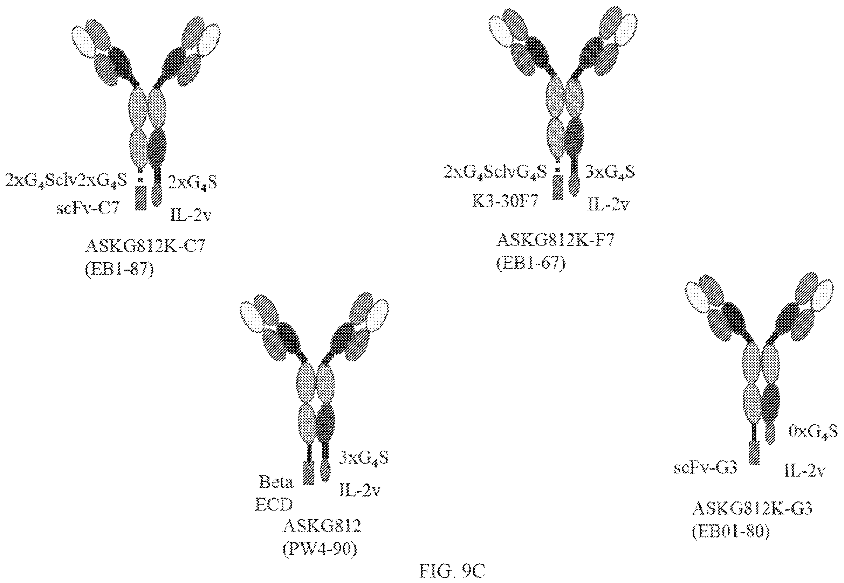

Accelerated stability study was also carried out for the prodrugs. The purities of the samples stored at 25°C and 40°C were tested by a SEC-HPLC method. Briefly, 3 µg of sample was injected onto a size-exclusion chromatography system comprised of a PhenomenexBioZen^{®} dSEC-2 column (3 µm particles, 4.6x300 mm, part number 00H-4788-E0) installed on an Agilent 1260 UHPLC system controlled by Chromeleon 7.2 software. Column temperature was set at 30°C. Mobile phase was 200 mM potassium phosphate, pH 6.2, with 250 mM potassium chloride, flowing at 0.35 mL/min. Protein signals were detected at UV 220 nm. It was surprising to observe that the prodrug masked with C7 showed no decrease in its main peak%, while all the other prodrugs showed significant decreases of their main peak% (**FIG. 9C**). Both prodrugs masked with F7 and G3 also led to higher stability than the one masked with IL-2Rβ-ECD. IL-2 showed poor thermostability and was prone to aggregation during storage at accelerated condition. Mask C7 may have potentially enhanced Tm of IL-2 by over 10°C, and significantly enhanced IL-2 stability.

### Example 8: Mask F7 Is Able to Mask IL-2v with L36I Mutation

The PD-1 antibody-IL-2v reference molecule (EB01-08), a PD-1 antibody-IL-2v (LL24-68), a PD-1 antibody-IL-2v/L36I (JR11.145.2), and a prodrug which comprises PD-1 antibody-IL-2v/L36I masked with F7 (JR11.145.4 masked) were expressed and purified. The samples were tested using the CTLL2 assay. The sample information is shown in **FIG. 10A****.** The results are shown in **FIG. 10B****.** The data showed that PD-1 antibody-IL-2v/L36I had similar activity as that of the reference molecule. The prodrug with IL-2v/L36I and mask F7 showed little activity in the CTLL2 assay, indicating that mask F7 was able to mask IL-2v/L36I efficiently. While introduction of the mutation of L36I reduces the potential risk of immunogenicity, the mutation does not interfere with the masking by F7.

### Example 9: Construction, Expression, and Ex Vivo Activity Analysis of Antibody-Cytokine Fusion Molecules

A number of antibody-IL-2 cytokine fusion molecules (ASKG222) are designed with structures as illustrated in **FIGs. 11A-11F****.** The IL-2 of the fusion molecules are designed to have no or little activity in binding to IL-2Rβ, while for the most part retaining its affinity to IL-2Rα and IL-2Rγ. This class of molecules are designed to be able to selectively stimulate Tregs over Teff and NK cells.

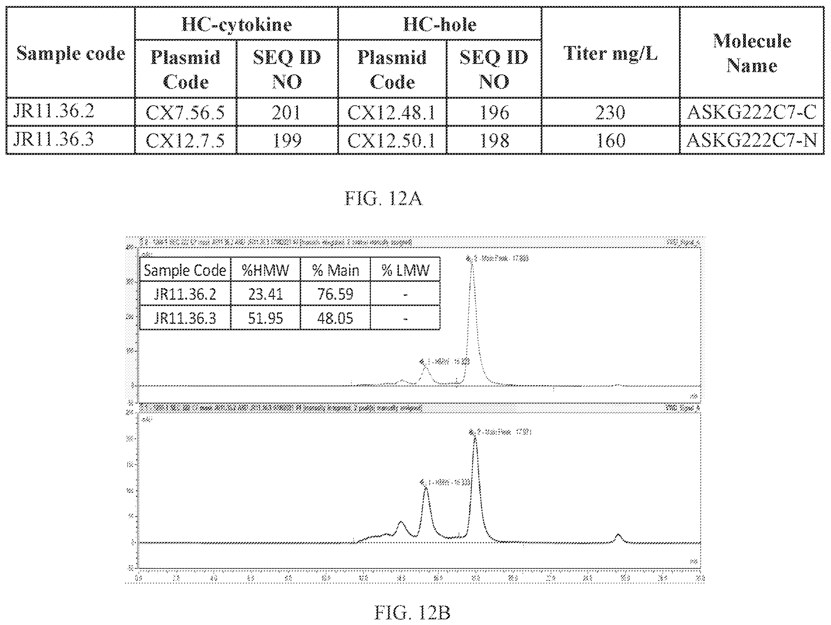

A comparison of C-terminus and N-terminus of Fc fragment fusion of K1-69C7 is shown in **FIGs. 12A-12B****.** Fc fusion molecules (as shown in **FIGs. 10A** and **10B****,** and **FIG. 12A**) were expressed in ExpiCHO cells and purified by Protein A affinity column. The SEC-HPLC purities of the two molecules **in** **FIG. 12A** are shown in **FIG. 12B****.**

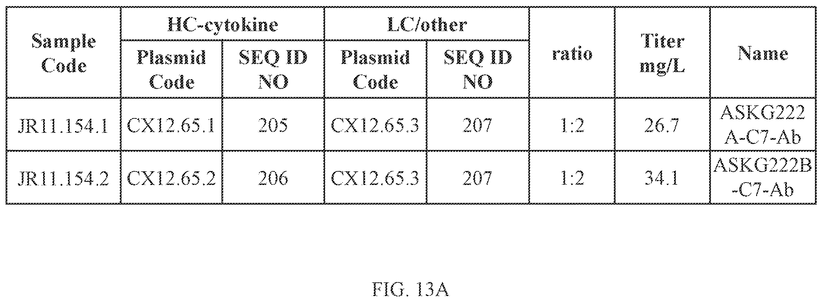

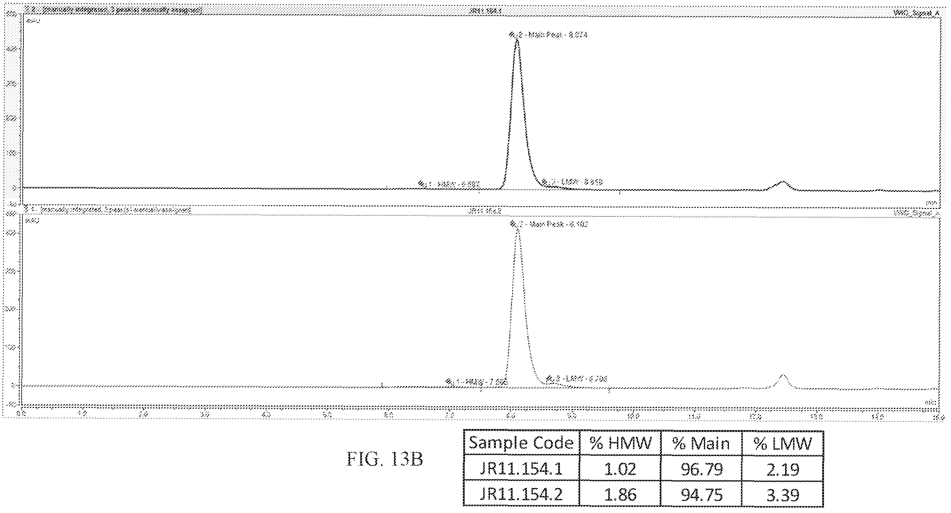

An antibody comprising the same VH and VL domains as that of scFv K1-69C7 was constructed, and was fused with an IL-2 polypeptide (**FIGs. 13A**). The antibody-cytokine fusion molecules were expressed in ExpiCHO cells and purified by a Protein A affinity column. The SEC-HPLC purities of the two molecules in **FIG. 13A** are shown in **FIG. 13B****.**

Three samples, ASKG222C7-C, ASKG222C7-N, and ASKG222A-C7-ab were further purified to high purity and tested in an *ex vivo* assay. Reference samples (R1, an analog of AMG592, and R2, an analog of PT101), wildtype IL-2, and a negative control (human IgG) were also included. The *ex vivo* assay with human PBMC was carried out to examine the abilities of the antibody-cytokine fusion molecules to selectively stimulate the expansion of Treg cells. The induction of Ki67 was used as the readout for this activity assay.

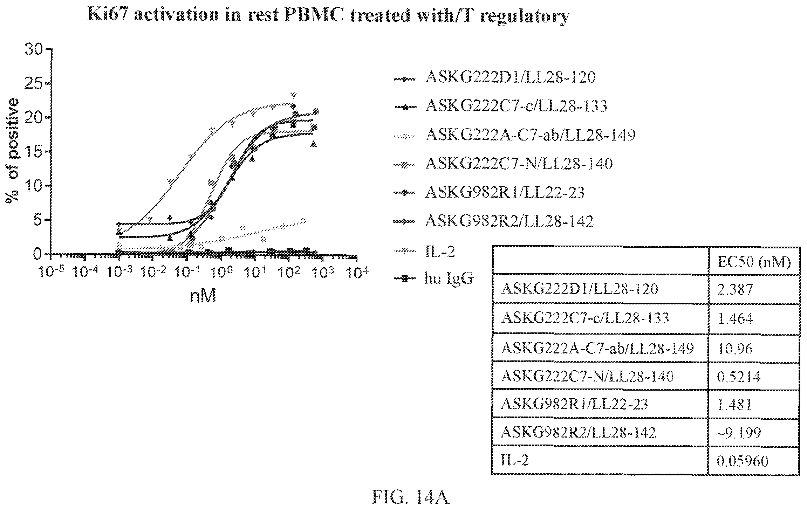

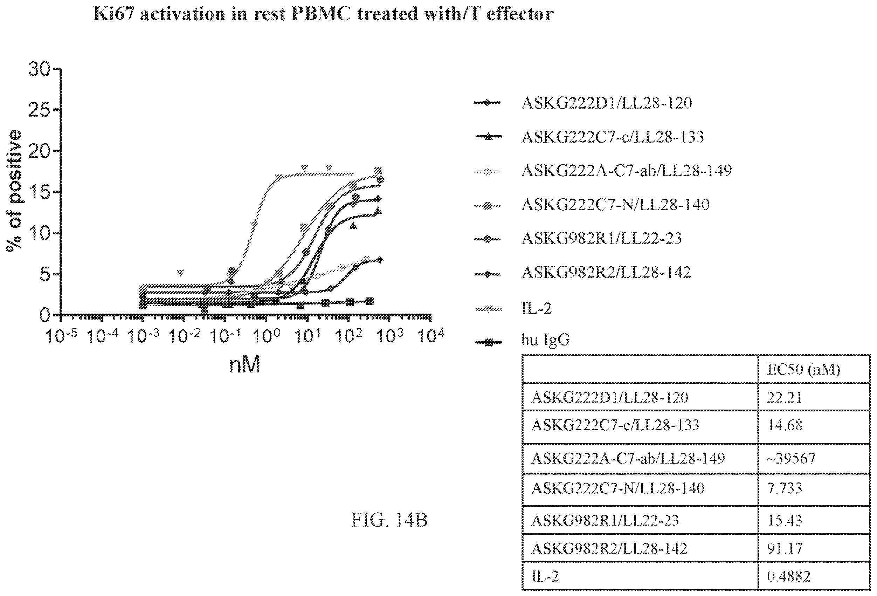

Briefly, an ASKG222 molecule assay dilution was prepared and 100 µl of 2x of sample was added to the wells of a plate. 100 µl of PBMCs (400-500k cells/well) were added into the wells with the diluted test articles and were cultured for 3 days. Cells were stained with different fluorochrome-labelled anti-CD3, anti-CD4, anti-CD8, anti-CD25, and anti-CD161a antibodies for cell surface staining. CD3-CD161a+ cells were defined as NK cells. Cells were further treated with Thermo Fisher Invitrogen Foxp3 fixation/permeabilization buffer with anti-Foxp3 and anti-Ki67 staining. Samples were analyzed by flow cytometry and data were graphed with GraphPad Prism 9 software. IL-2 was added as the positive control and human antibody (hIgG) was added as the negative control. Two reference molecules, R1 (an analog of AMG592 of Amgen) and R2 (an analog of PT101 of Padion Therapeutics) were also included. The results are shown in **FIG. 14A** (Treg cells) and **FIG. 14B** (Teff cells). The results showed that ASKG222C7-C and ASKG222C7-N had better selectivity for stimulating Treg cells over Teff cells than that of R1 while ASKG222A-C7-ab had better selectivity for Treg cells than R2. Both R1 and R2 are currently in clinical trials for autoimmune indications.

### Example 10: Rat PK/PD

3 male Sprague-Dawley rats 53-58 days old with jugular vein cannula (Envigo) were injected with purified ASKG222A-C7-ab (Lot# LL28-149) at 2 mg/Kg I.V. Blood was sampled at T₀ (pre-dose), 1, 2, 4, 6, 10, 24, 48, 72, 96, 120 and 144 hours after dosing for PK determination. Flow cytometric PD analysis was performed on T0, 72- and 120-hour samples, and plasma cytokine levels were determined on T₀, 24, 48, 72, 96, 120- and 144-hour samples.

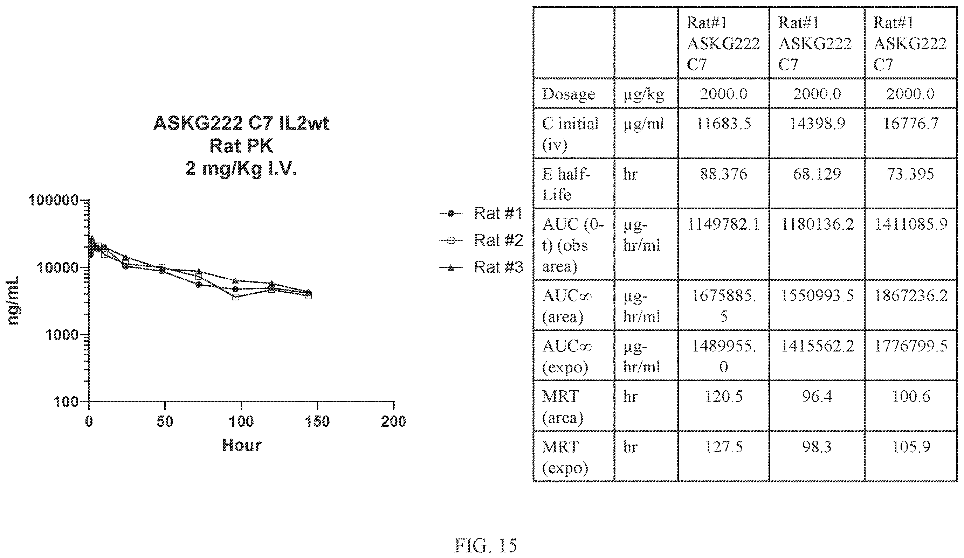

Test article concentrations in the plasma were determined by ELISA. ELISA plates were coated with 100 µL/well F(ab')₂ goat anti-human IgG Fcγ (Jackson Immuno-Research, #109-006-170) at 2 µg/mL in PBS overnight at 4°C for test article capture. Plates were blocked with 100 µL PBS/10% goat serum for 1 hour and washed with D.I. water. Plasma samples were serially diluted from 1:10 3x 8 wells, and the test article standard was diluted from 2 µg/mL 3 x 12 wells in 100 µL/well PBS/10% goat serum. Plates were incubated for 1 hour and washed with D.I. water. For test article detection 100 µL/well anti-IL2 biotin (eBioscience, #13-7028-85) was added at 0.5 µg/mL in PBS/10% goat serum, incubated for 1 hour and washed with D.I. water. Streptavidin-HRP (Jackson Immuno-Research) 1:1000, 100 µL/well was added in PBS/10% goat serum and incubated for 1 hour. After washing with D.I. water TMB substrate (Thermo Scientific) was added at 100 µL/well. Color development was stopped with 100 µL. H₂SO₄, and OD₄₅₀ was measured with a microplate spectrophotometer (Molecular Devices). **FIG. 15** shows the PK results of ASKG222A-C7-ab. Three rats were dosed once at 2 mg/kg, IV. The drug concentrations in serum samples is shown at various time points and were measured by ELISA. The data showed that the molecule had a half-life of approximately 68 hours to 88 hours, which is typical for an antibody.

### Example 11: In Vivo Efficacy Study with Colon 26 Syngeneic Tumor Model

An *in vivo* efficacy study was carried out with the Colon 26 syngeneic tumor model. Briefly, female 7-8 week old Balb/C mice were implanted with 5 x 10⁵ Colon 26 tumor cells delivered subcutaneously over the abdomen. When mean tumor volume reached approximately 82 mm³, mice were randomized into treatment groups such that each group had approximately the same mean tumor volume. Mice were treated with test article or vehicle by intraperitoneal injection in 100 µL of PBS given 3 days apart for a total of two treatments. Tumor volume was determined by measuring the longest (a) and shortest (b) diameter and calculating the volume using the formula = ab²π/6.

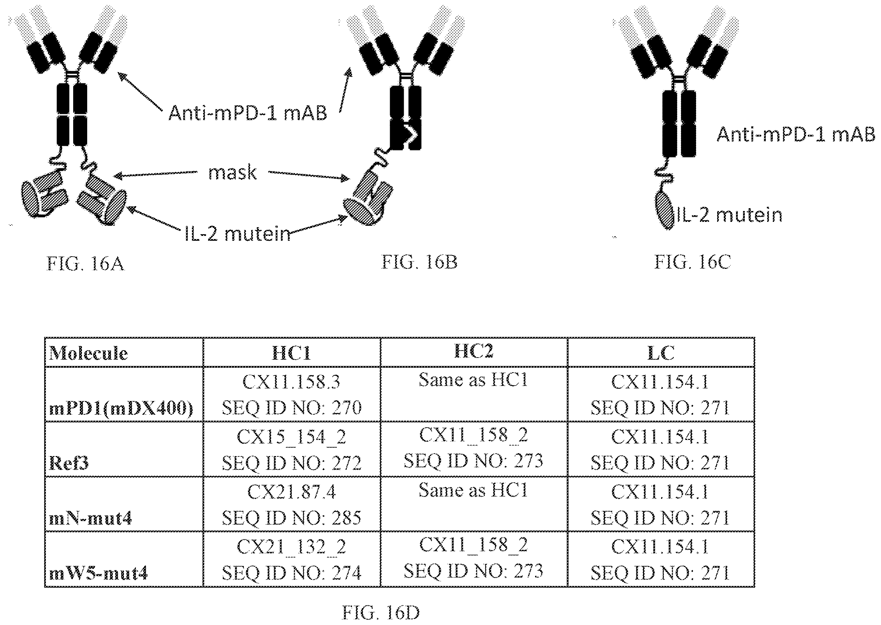

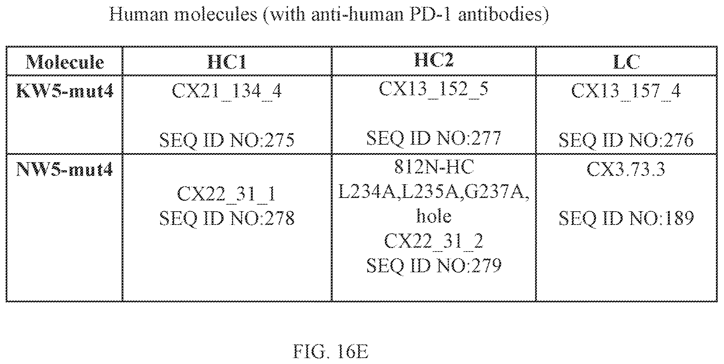

Fusion molecule 812mN-mut4 comprises two identical light chains of an anti-mouse PD-1 antibody, and two identical heavy chain polypeptide chains; wherein the heavy chain polypeptide chain comprises, from N-terminus to C-terminus, the heavy chain of the anti-mouse PD-1 antibody, a mask which binds to IL-2, and an IL-2 mutein. The structure of this molecule is illustrated in **FIG. 16A****,** and its sequence information is shown in **FIG. 16D****.**

Fusion molecule 812mW5-mut4 has the structure illustrated in **FIG. 16B****.** It is similar to 812mN-mut4 except that it comprises only one mask and one IL-2 mutein at the C-terminus of the heavy chain of the anti-mouse PD-1 antibody.

Ref3 is an anti-PD-1 antibody-IL-2 mutein fusion molecule, which has a structure as illustrated in **FIG. 16C****.** It is a mouse analog of eciskafusp alfa which is currently in clinical development.

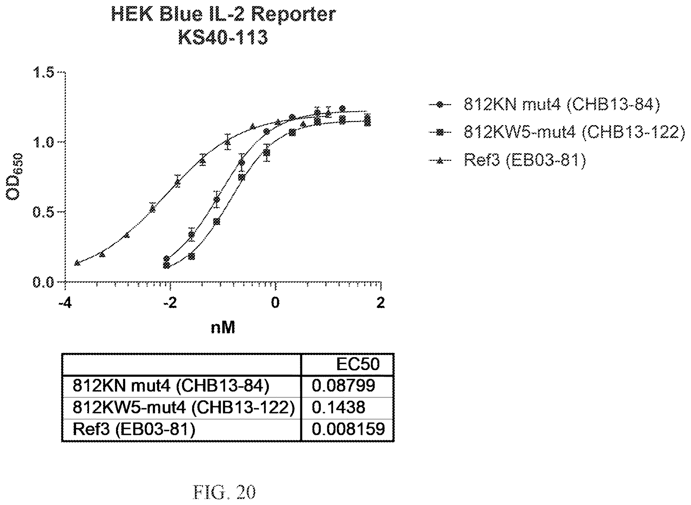

All three molecules have the same anti-PD-1 antibody as that of mPD-1 molecule. 812mN-mut4 comprises two copies of the masked IL-2 mutein. The human version (i.e. with anti-human PD-1 antibody, 812KN-mut4) of the fusion molecule 812mN-mut4 showed stronger *in vitro* cell-based biological activity than that of the human version (812KW5-mut4) of 812mW5-mut4, which comprises only one copy of the same masked IL-2 mutein **(****FIG. 20****).** The IL-2 mutein in Ref3 is not masked. It has the strongest *in vitro* cell-based IL-2 activity among the three molecules **(****FIG. 20****).** Hence, 812KW5-mut4 has weaker activity than 812KN-mut4 and the reference molecule Ref3. The IL-2 moieties are the same between the human versions and the mouse versions.

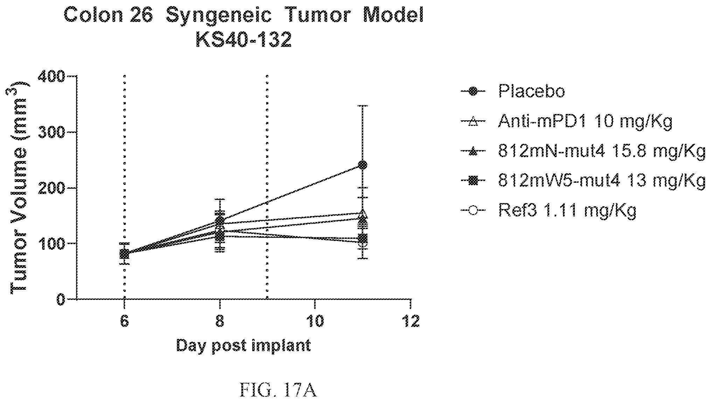

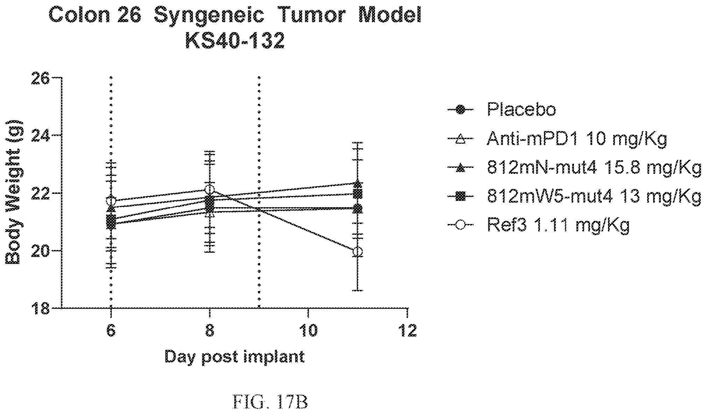

The *in vivo* efficacy and safety results of the two chimeric molecules, Ref3, and the anti-mouse PD-1 antibody are shown in **FIG. 17A** and **FIG. 17B****.** Surprisingly, 812mW5-mut4 has similar anti-tumor efficacy as that of Ref3 **(****FIG. 17A****),** yet 812mW5-mut4 is safer than Ref3 as there was no body weight drop for 812mW5-mut4, but significant body weight loss was observed with Ref3 **(****FIG. 17B****).** It was also surprising that 812mW5-mut4 was observed to have stronger *in vivo* efficacy than that of 812mN-mut4, as the latter was expected to have stronger *in vitro* cell-based activity.

### Example 12: In Vivo Efficacy Study with CT26 Syngeneic Tumor Model

An *in vivo* efficacy study was carried out with the CT26 syngeneic tumor model. Briefly, female, 7-8 week old Balb/C mice were implanted with 1 x 10⁶ CT26 tumor cells delivered subcutaneously over the abdomen. When mean tumor volume reached approximately 94 mm³, mice were randomized into treatment groups such that each group had approximately the same mean tumor volume. Mice were treated with test article or vehicle by intraperitoneal injection in 100 mL PBS given 3 days apart for 2 total treatments. Tumor volume was determined by measuring the longest (a) and shortest (b) diameter and calculated using the formula volume = ab²π/6.

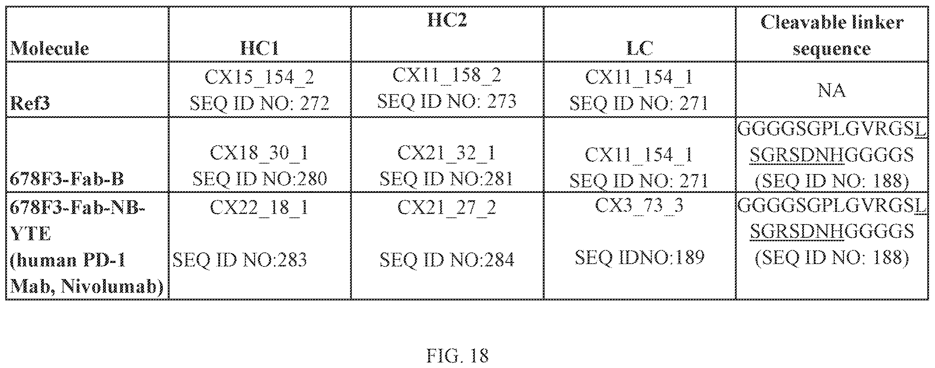

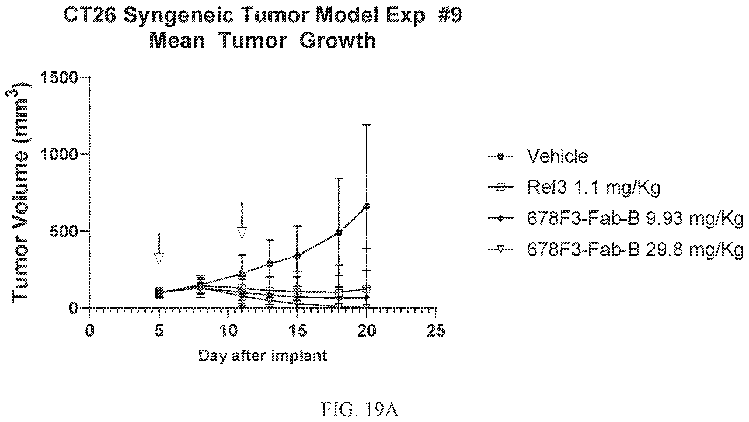

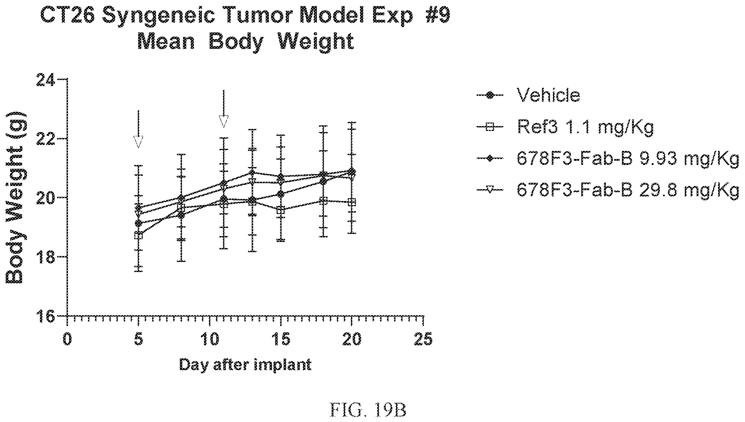

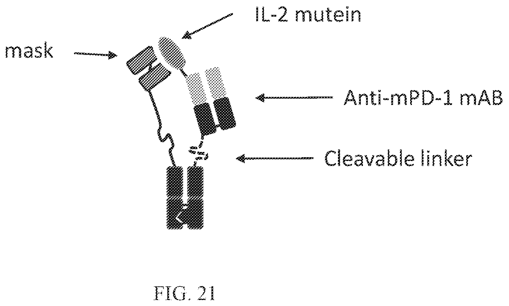

678F3-Fab-B and Ref3 were tested in the CT26 model. 678F3-Fab-B at dosages of 9.93 mg/kg and 29.8 mg/kg showed better efficacy and safety than Ref3 (**FIGs. 19A** and **19B****).** The sequence information of 678F3-Fab-B and Ref3 are shown in **FIG. 18****.** An illustration of the structure of 678F3-Fab-B is shown in **FIG. 21****.**

### SEQUENCES

In the sequences below, boxed residues indicate mutations. Underlines in cleavable linkers indicate protease substrate sequences. Underlined and bolded sequences refer to CDRs.
**SEQ ID NO:1** - human IL-2
**SEQ ID NO:2 -** IL-2 agonist polypeptide 
APXaa3SSSTKKT QLQLEHLLL Xaa20 LQMILNGINN YKNPKLTXaa38ML TXaa42KFXaa45MPKKA
TELKHLQCLE EXaa62LKPLEXaa68VL NLXaa73QSKNFHL RPRDLISXaa88IXaa90 VIVLELKGSE
TTFMCEYADE TATIVEFLNR WITFXaa125Xaa126SIIS TLT
 wherein Xₐₐ₃ is N or A; wherein Xₐₐ₁₂₅ is C or S; wherein Xₐₐ₂₀ is selected from D, H, K, L, M, N, Q, R, S, V, and Y; wherein Xₐₐ₃₈ is selected from A, K and S; wherein Xₐₐ₄₂ is selected from A, G, I, S, T, Q, E, N, D, R, and K; wherein Xₐₐ₄₅ is selected from A, G, S, T, Q, E, N, D, R, and K; wherein Xₐₐ₆₂ is selected from E, L, A and I; wherein Xₐₐ₆₈ is E or V; wherein Xₐₐ₇₃ is A or T; wherein Xₐₐ₈₈ is selected from N, A, E, F, H, K, L, M, S, T, V, W, and Y; wherein Xₐₐ₉₀ is N or T, and wherein Xₐₐ₁₂₆ is selected from A, D, F, G, H, I, K, L, P, S, T, W and Y; and wherein at least one of Xaa20, Xₐₐ₈₈, Xₐₐ₉₀, and Xₐₐ₁₂₆ is mutated.
**SEQ ID NO:3** - IL-2 agonist polypeptide 
APXaa3SSSTKKT QLQLEHLLLD LQMILNGINN YKNPXaa35ITRMLTXaa42KFXaa45MPKKA
TELKHLQCLE EELKPLEEVL NXaa72Xaa73QSKNFHL RPRDLISNIN VIVLELKGSE
TTFMCEYADE TATIVEFLNR WITFXaa125 Xaa126SIIS TLT
 wherein Xaa3 is N or A; wherein Xaa125 is C or A; wherein Xaa35 is selected from K and N; wherein Xaa42 is selected from A, G, S, T, Q, E, N, D, R, and K; wherein Xaa45 is selected from A, G, S, T, Q, E, N, D, R, and K; wherein Xaa72 is selected from A, G, S, T, Q, E, N, D, R, and K; wherein Xaa73 is selected from A and T; and wherein Xₐₐ₁₂₆ is selected from A, D, F, G, H, I, K, L, P, S, T, W and Y.
**SEQ ID NO:4** - IL-2 agonist polypeptide with L72G 
APASSSTKKT QLQLEHLLLD LQMILNGINN YKNPKITRML TAKFAMPKKA TELKHLQCLE
EELKPLEEVL NGAQSKNFHL RPRDLISNIN VIVLELKGSE TTFMCEYADE TATIVEFLNR
WITFSQAIIS TLT
**SEQ ID NO:5** - IL-2 agonist polypeptide mutein with T3A/C125A-L36I/R38S/F42A/Y45A/E62A 
APASSSTKKT QLQLEHLLLD LQMILNGINN YKNPKITSML TAKFAMPKKA TELKHLQCLE
EALKPLEEVL NLAQSKNFHL RPRDLISNIN VIVLELKGSE TTFMCEYADE TATIVEFLNR
WITFAQSIIS TLT
**SEQ ID NO:6** - IL-2 agonist polypeptide mutein with C125A- L36I R38S/F42A/Y45A/E62A 
APTSSSTKKT QLQLEHLLLD LQMILNGINN YKNPKITSML TAKFAMPKKA TELKHLQCLE
EALKPLEEVL NLAQSKNFHL RPRDLISNIN VIVLELKGSE TTFMCEYADE TATIVEFLNR
WITFAQSIIS TLT
**SEQ ID NO:7** - IL-2 agonist polypeptide mutein with T3A/C125A- L36I R38S/F42A/Y45A/E62L 
APASSSTKKT QLQLEHLLLD LQMILNGINN YKNPKITSML TAKFAMPKKA TELKHLQCLE
ELLKPLEEVL NLAQSKNFHL RPRDLISNIN VIVLELKGSE TTFMCEYADE TATIVEFLNR
WITFAQSIIS TLT
**SEQ ID NO:8 -** IL-2 agonist polypeptide mutein with T3A/C125A- L36I R38S/F42A/Y45A/E62L/ E68V 
APASSSTKKT QLQLEHLLLD LQMILNGINN YKNPKLTSMI TAKFAMPKKA TELKHLQCLE
ELLKPLEVVL NLAQSKNFHL RPRDLISNIN VIVLELKGSE TTFMCEYADE TATIVEFLNR
WITFAQSIIS TLT
**SEQ ID NO:9** - IL-2 agonist polypeptide mutein with T3A/C125A- L36I R38S/F42I/Y45A/E62A 
APASSSTKKT QLQLEHLLLD LQMILNGINN YKNPKLTSMI TIKFAMPKKA TELKHLQCLE
EALKPLEEVL NLAQSKNFHL RPRDLISNIN VIVLELKGSE TTFMCEYADE TATIVEFLNR
WITFAQSIIS TLT
**SEQ ID NO:10 -** IL-2 agonist polypeptide mutein with T3A/C125A- L36I R38S/F42K/Y45A/E62A 
APASSSTKKT QLQLEHLLLD LQMILNGINN YKNPKITSML TKKFAMPKKA TELKHLQCLE
EALKPLEEVL NLAQSKNFHL RPRDLISNIN VIVLELKGSE TTFMCEYADE TATIVEFLNR
WITFAQSIIS TLT
**SEQ ID NO:11 -** IL-2 agonist polypeptide mutein with T3A/C125A- L36I R38S/F42K/Y45N/E62A 
APASSSTKKT QLQLEHLLLD LQMILNGINN YKNPKITSML TAKFNMPKKA TELKHLQCLE
EALKPLEEVL NLAQSKNFHL RPRDLISNIN VIVLELKGSE TTFMCEYADE TATIVEFLNR
WITFAQSIIS TLT
**SEQ ID NO:12** - IL-2 agonist polypeptide mutein with T3A/C125A- L36I R38S/F42A/Y45R/E62A 
APASSSTKKT QLQLEHLLLD LQMILNGINN YKNPKITSML TAKFRMPKKA TELKHLQCLE
EALKPLEEVL NLAQSKNFHL RPRDLISNIN VIVLELKGSE TTFMCEYADE TATIVEFLNR
WITFAQSIIS TLT
**SEQ ID NO:13** - IL-2 agonist polypeptide mutein with T3A/C125A- L36I R38S/F42K/Y45A/E62A/ E68V 
APASSSTKKT QLQLEHLLLD LQMILNGINN YKNPKITSML TKKFAMPKKA TELKHLQCLE
EALKPLEVVL NLAQSKNFHL RPRDLISNIN VIVLELKGSE TTFMCEYADE TATIVEFLNR
WITFAQSIIS TLT
**SEQ ID NO:14** - IL-2 agonist polypeptide mutein with T3A/C125A- L36I R38S/F42A/Y45N/E62A/ E68V 
APASSSTKKT QLQLEHLLLD LQMILNGINN YKNPKITSML TAKFNMPKKA TELKHLQCLE
EALKPLEVVL NLAQSKNFHL RPRDLISNIN VIVLELKGSE TTFMCEYADE TATIVEFLNR
WITFAQSIIS TLT
**SEQ ID NO:15** - IL-2 agonist polypeptide mutein with T3A/C125A- L36I R38S/F42A/Y45R/E62A /E68V 
APASSSTKKT QLQLEHLLLD LQMILNGINN YKNPKITSML TAKFRMPKKA TELKHLQCLE
EALKPLEVVL NLAQSKNFHL RPRDLISNIN VIVLELKGSE TTFMCEYADE TATIVEFLNR
WITFAQSIIS TLT
**SEQ ID NO:16** - IL-2 agonist polypeptide mutein with T3A/ L36I R38S/F42A/Y45A/A73T/C125A 
APASSSTKKT QLQLEHLLLD LQMILNGINN YKNPKITSML TAKFAMPKKA TELKHLQCLE
EELKPLEEVL NLTQSKNFHL RPRDLISNIN VIVLELKGSE TTFMCEYADE TATIVEFLNR
WITFAQSIIS TLT
**SEQ ID NO:17** - IL-2 agonist polypeptide mutein with T3A/K35N/ L36I R38S/F42A/Y45A/A73T/ C125A 
APASSSTKKT QLQLEHLLLD LQMILNGINN YKNPNITSML TAKFAMPKKA TELKHLQCLE
EELKPLEEVL NLTQSKNFHL RPRDLISNIN VIVLELKGSE TTFMCEYADE TATIVEFLNR
WITFAQSIIS TLT
**SEQ ID NO:18 -** IL-2 agonist polypeptide mutein with T3A/ L36I R38S/F42I/Y45A/A73T/C125A 
APASSSTKKT QLQLEHLLLD LQMILNGINN YKNPKITSML TIKFAMPKKA TELKHLQCLE
EELKPLEEVL NLTQSKNFHL RPRDLISNIN VIVLELKGSE TTFMCEYADE TATIVEFLNR
WITFSQSIIS TLT
**SEQ ID NO:19** - IL-2 agonist polypeptide mutein with T3A/K35N/ L36I R38S/F42I/Y45A/A73T/ C125A 
APASSSTKKT QLQLEHLLLD LQMILNGINN YKNPNITSML TIKFAMPKKA TELKHLQCLE
EELKPLEEVL NLTQSKNFHL RPRDLISNIN VIVLELKGSE TTFMCEYADE TATIVEFLNR
WITFAQSIIS TLT
**SEQ ID NO:20** - IL-2 agonist polypeptide mutein with T3A/ L36I R38S/F42K/Y45A/A73T/C125A 
APASSSTKKT QLQLEHLLLD LQMILNGINN YKNPKITSML TKKFAMPKKA TELKHLQCLE
EELKPLEEVL NLTQSKNFHL RPRDLISNIN VIVLELKGSE TTFMCEYADE TATIVEFLNR
WITFAQSIIS TLT
**SEQ ID NO:21** - IL-2 agonist polypeptide mutein with T3A/K35N/ L36I R38S/F42K/Y45A/A73T/C125A 
APASSSTKKT QLQLEHLLLD LQMILNGINN YKNPNITSML TKKFAMPKKA TELKHLQCLE
EELKPLEEVL NLTQSKNFHL RPRDLISNIN VIVLELKGSE TTFMCEYADE TATIVEFLNR
WITFAQSIIS TLT
**SEQ ID NO:22** - IL-2 agonist polypeptide mutein with T3A/ L36I R38S/F42A/Y45N/A73T/C125A 
APASSSTKKT QLQLEHLLLD LQMILNGINN YKNPKITSML TAKFNMPKKA TELKHLQCLE
EELKPLEEVL NLTQSKNFHL RPRDLISNIN VIVLELKGSE TTFMCEYADE TATIVEFLNR
WITFAQSIIS TLT
**SEQ ID NO:23** - IL-2 agonist polypeptide mutein with T3A/ L36I R38S/F42A/Y45R/A73T/C125A 
APASSSTKKT QLQLEHLLLD LQMILNGINN YKNPKITSML TAKFRMPKKA TELKHLQCLE
EELKPLEEVL NLTQSKNFHL RPRDLISNIN VIVLELKGSE TTFMCEYADE TATIVEFLNR
WITFAQSIIS TLT
**SEQ ID NO:24** - IL-2 agonist polypeptide mutein with T3A/ L36I R38S/F42A/Y45A/E62A/C125A/Q126W 
APASSSTKKT QLQLEHLLLD LQMILNGINN YKNPKITSML TAKFAMPKKA TELKHLQCLE
EALKPLEEVL NLAQSKNFHL RPRDLISNIN VIVLELKGSE TTFMCEYADE TATIVEFLNR
WITFAWSIIS TLT
**SEQ ID NO:25** - IL-2 agonist polypeptide mutein with T3A/ L36I R38S/F42K/Y45A/E62A/A73T/ C125A 
APASSSTKKT QLQLEHLLLD LQMILNGINN YKNPKITSML TKKFAMPKKA TELKHLQCLE
EALKPLEEVL NLTQSKNFHL RPRDLISNIN VIVLELKGSE TTFMCEYADE TATIVEFLNR
WITFAQSIIS TLT
**SEQ ID NO:26** - IL-2 agonist polypeptide mutein with T3A/ L36I R38S/F42A/Y45N/E62A/A73T/ C125A 
APASSSTKKT QLQLEHLLLD LQMILNGINN YKNPKITSML TAKFNMPKKA TELKHLQCLE
EALKPLEEVL NLTQSKNFHL RPRDLISNIN VIVLELKGSE TTFMCEYADE TATIVEFLNR
WITFAQSIIS TLT
**SEQ ID NO:27** - IL-2 agonist polypeptide mutein with T3A/ L36I R38S/F42A/Y45R/E62A/A73T/ C125A 
APASSSTKKT QLQLEHLLLD LQMILNGINN YKNPKITSML TAKFRMPKKA TELKHLQCLE
EALKPLEEVL NLTQSKNFHL RPRDLISNIN VIVLELKGSE TTFMCEYADE TATIVEFLNR
WITFAQSIIS TLT
**SEQ ID NO:28** - IL-2 agonist polypeptide mutein with T3A/K35N/ L36I R38S/F42A/Y45N/ A73T/C125A 
APASSSTKKT QLQLEHLLLD LQMILNGINN YKNPNITSML TAKFNMPKKA TELKHLQCLE
EELKPLEEVL NLTQSKNFHL RPRDLISNIN VIVLELKGSE TTFMCEYADE TATIVEFLNR
WITFAQSIIS TLT
**SEQ ID NO:29 -** IL-2 agonist polypeptide mutein with T3A/K35N/ L36I R38S/F42A/Y45R/ A73T/C125A 
APASSSTKKT QLQLEHLLLD LQMILNGINN YKNPNITSML TAKFRMPKKA TELKHLQCLE
EELKPLEEVL NLTQSKNFHL RPRDLISNIN VIVLELKGSE TTFMCEYADE TATIVEFLNR
WITFAQSIIS TLT
**SEQ ID NO:30** - IL-2 agonist polypeptide mutein with T3A/C125A- L36I R38S/F42A/Y45A/E62A/Q126H 
APASSSTKKT QLQLEHLLLD LQMILNGINN YKNPKITSML TAKFAMPKKA TELKHLQCLE
EALKPLEEVL NLAQSKNFHL RPRDLISNIN VIVLELKGSE TTFMCEYADE TATIVEFLNR
WITFAHSIIS TLT
**SEQ ID NO:31** - IL-2 agonist polypeptide mutein with T3A/C125A- L36I R38S/F42A/Y45A/E62A/Q126A 
APASSSTKKT QLQLEHLLLD LQMILNGINN YKNPKITSML TAKFAMPKK ATELKHLQCL
EEALKPLEEV LNLAQSKNFH LRPRDLISNI NVIVLELKGS ETTFMCEYAD ETATIVEFLN
RWITFAASII STLT
**SEQ ID NO:32** - IL-2 agonist polypeptide mutein with T3A/C125A- L36I R38S/F42A/Y45A/E62A/Q126H 
APASSSTKKT QLQLEHLLLD LQMILNGINN YKNPKITSML TAKFAMPKKA TELKHLQCLE
EALKPLEEVL NLAQSKNFHL RPRDLISNIN VIVLELKGSE TTFMCEYADE TATIVEFLNR
WITFATSIIS TLT
**SEQ ID NO:33** - IL-2 agonist polypeptide mutein with T3A/C125A- L36I R38S/F42A/Y45A/E62A/Q126H 
APASSSTKKT QLQLEHLLLD LQMILNGINN YKNPKITSML TAKFAMPKKA TELKHLQCLE
EALKPLEEVL NLAQSKNFHL RPRDLISNIN VIVLELKGSE TTFMCEYADE TATIVEFLNR
WITFAWSIIS TLT
**SEQ ID NO:34** - Anti-IL-2 scFv C7 
DIQMTQSPSS LSASVGDRVT ITCRASQDIS NYLNWYQQKP GKAPKLLIYG ASSLQSGVPS
RFSGSGSGTD FTLTISSLQP EDFATYYCQQ TYTIPLTFGQ GTKVEIKAGG GGSGGGGSGG
GGSQVQLVQS GAEVKKPGSS VKVSCKASGG TFTSYAINWV RQAPGQGLEW MGRIIPIIGR
VDYAQKFQGR VTITADESTS TAYMELSSLR SEDTAVYYCA RGFVGGFDVW GQGTLVTVSS
**SEQ ID NO:35** - Anti-IL-2 scFv F7 
EIVLTQSPGT LSLSPGERAT LSCRASQSVS SSYLAWYQQK PGQAPRLLIY DASSRATGIP
DRFSGSGSGT DFTLTISRLE PEDFAVYYCQ QYGSSFPWTF GQGTKVEIKA GGGGSGGGGS
GGGGSQVQLV ESGGGVVQPG RSLRLSCAAS GFTFSTYGMH WVRQAPGKGL EWVAVISHEG
NNKYYADSVK GRFTISRDNS KNTLYLQMNS LRAEDTAVYY CARDWFYGRA RFDPWGQGTL
VTVSS
**SEQ ID NO:36** - Anti-IL-2 scFv G3 
EIVLTQSPGT LSLSPGERAT LSCRASQSVS SSYLAWYQQK PGQAPRLMIY GASSRATGIP
DRFSGSGSGT DFTLTISRLE PEDFAVYYCQ QYESSPPITF GQGTKVEIKA GGGGSGGGGS
GGGGSQVQLV QSGAEVKKPG SSVKVSCKAS GGTFSSYGIH WVRQAPGQGL EWMGGIIPSF
GITNYAQKFQ GRVTITADES TSTAYMELSS LRSEDTAVYY CARYPYSFDH WGQGTLVTVS S
**SEQ ID NO:37** - Human IL-2 Receptor Beta Subunit Extracellular Domain (https://www.uniprot.org/uniprot/P14784) 
AVNGTSQFTC FYNSRANISC VWSQDGALQD TSCQVHAWPD RRRWNQTCEL LPVSQASWAC
NLILGAPDSQ KLTTVDIVTL RVLCREGVRW RVMAIQDFKP FENLRLMAPI SLQVVHVETH
RCNISWEISQ ASHYFERHLE FEARTLSPGH TWEEAPLLTL KQKQEWICLE TLTPDTQYEF
QVRVKPLQGE FTTWSPWSQP LAFRTKPAAL GKDT
**SEQ ID NO:38** - pembrolizumab light chain CX11.56.4 LC 
EIVLTQSPAT LSLSPGERAT LSCRASKGVS TSGYSYLHWY QQKPGQAPRL LIYLASYLES
GVPARFSGSG SGTDFTLTIS SLEPEDFAVY YCQHSRDLPL TFGGGTKVEI KRTVAAPSVF
IFPPSDEQLK SGTASVVCLL NNFYPREAKV QWKVDNALQS GNSQESVTEQ DSKDSTYSLS
STLTLSKADY EKHKVYACEV THQGLSSPVT KSFNRGEC**
**SEQ ID NO:39** - pembrolizumab HC, IgG1, L234A, L235A, G237A CX11.56.5 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKAEP KSCDKTHTCP PCPAPEAAGA
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LPAPIEKTIS KAKGQPREPQ VYTLPPSREE
MTKNQVSLTC LVKGFYPSDI AVEWESNGQP ENNYKTTPPV LDSDGSFFLY SKLTVDKSRW
QQGNVFSCSV MHEALHNHYT QKSLSLSPGK**
**SEQ ID NO:40** - ASKG812K-PD1 L234A, L235A, G237A - IL2vvQ126T/L36I 2xG4S 
QVQ LVQSGVEVKK PGASVKVSCK ASGYTFTNYY MYWVRQAPGQ GLEWMGGINP
SNGGTNFNEK FKNRVTLTTD SSTTTAYMEL KSLQFDDTAV YYCARRDYRF DMGFDYWGQG
TTVTVSSAST KGPSVFPLAP SSKSTSGGTA ALGCLVKDYF PEPVTVSWNS GALTSGVHTF
PAVLQSSGLY SLSSVVTVPS SSLGTQTYIC NVNHKPSNTK VDKKAEPKSC DKTHTCPPCP
APEAAGAPSV FLFPPKPKDT LMISRTPEVT CVVVDVSHED PEVKFNWYVD GVEVHNAKTK
PREEQYNSTY RVVSVLTVLH QDWLNGKEYK CKVSNKALPA PIEKTISKAK GQPREPQVYT
LPPSREEMTK NQVSLTCLVK GFYPSDIAVE WESNGQPENN YKTTPPVLDS DGSFFLYSKL
TVDKSRWQQG NVFSCSVMHE ALHNHYTQKS LSLSPGGGGS GGGGSAPASS STKKTQLQLE
HLLLDLQMIL NGINNYKNPK ITSMLTAKFA MPKKATELKH LQCLEEALKP LEEVLNLAQS
KNFHLRPRDL ISNINVIVLE LKGSETTFMC EYADETATIV EFLNRWITFA TSIISTLT**
**SEQ ID NO:41** - ASKG812K-PD1 L234A, L235A, G237A -IL2vvQ126A/L36I 2xG4S 
QVQ LVQSGVEVKKP GASVKVSCKA SGYTFTNYYM YWVRQAPGQG LEWMGGINPS
NGGTNFNEKF KNRVTLTTDS STTTAYMELK SLQFDDTAVY YCARRDYRFD MGFDYWGQGT
TVTVSSASTK GPSVFPLAPS SKSTSGGTAA LGCLVKDYFP EPVTVSWNSG ALTSGVHTFP
AVLQSSGLYS LSSVVTVPSS SLGTQTYICN VNHKPSNTKV DKKAEPKSCD KTHTCPPCPA
PEAAGAPSVF LFPPKPKDTL MISRTPEVTC VVVDVSHEDP EVKFNWYVDG VEVHNAKTKP
REEQYNSTYR VVSVLTVLHQ DWLNGKEYKC KVSNKALPAP IEKTISKAKG QPREPQVYTL
PPSREEMTKN QVSLTCLVKG FYPSDIAVEW ESNGQPENNY KTTPPVLDSD GSFFLYSKLT
VDKSRWQQGN VFSCSVMHEA LHNHYTQKSL SLSPGGGGSG GGGSAPASSS TKKTQLQLEH
LLLDLQMILN GINNYKNPKI TSMLTAKFAM PKKATELKHL QCLEEALKPL EEVLNLAQSK
NFHLRPRDLI SNINVIVLEL KGSETTFMCE YADETATIVE FLNRWITFAA SIISTLT**
**SEQ ID NO:42** - ASKG812K-HC L234A, L235A, G237A -IL2scFv-C7 cleavable QVQ LVQSGVEVKK PGASVKVSCK ASGYTFTNYY MYWVRQAPGQ GLEWMGGINP 
SNGGTNFNEK FKNRVTLTTD SSTTTAYMEL KSLQFDDTAV YYCARRDYRF DMGFDYWGQG
TTVTVSSAST KGPSVFPLAP SSKSTSGGTA ALGCLVKDYF PEPVTVSWNS GALTSGVHTF
PAVLQSSGLY SLSSVVTVPS SSLGTQTYIC NVNHKPSNTK VDKKAEPKSC DKTHTCPPCP
APEAAGAPSV FLFPPKPKDT LMISRTPEVT CVVVDVSHED PEVKFNWYVD GVEVHNAKTK
PREEQYNSTY RVVSVLTVLH QDWLNGKEYK CKVSNKALPA PIEKTISKAK GQPREPQVYT
LPPSREEMTK NQVSLTCLVK GFYPSDIAVE WESNGQPENN YKTTPPVLDS DGSFFLYSKL
TVDKSRWQQG NVFSCSVMHE ALHNHYTQKS LSLSPGGGGS PLGLAGGGGS GGGGSDIQMT
QSPSSLSASV GDRVTITCRA SQDISNYLNW YQQKPGKAPK LLIYGASSLQ SGVPSRFSGS
GSGTDFTLTI SSLQPEDFAT YYCQQTYTIP LTFGQGTKVE IKAGGGGSGG GGSGGGGSQV
QLVQSGAEVK KPGSSVKVSC KASGGTFTSY AINWVRQAPG QGLEWMGRII PIIGRVDYAQ
KFQGRVTITA DESTSTAYME LSSLRSEDTA VYYCARGFVG GFDVWGQGTL VTVSS**
**SEQ ID NO:43** - ASKG812K-HC L234A, L235A, G237A -IL2scFv-C7 dual-cleavable 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKAEP KSCDKTHTCP PCPAPEAAGA
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LPAPIEKTIS KAKGQPREPQ VYTLPPSREE
MTKNQVSLTC LVKGFYPSDI AVEWESNGQP ENNYKTTPPV LDSDGSFFLY SKLTVDKSRW
QQGNVFSCSV MHEALHNHYT QKSLSLSPGG GGSPLGLAGR QARVVNAGGG SGGGGSDIQM
TQSPSSLSAS VGDRVTITCR ASQDISNYLN WYQQKPGKAP KLLIYGASSL QSGVPSRFSG
SGSGTDFTLT ISSLQPEDFA TYYCQQTYTI PLTFGQGTKV EIKAGGGGSG GGGSGGGGSQ
VQLVQSGAEV KKPGSSVKVS CKASGGTFTS YAINWVRQAP GQGLEWMGRI IPIIGRVDYA
QKFQGRVTIT ADESTSTAYM ELSSLRSEDT AVYYCARGFV GGFDVWGQGT LVTVSS**
**SEQ ID NO:44** - ASKG812K-HC L234A, L235A, G237A -IL2scFv-C7 dual-cleavable 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKAEP KSCDKTHTCP PCPAPEAAGA
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LPAPIEKTIS KAKGQPREPQ VYTLPPSREE
MTKNQVSLTC LVKGFYPSDI AVEWESNGQP ENNYKTTPPV LDSDGSFFLY SKLTVDKSRW
QQGNVFSCSV MHEALHNHYT QKSLSLSPGG GGSISSGLLS SGGSLSGRSD NHGGGSGGGG
SDIQMTQSPS SLSASVGDRV TITCRASQDI SNYLNWYQQK PGKAPKLLIY GASSLQSGVP
SRFSGSGSGT DFTLTISSLQ PEDFATYYCQ QTYTIPLTFG QGTKVEIKAG GGGSGGGGSG
GGGSQVQLVQ SGAEVKKPGS SVKVSCKASG GTFTSYAINW VRQAPGQGLE WMGRIIPIIG
RVDYAQKFQG RVTITADEST STAYMELSSL RSEDTAVYYC ARGFVGGFDV WGQGTLVTVS
S**
**SEQ ID NO:45** - ASKG812K-PD1 L234A, L235A, G237A-IL2vv Q126T/L36I knob-2xG4S 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKAEP KSCDKTHTCP PCPAPEAAGA
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LPAPIEKTIS KAKGQPREPQ VYTLPPCREE
MTKNQVSLWC LVKGFYPSDI AVEWESNGQP ENNYKTTPPV LDSDGSFFLY SKLTVDKSRW
QQGNVFSCSV MHEALHNHYT QKSLSLSPGG GGSGGGGSAP ASSSTKKTQL QLEHLLLDLQ
MILNGINNYK NPKITSMLTA KFAMPKKATE LKHLQCLEEA LKPLEEVLNL AQSKNFHLRP
RDLISNINVI VLELKGSETTF MCEYADETAT IVEFLNRWIT FATSIISTLT**
**SEQ ID NO:46 -** ASKG812K-PD1 L234A, L235A, G237A - IL2vvQ126A/L36I knob-2xG4S 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKAEP KSCDKTHTCP PCPAPEAAGA
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LPAPIEKTIS KAKGQPREPQ VYTLPPCREE
MTKNQVSLWC LVKGFYPSDI AVEWESNGQP ENNYKTTPPV LDSDGSFFLY SKLTVDKSRW
QQGNVFSCSV MHEALHNHYT QKSLSLSPGG GGSGGGGSAP ASSSTKKTQL QLEHLLLDLQ
MILNGINNYK NPKITSMLTA KFAMPKKATE LKHLQCLEEA LKPLEEVLNL AQSKNFHLRP
RDLISNINVI VLELKGSETT FMCEYADETA TIVEFLNRWI TFAASIISTL T**
**SEQ ID NO:47** - ASKG812K-HC L234A, L235A, G237A, hole 
QVQLVQSGVE VKKPGASVKV SCKASGYTFT NYYMYWVRQA PGQGLEWMGG INPSNGGTNFNEK
FKNRVTLTTD SSTTTAYMEL KSLQFDDTAV YYCARRDYRF DMGFDYWGQG TTVTVSSAST
KGPSVFPLAP SSKSTSGGTA ALGCLVKDYF PEPVTVSWNS GALTSGVHTF PAVLQSSGLY
SLSSVVTVPS SSLGTQTYIC NVNHKPSNTK VDKKAEPKSC DKTHTCPPCP APEAAGAPSV
FLFPPKPKDT LMISRTPEVT CVVVDVSHED PEVKFNWYVD GVEVHNAKTK PREEQYNSTY
RVVSVLTVLH QDWLNGKEYK CKVSNKALPA PIEKTISKAK GQPREPQVCT LPPSREEMTK
NQVSLSCAVK GFYPSDIAVE WESNGQPENN YKTTPPVLDS DGSFFLVSKL TVDKSRWQQG
NVFSCSVMHE ALHNHYTQKS LSLSPGK**
**SEQ ID NO:48** - ASKG812K-HC- L234A, L235A, G237A- IL2vvQ126T/L36I-Clv-C7 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKAEP KSCDKTHTCP PCPAPEAAGA
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LPAPIEKTIS KAKGQPREPQ VYTLPPSREE
MTKNQVSLTC LVKGFYPSDI AVEWESNGQP ENNYKTTPPV LDSDGSFFLY SKLTVDKSRW
QQGNVFSCSV MHEALHNHYT QKSLSLSPGG GGSGGGGSAP ASSSTKKTQL QLEHLLLDLQ
MILNGINNYK NPKITSMLTA KFAMPKKATE LKHLQCLEEA LKPLEEVLNL AQSKNFHLRP
RDLISNINVI VLELKGSETT FMCEYADETA TIVEFLNRWI TFATSIISTL TGGGGSPLGL
AGGGGSGGGG SDIQMTQSPS SLSASVGDRV TITCRASQDI SNYLNWYQQK PGKAPKLLIY
GASSLQSGVP SRFSGSGSGT DFTLTISSLQ PEDFATYYCQ QTYTIPLTFG QGTKVEIKAG
GGGSGGGGSG GGGSQVQLVQ SGAEVKKPGS SVKVSCKASG GTFTSYAINW VRQAPGQGLE
WMGRIIPIIG RVDYAQKFQG RVTITADEST STAYMELSSL RSEDTAVYYC ARGFVGGFDV
WGQGTLVTVS S**
**SEQ ID NO:49** - ASKG812K-HC- L234A, L235A, G237A- IL2vvQ126A/L36I-Clv-C7 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKAEP KSCDKTHTCP PCPAPEAAGA
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LPAPIEKTIS KAKGQPREPQ VYTLPPSREE
MTKNQVSLTC LVKGFYPSDI AVEWESNGQP ENNYKTTPPV LDSDGSFFLY SKLTVDKSRW
QQGNVFSCSV MHEALHNHYT QKSLSLSPGG GGSGGGGSAP ASSSTKKTQL QLEHLLLDLQ
MILNGINNYK NPKITSMLTA KFAMPKKATE LKHLQCLEEA LKPLEEVLNL AQSKNFHLRP
RDLISNINVI VLELKGSETT FMCEYADETA TIVEFLNRWI TFAASIISTL TGGGGSPLGL
AGGGGSGGGG SDIQMTQSPS SLSASVGDRV TITCRASQDI SNYLNWYQQK PGKAPKLLIY
GASSLQSGVP SRFSGSGSGT DFTLTISSLQ PEDFATYYCQ QTYTIPLTFG QGTKVEIKAG
GGGSGGGGSG GGGSQVQLVQ SGAEVKKPGS SVKVSCKASG GTFTSYAINW VRQAPGQGLE
WMGRIIPIIG RVDYAQKFQG RVTITADEST STAYMELSSL RSEDTAVYYC ARGFVGGFDV
WGQGTLVTVS S**
**SEQ ID NO:50** - ASKG812K-HC- L234A, L235A, G237A-G3-IL2vvQ126T/L36I 
QVQLVQSGVE VKKPGASVKV SCKASGYTFT NYYMYWVRQA PGQGLEWMGG INPSNGGTNFNEK
FKNRVTLTTD SSTTTAYMEL KSLQFDDTAV YYCARRDYRF DMGFDYWGQG TTVTVSSAST
KGPSVFPLAP SSKSTSGGTA ALGCLVKDYF PEPVTVSWNS GALTSGVHTF PAVLQSSGLY
SLSSVVTVPS SSLGTQTYIC NVNHKPSNTK VDKKAEPKSC DKTHTCPPCP APEAAGAPSV
FLFPPKPKDT LMISRTPEVT CVVVDVSHED PEVKFNWYVD GVEVHNAKTK PREEQYNSTY
RVVSVLTVLH QDWLNGKEYK CKVSNKALPA PIEKTISKAK GQPREPQVYT LPPSREEMTK
NQVSLTCLVK GFYPSDIAVE WESNGQPENN YKTTPPVLDS DGSFFLYSKL TVDKSRWQQG
NVFSCSVMHE ALHNHYTQKS LSLSPGGGGS GGGGSEIVLT QSPGTLSLSP GERATLSCRA
SQSVSSSYLA WYQQKPGQAP RLMIYGASSR ATGIPDRFSG SGSGTDFTLT ISRLEPEDFA
VYYCQQYESS PPITFGQGTK VEIKSGGGGS GGGGSGGGGS QVQLVQSGAE VKKPGSSVKV
SCKASGGTFS SYGIHWVRQA PGQGLEWMGG IIPSFGITNY AQKFQGRVTI TADESTSTAY
MELSSLRSED TAVYYCARYP YSFDHWGQGT LVTVSSGGGS GGGGSAAGGG SGGGGSAPAS
SSTKKTQLQL EHLLLDLQMI LNGINNYKNP KITSMLTAKF AMPKKATELK HLQCLEEALK
PLEEVLNLAQ SKNFHLRPRD LISNINVIVL ELKGSETTFM CEYADETATI VEFLNRWITF
ATSIISTLT* *
**SEQ ID NO:51** - ASKG812K-HC- L234A, L235A, G237A-F7-IL2vvQ126T/L36I 
QVQLVQSGVE VKKPGASVKV SCKASGYTFT NYYMYWVRQA PGQGLEWMGG INPSNGGTNFNEK
FKNRVTLTTD SSTTTAYMEL KSLQFDDTAV YYCARRDYRF DMGFDYWGQG TTVTVSSAST
 
KGPSVFPLAP SSKSTSGGTA ALGCLVKDYF PEPVTVSWNS GALTSGVHTF PAVLQSSGLY
SLSSVVTVPS SSLGTQTYIC NVNHKPSNTK VDKKAEPKSC DKTHTCPPCP APEAAGAPSV
FLFPPKPKDT LMISRTPEVT CVVVDVSHED PEVKFNWYVD GVEVHNAKTK PREEQYNSTY
RVVSVLTVLH QDWLNGKEYK CKVSNKALPA PIEKTISKAK GQPREPQVYT LPPSREEMTK
NQVSLTCLVK GFYPSDIAVE WESNGQPENN YKTTPPVLDS DGSFFLYSKL TVDKSRWQQG
NVFSCSVMHE ALHNHYTQKS LSLSPGGGGS GGGGSEIVLT QSPGTLSLSP GERATLSCRA
SQSVSSSYLA WYQQKPGQAP RLLIYDASSR ATGIPDRFSG SGSGTDFTLT ISRLEPEDFA
VYYCQQYGSS FPWTFGQGTK VEIKSGGGGS GGGGSGGGGS QVQLVESGGG VVQPGRSLRL
SCAASGFTFS TYGMHWVRQA PGKGLEWVAV ISHEGNNKYY ADSVKGRFTI SRDNSKNTLY
LQMNSLRAED TAVYYCARDW FYGRARFDPW GQGTLVTVSS GGGSGGGGSA AGGGSGGGGS
APASSSTKKT QLQLEHLLLD LQMILNGINN YKNPKITSML TAKFAMPKKA TELKHLQCLE
EALKPLEEVL NLAQSKNFHL RPRDLISNIN VIVLELKGSE TTFMCEYADE TATIVEFLNR
WITFATSIIS TLT**
**SEQ ID NO:52 - OKT8 L1** 
DIKMTQSPSS LSASVGDRVT ITCRTSRSIS QYLAWYQEKP GKTNKLLIYS GSTLQSGIPS
RFSGSGSGTD FILTISSLQP EDFATYYCQQ HNENPLTFGA GTKLEIK
**SEQ ID NO:53** - **OKT8 H1** 
EVQLQQSAGE VKKPGASVKV SCKASGFNIK DTYIHWVRQA PGKGLEWMG RIDPANDNTL
YASKFQGRVT ITADTSTNTA YMELSSLRSE DTAVYYCGRG YGYYVFDHW GQGTTVTVSS
[NO]
**SEQ ID NO:54 - OKT8 H2** 
QVQLVQSGAE VKKPGASVKV SCKASGFNIK DTYIHWVRQA PGKGLEWMG RIDPANDNTL
YASKFQGRVT ITADTSINTA YMELSRLRSD DTAVYYCGRG YGYYVFDHW GQGTTVTVSS
**SEQ ID NO:55-124** - Cleavable peptide linkers 
SGRSA (SEQ ID NO:55)
GGGGSISSGL LSSGGSGGSL GGSGRSANAI LEGGGGSGGG GS (SEQ ID NO:56)
GGGGSISSGL LSSGGSLGGS GRSANAILEG GGGS (SEQ ID NO:57)
GGGGSLGGSG RSANAILEGG SGGSISSGLL SSGGGGS (SEQ ID NO:58)
GGGGSLGGSG RSANAILEGG SISSGLLSSG GGGS (SEQ ID NO:59)
GGGGSLGGSG RSANAILEGG SGGSISSGLL SSGGGGSGGG GS (SEQ ID NO:60)
GGGGSLGGSG RSANAILEGG GGSGGGGSGG GGS (SEQ ID NO:61)
GGGGSGGGGS GGGGSISSGL LSSGGGGS (SEQ ID NO:62)
GGGGSLSGRS DNHGGGGS (SEQ ID NO:63)
(GGGGS)nGGWHTGRN(GGGGS)m (SEQ ID NO:64)
(GGGGS)nTGRGPSWV(GGGGS)m (SEQ ID NO:65)
(GGGGS)nSARGPSRW(GGGGS)m (SEQ ID NO:66)
(GGGGS)nTARGPSFK(GGGGS)m (SEQ ID NO:67)
(GGGGS)nTARGPSW(GGGGS)m (SEQ ID NO:68)
(GGGGS)nLSGRSDNH(GGGGS)m (SEQ ID NO:69)
(GGGGS)nLGGSGRSANAILEGPLGVR (GGGGS)m (SEQ ID NO:70)
(GGGGS)nLGGSGRSANAILEGGSGPLGVR (GGGGS)m (SEQ ID NO:71)
(GGGGS)nLGGSGRSANAILEGGSGGSGPLGVR (GGGGS)m (SEQ ID NO:72)
(GGGGS)nISSGLLSSLSGRSDNH(GGGGS)m (SEQ ID NO:73)
(GGGGS)nISSGLLSSGGSLSGRSDNH(GGGGS)m (SEQ ID NO:74)
(GGGGS)nISSGLLSSGGSGGGSLSGRSDNH(GGGGS)m (SEQ ID NO:75)
wherein n = 0, 1, 2, 3, or 4; and wherein m = 0, 1, 2, 3, or 4
VNGGGGSGPLGVRAAQPA (SEQ ID NO:76)
GGGGSGPLGVRGGGGS (SEQ ID NO:77)
GGGGSGPLGVRGGS (SEQ ID NO:78)
(GGGGS) n1(QGQSGQ) n2 PLGL(GGGGS) n3 (SEQ ID NO:79)
(GGGGS) n1 (QGQSGQ) n2 HTGRSGAL(GGGGS) n3 (SEQ ID NO:80)
 
(GGGGS) n1 (QGQSGQ) n2 PLTGRSGG(GGGGS) n3 (SEQ ID NO:81)
(GGGGS) n1 (QGQSGQ) n2 AARGPAIH(GGGGS) n3 (SEQ ID NO:82)
(GGGGS) n1 (QGQSGQ) n2 RGPAFNPM(GGGGS) n3 (SEQ ID NO:83)
(GGGGS) n1 (QGQSGQ) n2 SSRGPAYL(GGGGS) n3 (SEQ ID NO:84)
(GGGGS) n1 (QGQSGQ) n2 RGPATPIM(GGGGS) n3 (SEQ ID NO:85)
(GGGGS) n1 (QGQSGQ) n2 RGPA(GGGGS) n3 (SEQ ID NO:86)
(GGGGS) n1 (QGQSGQ) n2 GGQPSGMWGW(GGGGS) n3 (SEQ ID NO:87)
(GGGGS) n1 (QGQSGQ) n2 FPRPLGITGL(GGGGS) n3 (SEQ ID NO:88)
(GGGGS) n1 (QGQSGQ) n2 VILMPLGFLGP(GGGGS) n3 (SEQ ID NO:89)
(GGGGS) n1 (QGQSGQ) n2 SPLTGRSG(GGGGS) n3 (SEQ ID NO:90)
(GGGGS) n1 (QGQSGQ) n2 SAGFSLPA(GGGGS) n3 (SEQ ID NO:91)
(GGGGS) n1 (QGQSGQ) n2 LAPLGLQRR(GGGGS) n3 (SEQ ID NO:92)
(GGGGS) n1 (QGQSGQ) n2 SGGPLGVR(GGGGS) n3 (SEQ ID NO:93)
(GGGGS) n1 (QGQSGQ) n2 GPLGVR(GGGGS) n3 (SEQ ID NO:94)
(GGGGS) n1 (QGQSGQ) n2 ISSGLLSS(GGGGS) n3 (SEQ ID NO:95)
(GGGGS) n1 (QGQSGQ) n2 QNQALRMA(GGGGS) n3 (SEQ ID NO:96)
(GGGGS) n1 (QGQSGQ) n2 AQNLLGMV(GGGGS) n3 (SEQ ID NO:97)
(GGGGS) n1 (QGQSGQ) n2 STFPFGMF(GGGGS) n3 (SEQ ID NO:98)
(GGGGS) n1 (QGQSGQ) n2 PVGYTSSL(GGGGS) n3 (SEQ ID NO:99)
(GGGGS) n1 (QGQSGQ) n2 DWLYWPGI(GGGGS) n3 (SEQ ID NO:100)
(GGGGS) n1 (QGQSGQ) n2 MIAPVAYR(GGGGS) n3 (SEQ ID NO:101)
(GGGGS) n1 (QGQSGQ) n2 RPSPMWAY(GGGGS) n3 (SEQ ID NO:102)
(GGGGS) n1 (QGQSGQ) n2 WATPRPMR(GGGGS) n3 (SEQ ID NO:103)
(GGGGS) n1 (QGQSGQ) n2 FRLLDWQW(GGGGS) n3 (SEQ ID NO:104)
(GGGGS) n1 (QGQSGQ) n2 LKAAPRWA(GGGGS) n3 (SEQ ID NO:105)
(GGGGS) n1 (QGQSGQ) n2 GPSHLVLT(GGGGS) n3 (SEQ ID NO:106)
(GGGGS) n1 (QGQSGQ) n2 LPGGLSPW(GGGGS) n3 (SEQ ID NO:107)
(GGGGS) n1 (QGQSGQ) n2 MGLFSEAG(GGGGS) n3 (SEQ ID NO:108)
(GGGGS) n1 (QGQSGQ) n2 SPLPLRVP(GGGGS) n3 (SEQ ID NO:109)
(GGGGS) n1 (QGQSGQ) n2 RMHLRSLG(GGGGS) n3 (SEQ ID NO:110)
(GGGGS) n1 (QGQSGQ) n2 LAAPLGLL(GGGGS) n3 (SEQ ID NO:111)
(GGGGS) n1 (QGQSGQ) n2 AVGLLAPP(GGGGS) n3 (SEQ ID NO:112)
(GGGGS) n1 (QGQSGQ) n2 LLAPSHRA(GGGGS) n3 (SEQ ID NO:113)
(GGGGS) n1 (QGQSGQ) n2 PAGLWLDP(GGGGS) n3 (SEQ ID NO:114)
(GGGGS) n1 (QGQSGQ) n2 ISSGLSS(GGGGS) n3 (SEQ ID NO:115)
(GGGGS) n1 ISSGLLSSGGSGGSLSGRSDNH(GGGGS) n3 (SEQ ID NO:116)
(GGGGS) n1 LSGRSDNHGGSGGSISSGLLSS(GGGGS) n3 (SEQ ID NO:117)
(GGGGS)n1 (QGQSGQ) n2 LSGRSDNH(GGGGS) n3 (SEQ ID NO:118)
(GGGGS) n1 (QGQSGQ) n2 TARGPSFK(GGGGS) n3 (SEQ ID NO:119)
wherein n1 = 0, 1, 2, 3, or 4; n2 = 0 or 1; and n3 = 0, 1, 2, 3, or
4
VPLSLYSGRSA (SEQ ID NO:120)
PLGLAG (SEQ ID NO:121)
LSGRSDNH (SEQ ID NO:122)
ISSGLLSS (SEQ ID NO:123)
GPLGVR (SEQ ID NO:124)
**SEQ ID NO:125** - ASKG812K-PD1 L234A, L235A, G237A - IL2v/L36I 2xG4S 
QVQLVQSGVE VKKPGASVKV SCKASGYTFT NYYMYWVRQA PGQGLEWMGG INPSNGGTNFNEK
FKNRVTLTTD SSTTTAYMEL KSLQFDDTAV YYCARRDYRF DMGFDYWGQG TTVTVSSAST
KGPSVFPLAP SSKSTSGGTA ALGCLVKDYF PEPVTVSWNS GALTSGVHTF PAVLQSSGLY
SLSSVVTVPS SSLGTQTYIC NVNHKPSNTK VDKKAEPKSC DKTHTCPPCP APEAAGAPSV
FLFPPKPKDT LMISRTPEVT CVVVDVSHED PEVKFNWYVD GVEVHNAKTK PREEQYNSTY
RVVSVLTVLH QDWLNGKEYK CKVSNKALPA PIEKTISKAK GQPREPQVYT LPPSREEMTK
NQVSLTCLVK GFYPSDIAVE WESNGQPENN YKTTPPVLDS DGSFFLYSKL TVDKSRWQQG
 
NVFSCSVMHE ALHNHYTQKS LSLSPGGGGS GGGGSAPASS STKKTQLQLE HLLLDLQMIL
NGINNYKNPK ITSMLTAKFA MPKKATELKH LQCLEEALKP LEEVLNLAQS KNFHLRPRDL
ISNINVIVLE LKGSETTFMC EYADETATIV EFLNRWITFA QSIISTLT**
**SEQ ID NO:126 -** trastuzumab light chain 
DIQMTQSPSS LSASVGDRVT ITCRASQDVN TAVAWYQQKP GKAPKLLIYS ASFLYSGVPS
RFSGSRSGTD FTLTISSLQP EDFATYYCQQ HYTTPPTFGQ GTKVEIKRTV AAPSVFIFPP
SDEQLKSGTA SVVCLLNNFY PREAKVQWKV DNALQSGNSQ ESVTEQDSKD STYSLSSTLT
LSKADYEKHK VYACEVTHQG LSSPVTKSFN RGEC
**SEQ ID NO:127** - trastuzumab heavy chain 
EVQLVESGGG LVQPGGSLRL SCAASGFNIK DTYIHWVRQA PGKGLEWVAR IYPTNGYTRY
ADSVKGRFTI SADTSKNTAY LQMNSLRAED TAVYYCSRWG GDGFYAMDYW GQGTLVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKVEP PKSCDKTHTC PPCPAPELLG
GPSVFLFPPK PKDTLMISRT PEVTCVVVDV SHEDPEVKFN WYVDGVEVHN AKTKPREEQY
NSTYRVVSVL TVLHQDWLNG KEYKCKVSNK ALPAPIEKTI SKAKGQPREP QVYTLPPSRD
ELTKNQVSLT CLVKGFYPSD IAVEWESNGQ PENNYKTTPP VLDSDGSFFL YSKLTVDKSR
WQQGNVFSCS VMHEALHNHY TQKSLSLSPG K
**SEQ ID NO:128** - rituximab light chain 
QIVLSQSPAI LSASPGEKVT MTCRASSSVS YIHWFQQKPG SSPKPWIYAT SNLASGVPVR
FSGSGSGTSY SLTISRVEAE DAATYYCQQW TSNPPTFGGG TKLEIKRTVA APSVFIFPPS
DEQLKSGTAS VVCLLNNFYP REAKVQWKVD NALQSGNSQE SVTEQDSKDS TYSLSSTLTL
SKADYEKHKV YACEVTHQGL SSPVTKSFNR GEC
**SEQ ID NO:129 -** rituximab heavy chain 
QVQLQQPGAE LVKPGASVKM SCKASGYTFT SYNMHWVKQT PGRGLEWIGA IYPGNGDTSY
NQKFKGKATL TADKSSSTAY MQLSSLTSED SAVYYCARST YYGGDWYFNV WGAGTTVTVS
AASTKGPSVF PLAPSSKSTS GGTAALGCLV KDYFPEPVTV SWNSGALTSG VHTFPAVLQS
SGLYSLSSVV TVPSSSLGTQ TYICNVNHKP SNTKVDKKAE PKSCDKTHTC PPCPAPELLG
GPSVFLFPPK PKDTLMISRT PEVTCVVVDV SHEDPEVKFN WYVDGVEVHN AKTKPREEQY
NSTYRVVSVL TVLHQDWLNG KEYKCKVSNK ALPAPIEKTI SKAKGQPREP QVYTLPPSRD
ELTKNQVSLT CLVKGFYPSD IAVEWESNGQ PENNYKTTPP VLDSDGSFFL YSKLTVDKSR
WQQGNVFSCS VMHEALHNHY TQKSLSLSPG K
**SEQ ID NO:130** - brentuximab light chain 
DIVLTQSPAS LAVSLGQRAT ISCKASQSVD FDGDSYMNWY QQKPGQPPKV LIYAASNLES
GIPARFSGSG SGTDFTLNIH PVEEEDAATY YCQQSNEDPW TFGGGTKLEI KRTVAAPSVF
IFPPSDEQLK SGTASVVCLL NNFYPREAKV QWKVDNALQS GNSQESVTEQ DSKDSTYSLS
STLTLSKADY EKHKVYACEV THQGLSSPVT KSFNRGEC
**SEQ ID NO:131** - brentuximab heavy chain 
QIQLQQSGPE VVKPGASVKI SCKASGYTFT DYYITWVKQK PGQGLEWIGW IYPGSGNTKY
NEKFKGKATL TVDTSSSTAF MQLSSLTSED TAVYFCANYG NYWFAYWGQG TQVTVSAAST
KGPSVFPLAP SSKSTSGGTA ALGCLVKDYF PEPVTVSWNS GALTSGVHTF PAVLQSSGLY
SLSSVVTVPS SSLGTQTYIC NVNHKPSNTK VDKKVEPKSC DKTHTCPPCP APELLGGPSV
FLFPPKPKDT LMISRTPEVT CVVVDVSHED PEVKFNWYVD GVEVHNAKTK PREEQYNSTY
RVVSVLTVLH QDWLNGKEYK CKVSNKALPA PIEKTISKAK GQPREPQVYT LPPSRDELTK
NQVSLTCLVK GFYPSDIAVE WESNGQPENN YKTTPPVLDS DGSFFLYSKL TVDKSRWQQG
NVFSCSVMHE ALHNHYTQKS LSLSPG
**SEQ ID NO:132** - cetuximab light chain 
DILLTQSPVI LSVSPGERVS FSCRASQSIG TNIHWYQQRT NGSPRLLIKY ASESISGIPS
RFSGSGSGTD FTLSINSVES EDIADYYCQQ NNNWPTTFGA GTKLELKRTV AAPSVFIFPP
 
SDEQLKSGTA SVVCLLNNFY PREAKVQWKV DNALQSGNSQ ESVTEQDSKD STYSLSSTLT
LSKADYEKHK VYACEVTHQG LSSPVTKSFN RGEC
**SEQ ID NO:133 -** cetuximab heavy chain 
QVQLKQSGPG LVQPSQSLSI TCTVSGFSLT NYGVHWVRQS PGKGLEWLGV IWSGGNTDYN
TPFTSRLSIN KDNSKSQVFF KMNSLQSNDT AIYYCARALT YYDYEFAYWG QGTLVTVSAA
STKGPSVFPL APSSKSTSGG TAALGCLVKD YFPEPVTVSW NSGALTSGVH TFPAVLQSSG
LYSLSSVVTV PSSSLGTQTY ICNVNHKPSN TKVDKKVEPK SCDKTHTCPP CPAPELLGGP
SVFLFPPKPK DTLMISRTPE VTCVVVDVSH EDPEVKFNWY VDGVEVHNAK TKPREEQYNS
TYRVVSVLTV LHQDWLNGKE YKCKVSNKAL PAPIEKTISK AKGQPREPQV YTLPPSRDEL
TKNQVSLTCL VKGFYPSDIA VEWESNGQPE NNYKTTPPVL DSDGSFFLYS KLTVDKSRWQ
QGNVFSCSVM HEALHNHYTQ KSLSLSPGK
**SEQ ID NO:134** - panitumumab light chain 
DIQMTQSPSS LSASVGDRVT ITCQASQDIS NYLNWYQQKP GKAPKLLIYD ASNLETGVPS
RFSGSGSGTD FTFTISSLQP EDIATYFCQH FDHLPLAFGG GTKVEIKRTV AAPSVFIFPP
SDEQLKSGTA SVVCLLNNFY PREAKVQWKV DNALQSGNSQ ESVTEQDSKD STYSLSSTLT
LSKADYEKHK VYACEVTHQG LSSPVTKSFN RGEC
**SEQ ID NO:135** - panitumumab heavy chain 
GHIYYSGNTN YNPSLKSRLT ISIDTSKTQF SLKLSSVTAA DTAIYYCVRD RVTGAFDIWG
QGTMVTVSSA STKGPSVFPL APCSRSTSES TAALGCLVKD YFPEPVTVSW NSGALTSGVH
TFPAVLQSSG LYSLSSVVTV PSSNFGTQTY TCNVDHKPSN TKVDERKCCV ECPAGPSVFL
FPPKPKDTLM ISRTPEVTCV VVDVSHEDPE VQFNWYVDGV EVHNAKTKPR EEQFNSTFRV
VSVLTVVHQD WLNGKEYKCK VSNKGLPAPI EKTISKTKGQ PREPQVYTLP PSREEMTKNQ
VSLTCLVKGF YPSDIAVEWE SNGQPENNYK TTPPMLDSDG SFFLYSKLTV DKSRWQQGNV
FSCSVMHEAL HNHYTQKSLS LSPGK
**SEQ ID NO:136 -** anti-c-MET antibody light chain 
DIVMTQAAPS VPVTPGESVS ISCRSSKSLL HSNGNTYLYW FLQRPGQSPQ VLIYRMSNLA
SGVPDRFSGS GSGTAFTLRI RRVEAEDVGV YYCMQNLEYP FTFGGGTKLE IKRTVAAPSV
FIFPPSDEQL KSGTASVVCL LNNFYPREAK VQWKVDNALQ SGNSQESVTE QDSKDSTYSL
SSTLTLSKAD YEKHKVYACE VTHQGLSSPV TKSFNRGEC
**SEQ ID NO:137** - anti-c-MET antibody heavy chain 
QVQLQQSGPE LVKSGASVKM SCKASGNTLK DDHVHWVKQR PGQGLEWIGW IYPGGGRTRY
NEKFKGKTTL TADKPSSTVN MLLSSLTSED SAIYFCTNLV FDVWGAGTTV TVSSASTKGP
SVFPLAPSSK STSGGTAALG CLVKDYFPEP VTVSWNSGAL TSGVHTFPAV LQSSGLYSLS
SVVTVPSSSL GTQTYICNVN HKPSNTKVDK KVEPKSCDKT HTCPPCPAPE LLGGPSVFLF
PPKPKDTLMI SRTPEVTCVV VDVSHEDPEV KFNWYVDGVE VHNAKTKPRE EQYNSTYRVV
SVLTVLHQDW LNGKEYKCKV SNKALPAPIE KTISKAKGQP REPQVYTLPP SREEMTKNQV
SLTCLVKGFY PSDIAVEWES NGQPENNYKT TPPVLDSDGS FFLYSKLTVD KSRWQQGNVF
SCSVMHEALH NHYTQKSLSL SPGK
**SEQ ID NO:138** - anti-GPC3 antibody light chain 
DVVMTQSPLS LPVTPGEPAS ISCRSSQSLV HSNANTYLHW YLQKPGQSPQ LLIYKVSNRF
SGVPDRFSGS GSGTDFTLKI SRVEAEDVGV YYCSQNTHVP PTFGQGTKLE IKRTVAAPSV
FIFPPSDEQL KSGTASVVCL LNNFYPREAK VQWKVDNALQ SGNSQESVTE QDSKDSTYSL
SSTLTLSKAD YEKHKVYACE VTHQGLSSPV TKSFNRGEC
**SEQ ID NO:139** - anti-GPC3 antibody heavy chain 
QVQLVQSGAE VKKPGASVKV SCKASGYTFT DYEMHWVRQA PGQGLEWMGA LDPKTGDTAY
SQKFKGRVTL TADKSTSTAY MELSSLTSED TAVYYCTRFY SYTYWGQGTL VTVSSASTKG
PSVFPLAPSS KSTSGGTAAL GCLVKDYFPE PVTVSWNSGA LTSGVHTFPA VLQSSGLYSL
SSVVTVPSSS LGTQTYICNV NHKPSNTKVD KKVEPKSCDK THTCPPCPAP ELLGGPSVFL
 
FPPKPKDTLM ISRTPEVTCV VVDVSHEDPE VKFNWYVDGV EVHNAKTKPR EEQYNSTYRV
VSVLTVLHQD WLNGKEYKCK VSNKALPAPI EKTISKAKGQ PREPQVYTLP PSREEMTKNQ
VSLTCLVKGF YPSDIAVEWE SNGQPENNYK TTPPVLDSDG SFFLYSKLTV DKSRWQQGNV
FSCSVMHEAL HNHYTQKSLS LSPGK
**SEQ ID NO:140** - anti-Claudin 18.2 antibody light chain 
DIVMTQSPSS LTVTAGEKVT MSCKSSQSLL NSGNQKNYLT WYQQKPGQPP KLLIYWASTR
ESGVPDRFTG SGSGTDFTLT ISSVQAEDLA VYYCQNDYSY PFTFGSGTKL EIKRTVAAPS
VFIFPPSDEQ LKSGTASVVC LLNNFYPREA KVQWKVDNAL QSGNSQESVT EQDSKDSTYS
LSSTLTLSKA DYEKHKVYAC EVTHQGLSSP VTKSFNRGEC
**SEQ ID NO:141** - anti-Claudin 18.2 antibody heavy chain 
QVQLQQPGAE LVRPGASVKL SCKASGYTFT SYWINWVKQR PGQGLEWIGN IYPSDSYTNY
NQKFKDKATL TVDKSSSTAY MQLSSPTSED SAVYYCTRSW RGNSFDYWGQ GTTLTVSSAS
TKGPSVFPLA PSSKSTSGGT AALGCLVKDY FPEPVTVSWN SGALTSGVHT FPAVLQSSGL
YSLSSVVTVP SSSLGTQTYI CNVNHKPSNT KVDKKVEPKS CDKTHTCPPC PAPELLGGPS
VFLFPPKPKD TLMISRTPEV TCVVVDVSHE DPEVKFNWYV DGVEVHNAKT KPREEQYNST
YRVVSVLTVL HQDWLNGKEY KCKVSNKALP APIEKTISKA KGQPREPQVY TLPPSRDELT
KNQVSLTCLV KGFYPSDIAV EWESNGQPEN NYKTTPPVLD SDGSFFLYSK LTVDKSRWQQ
GNVFSCSVMH EALHNHYTQK SLSLSPGK
**SEQ ID NO:142** - anti-Trop-2 antibody light chain CDR1
   KASQDVSIAVA
**SEQ ID NO:143** - anti-Trop-2 antibody light chain CDR2
   SASYRYT
**SEQ ID NO:144** - anti-Trop-2 antibody light chain CDR3
   QQHYITPLT
**SEQ ID NO:145** - anti-Trop-2 antibody heavy chain CDR1
   NYGMN
**SEQ ID NO:146** - anti-Trop-2 antibody heavy chain CDR2
   WINTYTGEPTYTDDFKG
**SEQ ID NO:147** - anti-Trop-2 antibody heavy chain CDR3
   GGFGSSYWYFDV
**SEQ ID NO:148** - anti-mesothelin antibody light chain CDR1
   SASSSVSYMH
**SEQ ID NO:149** - anti-mesothelin antibody light chain CDR2
   DTSKLAS
**SEQ ID NO:150** - anti-mesothelin antibody light chain CDR3
   QQWSGYPLT
**SEQ ID NO:151** - anti-mesothelin antibody heavy chain CDR1
   GYTMN
**SEQ ID NO:152** - anti-mesothelin antibody heavy chain CDR2
   LITPYNGASSYNQKFRG
**SEQ ID NO:153** - anti-mesothelin antibody heavy chain CDR3
   GGYDGRGFDY
**SEQ ID NO:154** - Light Chain variable domain of PR1A3. 
GDIVMTQSQR FMSTSVGDRV SVTCKASQNV GTNVAWYQQ KPGQSPKALI YSASYRYSG
VPDRFTGSG- SGTDFTLTIS NVQSEDLAEY FCHQYYTYP LFTFGSGTKL EMKR
**SEQ ID NO:155** - Heavy Chain variable domain of PR1A3. 
QVKLQQSGPE LKKPGETVKI SCKASGYTFT VFGMNWVKQA PGKGLKWMGW INTKTGEATY
VEEFKGRFAF SLETSATTAY LQINNLKNED TAKYFCARWD FYDYVEAMDY WGQGTTVTVSS
**SEQ ID NO:156** - Humanized Light Chain variable domain PR1A3. 
DIQMTQSPSS LSASVGDRVT ITCKASQNVG TNVAWYQQKP GKAPKLLIYS ASYRYSGVPS
RFSGSGSGTD FTFTISSLQP EDIATYYCHQ YYTYPLFTFG QGTKVEIKR
**SEQ ID NO:157** - Humanized Heavy Chain variable domain of PR1A3. 
QVQLVQSGSE LKKPGASVKV SCKASGYTFT VFGMNWVRQA PGQGLEWMGW INTKTGEATY
VEEFKGRFVF SLDTSVSTAY LQISSLKADD TAVYYCARWD FYDYVEAMDY WGQGTTVTVS S
**SEQ ID NO:158** - Anti-FAP version 1 LC (protein sequence) 
QIVLTQSPAT LSLSPGERAT LSCSASSGVN FMHWYQQKPG QAPRRLIFDT SKLASGIPAR
FSGSGSGTDY TLTISSLEPE DFAVYYCQQW SFNPPTFGQG TKVEIKRTVA APSVFIFPPS
DEQLKSGTAS VVCLLNNFYP REAKVQWKVD NALQSGNSQE SVTEQDSKDS TYSLSSTLTL
SKADYEKHKV YACEVTHQGL SSPVTKSFNR GEC
**SEQ** ID NO:159 - Anti-FAP LC version 2 (protein sequence) 
QIVLTQSPAT LSLSPGERAT LSCSASSGVN FMHWYQQKPG QAPKRLIFDT SKLASGVPAR
FSGSGSGTDY TLTISSLEPE DFAVYYCQQW SFNPPTFGQG TKVEIKRTVA APSVFIFPPS
DEQLKSGTAS VVCLLNNFYP REAKVQWKVD NALQSGNSQE SVTEQDSKDS TYSLSSTLTL
SKADYEKHKV YACEVTHQGL SSPVTKSFNR GEC
**SEQ ID NO:160** - Anti-FAP VH (protein sequence) 
QVQLVQSGAE VKKPGASVKV SCKASGYTFT NNGINWLRQA PGQGLEWMGE IYPRSTNTLY
AQKFQGRVTI TADRSSNTAY MELSSLRSED TAVYFCARTL TAPFAFWGQG TLVTVSSAST
KGPSVFPLAP SSKSTSGGTA ALGCLVKDYF PEPVTVSWNS GALTSGVHTF PAVLQSSGLY
SLSSVVTVPS SSLGTQTYIC NVNHKPSNTK VDKKVEPKSC DKTHTCPPCP APELLGGPSV
FLFPPKPKDT LMISRTPEVT CVVVDVSHED PEVKFNWYVD GVEVHNAKTK PREEQYNSTY
RVVSVLTVLH QDWLNGKEYK CKVSNKALPA PIEKTISKAK GQPREPQVYT LPPSRDELTK
NQVSLTCLVK GFYPSDIAVE WESNGQPENN YKTTPPVLDS DGSFFLYSKL TVDKSRWQQG
NVFSCSVMHE ALHNHYTQKS LSLSPGK
**SEQ ID NO:161** - Humanized Light Chain variable domain of FAPalpha antibody BIBH1 
DIVMTQSPDS LAVSLGERAT INCKSSQSLL YSRNQKNYLA WYQQKPGQPP KLLIFWASTR
ESGVPDRFSG SGFGTDFTLT ISSLQAEDVA VYYCQQYFSY PLTFGQGTKV EIK
**SEQ ID NO:162** - Humanized Heavy Chain variable domain FAPalpha antibody BIBH1 
QVQLVQSGAE VKKPGASVK VSCKTSRYTFT EYTIHWVRQA PGQRLEWIGG INPNNGIPNY
NQKFKGRVTI TVDTSASTAY MELSSLRSED TAVYYCARRR IAYGYDEGHA MDYWGQGTLV
TVSS
**SEQ ID NO:163** - Humanized H8 anti-5T4 version 1 VH (protein sequence) 
QVQLVQSGAE VKKPGASVKVS CKASGYSFT GYYMHWVKQSP GQGLEWIGR INPNNGVTLY
NQKFKDRVTM TRDTSISTAYM ELSRLRSDD TAVYYCARSTM ITNYVMDYW GQGTLWTVSS
**SEQ ID NO:164** - Humanized H8 anti-5T4 VH version 2 (protein sequence) 
QVQLVQSGAE VKKPGASVKV SCKASGYSFT GYYMHWVRQA PGQGLEWMGR INPNNGVTLY
NQKFKDRVTM TRDTSISTAY MELSRLRSDD TAVYYCARST MITNYVMDYW GQGTLVTVSS
**SEQ ID NO:165** - Humanized H8 anti-5T4 version 1 VL (protein sequence) 
DIVMTQSPDS LAVSLGERAT INCKASQSVS NDVAWYQQKP GQSPKLLISY TSSRYAGVPD
RFSGSGSGTD FTLTISSLQA EDVAVYFCQQ DYNSPPTFGG GTKLEIK
**SEQ ID NO:166** - Humanized H8 anti-5T4 VL version 2 (protein sequence) 
DIVMTQSPDS LAVSLGERAT INCKASQSVS NDVAWYQQKP GQPPKLLIYY TSSRYAGVPD
RFSGSGSGTD FTLTISSLQA EDVAVYYCQQ DYNSPPTFGG GTKLEIK
**SEQ ID NO:167** - Anti-PDL1 atezolizumab LC 
DIQMTQSPSS LSASVGDRVT ITCRASQDVS TAVAWYQQKP GKAPKLLIYS ASFLYSGVPS
RFSGSGSGTD FTLTISSLQP EDFATYYCQQ YLYHPATFGQ GTKVEIKRTV AAPSVFIFPP
SDEQLKSGTA SVVCLLNNFY PREAKVQWKV DNALQSGNSQ ESVTEQDSKD STYSLSSTLT
LSKADYEKHK VYACEVTHQG LSSPVTKSFN RGEC
**SEQ ID NO:168** - Anti-PDL1 atezolizumab HC (protein sequence) 
EVQLVESGGG LVQPGGSLRL SCAASGFTFS DSWIHWVRQA PGKGLEWVAW ISPYGGSTYY
ADSVKGRFTI SADTSKNTAY LQMNSLRAED TAVYYCARRH WPGGFDYWGQ GTLVTVSSAS
TKGPSVFPLA PSSKSTSGGT AALGCLVKDY FPEPVTVSWN SGALTSGVHT FPAVLQSSGL
YSLSSVVTVP SSSLGTQTYI CNVNHKPSNT KVDKKVEPKS CDKTHTCPPC PAPELLGGPS
VFLFPPKPKD TLMISRTPEV TCVVVDVSHE DPEVKFNWYV DGVEVHNAKT KPREEQYAST
YRVVSVLTVL HQDWLNGKEY KCKVSNKALP APIEKTISKA KGQPREPQVY TLPPSREEMT
KNQVSLTCLV KGFYPSDIAV EWESNGQPEN NYKTTPPVLD SDGSFFLYSK LTVDKSRWQQ
GNVFSCSVMH EALHNHYTQK SLSLSPGK
**SEQ ID NO:169** - human albumin 
DAHKSEVAHR FKDLGEENFK ALVLIAFAQY LQQCPFEDHV KLVNEVTEFA KTCVADESAE
NCDKSLHTLF GDKLCTVATL RETYGEMADC CAKQEPERNE CFLQHKDDNP NLPRLVRPEV
DVMCTAFHDN EETFLKKYLY EIARRHPYFY APELLFFAKR YKAAFTECCQ AADKAACLLP
KLDELRDEGK ASSAKQRLKC ASLQKFGERA FKAWAVARLS QRFPKAEFAE VSKLVTDLTK
VHTECCHGDL LECADDRADL AKYICENQDS ISSKLKECCE KPLLEKSHCI AEVENDEMRA
DLPSLAADFV ESKDVCKNYA EAKDVFLGMF LYEYARRHPD YSVVLLLRLA KTYETTLEKC
CAAADPHECY AKVFDEFKPL VEEPQNLIKQ NCELFEQLGE YKFQNALLVR YTKKVPQVST
PTLVEVSRNL GKVGSKCCKH PEAKRMPCAE DYLSVVLNQL CVLHEKTPVS DRVTKCCTES
LVNRRPCFSA LEVDETYVPK EFNAETFTFH ADICTLSEKE RQIKKQTALV ELVKHKPKAT
KEQLKAVMDD FAAFVEKCCK ADDKETCFAE EGKKLVAASQ AALGL
**SEQ ID NO:170** - anti-IL-2 antibody heavy chain CDR1
   TSYAI
**SEQ ID NO:171** - anti-IL-2 antibody heavy chain CDR2
   RIIPIIGRVD YAQKFQG
**SEQ ID NO:172** - anti-IL-2 antibody heavy chain CDR3
   FVGGFDV
**SEQ ID NO:173** - anti-IL-2 antibody light chain CDR1
   RASQDISNYLN
**SEQ ID NO:174** - anti-IL-2 antibody light chain CDR2
   GASSLQS
**SEQ ID NO:175** - anti-IL-2 antibody light chain CDR3
   QTYTIPL
**SEQ ID NO:176** - IL-2 agonist polypeptide mutein with T3A/R38S/F42A/Y45A/E62A/C125S 
APASSSTKKT QLQLEHLLLD LQMILNGINN YKNPKLTSML TAKFAMPKKA TELKHLQCLE
EALKPLEEVL NLAQSKNFHL RPRDLISNIN VIVLELKGSE TTFMCEYADE TATIVEFLNR
WITFSQSIIS TLT
**SEQ ID NOs:177-183** - Non-cleavable linker 
GGS (SEQ ID NO:177)
GGGGS (SEQ ID NO:178)
GGSGGS (SEQ ID NO:179)
GGGGSGGGGS (SEQ ID NO:180)
GGGGSGGGGS GGGGS (SEQ ID NO:181)
GGGGSAAGGG GAGGGGA (SEQ ID NO:182)
GGGGSGGGGS AAGGGGSGGG GS (SEQ ID NO:183).
**SEQ ID NO:184** - IL-2 agonist polypeptide mutein with T3A/L36I/R38S/F42A/Y45A/E62A/C125A 
APASSSTKKT QLQLEHLLLD LQMILNGINN YKNPKITSML TAKFAMPKKA TELKHLQCLE
EALKPLEEVL NLAQSKNFHL RPRDLISNIN VIVLELKGSE TTFMCEYADE TATIVEFLNR
WITFAQSIIS TLT
**SEQ ID NO:185** - ASKG812N-PD1 L234A, L235A,G237A -IL2vL36I 2xG4S 
QVQ LVESGGGVVQ PGRSLRLDCK ASGITFSNSG MHWVRQAPGK GLEWVAVIWY
DGSKRYYADS VKGRFTISRD NSKNTLFLQM NSLRAEDTAV YYCATNDDYW GQGTLVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKAEP KSCDKTHTCP PCPAPEAAGA
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LPAPIEKTIS KAKGQPREPQ VYTLPPSREE
MTKNQVSLTC LVKGFYPSDI AVEWESNGQP ENNYKTTPPV LDSDGSFFLY SKLTVDKSRW
QQGNVFSCSV MHEALHNHYT QKSLSLSPGG GGSGGGGSAP ASSSTKKTQL QLEHLLLDLQ
MILNGINNYK NPKITSMLTA KFAMPKKATE LKHLQCLEEA LKPLEEVLNL AQSKNFHLRP
RDLISNINVI VLELKGSETT FMCEYADETA TIVEFLNRWI TFAQSIISTL T
**SEQ ID NO:186** - ASKG812N-HC L234A, L235A,G237A -IL2scFv-C7 cleavable (CDRs are bolded and underlined) 
QVQ LVESGGGVVQ PGRSLRLDCK ASGITFSNSG MHWVRQAPGK GLEWVAVIWY
DGSKRYYADS VKGRFTISRD NSKNTLFLQM NSLRAEDTAV YYCATNDDYW GQGTLVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKAEP KSCDKTHTCP PCPAPEAAGA
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LPAPIEKTIS KAKGQPREPQ VYTLPPSREE
MTKNQVSLTC LVKGFYPSDI AVEWESNGQP ENNYKTTPPV LDSDGSFFLY SKLTVDKSRW
QQGNVFSCSV MHEALHNHYT QKSLSLSPGG GGSISSGLLS GRSDNHGGSG GSDIQMTQSP
SSLSASVGDR VTITCRASQD ISNYLNWYQQ KPGKAPKLLI YGASSLQSGV PSRFSGSGSG
TDFTLTISSL QPEDFATYYC QQTYTIPLTF GQGTKVEIKA GGGGSGGGGS GGGGSQVQLV
QSGAEVKKPG SSVKVSCKAS GGTFTSYAIN WVRQAPGQGL EWMGRIIPII GRVDYAQKFQ
GRVTITADES TSTAYMELSS LRSEDTAVYY CARGFVGGFD VWGQGTLVTV SS**
**SEQ ID NO: 187** - 812N-mut4 
QVQLVESGGG VVQPGRSLRL DCKASGITFS NSGMHWVRQA PGKGLEWVAV IWYDGSKRYY
ADSVKGRFTI SRDNSKNTLF LQMNSLRAED TAVYYCATND DYWGQGTLVT VSSASTKGPS
VFPLAPSSKS TSGGTAALGC LVKDYFPEPV TVSWNSGALT SGVHTFPAVL QSSGLYSLSS
VVTVPSSSLG TQTYICNVNH KPSNTKVDKK VEPKSCDKTH TCPPCPAPEA AGAPSVFLFP
PKPKDTLMIS RTPEVTCVVV DVSHEDPEVK FNWYVDGVEV HNAKTKPREE QYNSTYRVVS
VLTVLHQDWL NGKEYKCKVS NKALPAPIEK TISKAKGQPR EPQVYTLPPS REEMTKNQVS
LTCLVKGFYP SDIAVEWESN GQPENNYKTT PPVLDSDGSF FLYSKLTVDK SRWQQGNVFS
CSVMHEALHN HYTQKSLSLS PGGGGSGGGG SEIVLTQSPG TLSLSPGERA TLSCRASQSV
SSSYLAWYQQ KPGQAPRLLI YDASSRATGI PDRFSGSGSG TDFTLTISRL EPEDFAVYYC
QQYGSSFPWT FGQGTKVEIK SGGGGSGGGG SAAGGGGSGG GGSQVQLVES GGGVVQPGRS
LRLSCAASGF TFSTYGMHWV RQAPGKGLEW VAVISHEGNN KYYADSVKGR FTISRDNSKN
TLYLQMNSLR AEDTAVYYCA RDWFYGRARF DPWGQGTLVT VSSGGGGSGG GGSAAGGGGS
GGGGSAPASS STKKTQLQLE HLLLDLQMIL NGINNYKNPK ITSMLTFKFY MPKKATELKH
LQCLEEELKP LEEVLNLAQS KNFHLRPRDL ISNINVIVLE LKGSETTFMC EYADETATIV
EFLNRWITFA QSIISTLT**
**SEQ ID NO: 188** - **Cleavable Peptide Linker**
   GGGGSGPLGV RGSLSGRSDN HGGGGS
**SEQ ID NO:189** - PD1-Nivolumab-LC CX3.73.3 
EIVLTQSP ATLSLSPGER ATLSCRASQS VSSYLAWYQQ KPGQAPRLLI YDASNRATGI
PARFSGSGSG TDFTLTISSL EPEDFAVYYC QQSSNWPRTF GQGTKVEIKR TVAAPSVFIF
PPSDEQLKSG TASVVCLLNN FYPREAKVQW KVDNALQSGN SQESVTEQDS KDSTYSLSST
LTLSKADYEK HKVYACEVTH QGLSSPVTKS FNRGEC**
**SEQ ID NO:190** - Anti-IL-2 VL 
DIQMTQSPSS LSASVGDRVT ITCRASQDIS NYLNWYQQKP GKAPKLLIYG ASSLQSGVPS
RFSGSGSGTD FTLTISSLQP EDFATYYCQQ TYTIPLTFGQ GTKVEIK
**SEQ ID NO:191** - Anti-IL-2 VH 
QVQLVQSGAE VKKPGSSVKV SCKASGGTFT SYAINWVRQA PGQGLEWMGR IIPIIGRVDY
AQKFQGRVTI TADESTSTAY MELSSLRSED TAVYYCARGF VGGFDVWGQG TLVTVSS
**SEQ ID NO:192** - Anti-IL-2 VL 
EIVLTQSPGT LSLSPGERAT LSCRASQSVS SSYLAWYQQK PGQAPRLLIY DASSRATGIP
DRFSGSGSGT DFTLTISRLE PEDFAVYYCQ QYGSSFPWTF GQGTKVEIK
**SEQ ID NO:193** - Anti-IL-2 VH 
QVQLVESGGG VVQPGRSLRL SCAASGFTFS TYGMHWVRQA PGKGLEWVAV ISHEGNNKYY
ADSVKGRFTI SRDNSKNTLY LQMNSLRAED TAVYYCARDW FYGRARFDPW GQGTLVTVSS
**SEQ ID NO:194** - Anti-IL-2 VL 
EIVLTQSPGT LSLSPGERAT LSCRASQSVS SSYLAWYQQK PGQAPRLMIY GASSRATGIP
DRFSGSGSGT DFTLTISRLE PEDFAVYYCQ QYESSPPITF GQGTKVEIK
**SEQ ID NO:195** - Anti-IL-2 VH 
QVQLVQSGAE VKKPGSSVKV SCKASGGTFS SYGIHWVRQA PGQGLEWMGG IIPSFGITNYA
QKFQGRVTIT ADESTSTAYM ELSSLRSEDT AVYYCARYPY SFDHWGQGTL VTVSS
**SEQ ID NO:196** - Fc-Igg4-hole-scFv C7(K1-69), ASKG222.CX12.48.1 
AESKYGPPCP PCPAPEAAGG PSVFLFPPKP KDTLMISRTP EVTCVVVDVS QEDPEVQFNW
YVDGVEVHNA KTKPREEQFN STYRVVSVLT VLHQDWLNGK EYKCKVSNKG LPSSIEKTIS
 
KAKGQPREPQ VCTLPPSQEE MTKNQVSLSC AVKGFYPSDI AVEWESNGQP ENNYKTTPPV
LDSDGSFFLV SRLTVDKSRW QEGNVFSCSV MHEALHNHYT QKSLSLSLGA GGGGSGGGGS
AAGGGGSGGG GSDIQMTQSP SSLSASVGDR VTITCRASQD ISNYLNWYQQ KPGKAPKLLI
YGASSLQSGV PSRFSGSGSG TDFTLTISSL QPEDFATYYC QQTYTIPLTF GQGTKVEIKA
GGGGSGGGGS GGGGSQVQLV QSGAEVKKPG SSVKVSCKAS GGTFTSYAIN WVRQAPGQGL
EWMGRIIPII GRVDYAQKFQ GRVTITADES TSTAYMELSS LRSEDTAVYY CARGFVGGFD
VWGQGTLVTV SS**
**SEQ ID NO:197** - Fc-Igg4-hole-scFv H5(K1-69); ASKG222.CX12.48.2 
AESKYGPPCP PCPAPEAAGG PSVFLFPPKP KDTLMISRTP EVTCVVVDVS QEDPEVQFNW
YVDGVEVHNA KTKPREEQFN STYRVVSVLT VLHQDWLNGK EYKCKVSNKG LPSSIEKTIS
KAKGQPREPQ VCTLPPSQEE MTKNQVSLSC AVKGFYPSDI AVEWESNGQP ENNYKTTPPV
LDSDGSFFLV SRLTVDKSRW QEGNVFSCSV MHEALHNHYT QKSLSLSLGA GGGGSGGGGS
AAGGGGSGGG GSEIVLTQSP GTLSLSPGER ATLSCRASQS VSSSYLAWYQ QKPGQAPRLL
IYGASSRATG IPDRFSGSGS GTDFTLTISR LEPEDFAVYY CQQYSRAPGT FGQGTKVEIK
AGGGGSGGGG SGGGGSQVQL VQSGAEVKKP GSSVKVSCKA SGGTFSSYGI HWVRQAPGQG
LEWMGGIIPI LGTANYAQKF QGRVTITADE STSTAYMELS SLRSEDTAVY YCAVPFFDVW
GQGTLVTVSS **
**SEQ ID NO:198** - scFv-C7 -IGG4 YTE hole; ASKG222.CX12.50.1 
DIQMTQSPSS LSASVGDRVT ITCRASQDIS NYLNWYQQKP GKAPKLLIYG ASSLQSGVPS
RFSGSGSGTD FTLTISSLQP EDFATYYCQQ TYTIPLTFGQ GTKVEIKAGG GGSGGGGSGG
GGSQVQLVQS GAEVKKPGSS VKVSCKASGG TFTSYAINWV RQAPGQGLEW MGRIIPIIGR
VDYAQKFQGR VTITADESTS TAYMELSSLR SEDTAVYYCA RGFVGGFDVW GQGTLVTVSS
GGGGSGGGGS AAGGGGSGGG GSAESKYGPP CPPCPAPEAA GGPSVFLFPP KPKDTLYITR
EPEVTCVVVD VSQEDPEVQF NWYVDGVEVH NAKTKPREEQ FNSTYRVVSV LTVLHQDWLN
GKEYKCKVSN KGLPSSIEKT ISKAKGQPRE PQVCTLPPSQ EEMTKNQVSL SCAVKGFYPS
DIAVEWESNG QPENNYKTTP PVLDSDGSFF LVSRLTVDKS RWQEGNVFSC SVMHEALHNH
YTQKSLSLSL GK**
**SEQ ID NO:199** - IL-2-IGG4 Fc, YTE, Knob; ASKG222.CX12.7.5 
APTSSSTKKT QLQLEHLLLD LQMILNGINN YKNPKLTRML TFKFYMPKKA TELKHLQCLE
EELKPLEEVL NLAQSKNFHL RPRDLISNIN VIVLELKGSE TTFMCEYADE TATIVEFLNR
WITFAQSIIS TLTGGGGSGG GGSGGGGSAE SKYGPPCPPC PAPEAAGGPS VFLFPPKPKD
TLYITREPEV TCVVVDVSQE DPEVQFNWYV DGVEVHNAKT KPREEQFNST YRVVSVLTVL
HQDWLNGKEY KCKVSNKGLP SSIEKTISKA KGQPREPQVY TLPPCQEEMT KNQVSLWCLV
KGFYPSDIAV EWESNGQPEN NYKTTPPVLD SDGSFFLYSR LTVDKSRWQE GNVFSCSVMH
EALHNHYTQK SLSLSLGK**
**SEQ ID NO:200** - ASKD982-D1, Hole Chain (No gamma ECD; only beta ECD), ASKG222D1; ASKD982.CX7.56.6, 
AESKYGPPCP PCPAPEAAGG PSVFLFPPKP KDTLMISRTP EVTCVVVDVS QEDPEVQFNW
YVDGVEVHNA KTKPREEQFN STYRVVSVLT VLHQDWLNGK EYKCKVSNKG LPSSIEKTIS
KAKGQPREPQ VCTLPPSQEE MTKNQVSLSC AVKGFYPSDI AVEWESNGQP ENNYKTTPPV
LDSDGSFFLV SRLTVDKSRW QEGNVFSCSV MHEALHNHYT QKSLSLSLGA GGGGSGGGGS
AAGGGGSGGG GSAVNGTSQF TCFYNSRANI SCVWSQDGAL QDTSCQVHAW PDRRRWNQTC
ELLPVSQASW ACNLILGAPD SQKLTTVDIV TLRVLCREGV RWRVMAIQDF KPFENLRLMA
PISLQVVHVE THRCNISWEI SQASHYFERH LEFEARTLSP GHTWEEAPLL TLKQKQEWIC
LETLTPDTQY EFQVRVKPLQ GEFTTWSPWS QPLAFRTKPA ALGKDT**
**SEQ ID NO:201** - ASKD982-C, Knob Chain (only C125A mutation); ASKD982.CX7.56.5 
AESKYGPPCP PCPAPEAAGG PSVFLFPPKP KDTLMISRTP EVTCVVVDVS QEDPEVQFNW
YVDGVEVHNA KTKPREEQFN STYRVVSVLT VLHQDWLNGK EYKCKVSNKG LPSSIEKTIS
 
KAKGQPREPQ VYTLPPCQEE MTKNQVSLWC LVKGFYPSDI AVEWESNGQP ENNYKTTPPV
LDSDGSFFLY SRLTVDKSRW QEGNVFSCSV MHEALHNHYT QKSLSLSLGA GGGGSGGGGS
GGGGSAPTSS STKKTQLQLE HLLLDLQMIL NGINNYKNPK LTRMLTFKFY MPKKATELKH
LQCLEEELKP LEEVLNLAQS KNFHLRPRDL ISNINVIVLE LKGSETTFMC EYADETATIV
EFLNRWITFA QSIISTLT**
**SEQ ID NO:202** - IL-2w C125A - IGG4 YTE knob H435R, Y436F 2xG4S; ASKG222.CX12.25.1 
APTSSSTKKT QLQLEHLLLD LQMILNGINN YKNPKLTRML TFKFYMPKKA TELKHLQCLE
EELKPLEEVL NLAQSKNFHL RPRDLISNIN VIVLELKGSE TTFMCEYADE TATIVEFLNR
WITFAQSIIS TLTGGGGSGG GGSAESKYGP PCPPCPAPEA AGGPSVFLFP PKPKDTLYIT
REPEVTCVVV DVSQEDPEVQ FNWYVDGVEV HNAKTKPREE QFNSTYRVVS VLTVLHQDWL
NGKEYKCKVS NKGLPSSIEK TISKAKGQPR EPQVYTLPPC QEEMTKNQVS LWCLVKGFYP
SDIAVEWESN GQPENNYKTT PPVLDSDGSF FLYSRLTVDK SRWQEGNVFS CSVMHEALHN
RFTQKSLSLS LGK**
**SEQ ID NO:203** - IL-2w C125A - IGG4 YTE knob H435R, Y436F 1xG₄S; ASKG222.CX12.25.2 
APTSSSTKKT QLQLEHLLLD LQMILNGINN YKNPKLTRML TFKFYMPKKA TELKHLQCLE
EELKPLEEVL NLAQSKNFHL RPRDLISNIN VIVLELKGSE TTFMCEYADE TATIVEFLNR
WITFAQSIIS TLTGGGGSAE SKYGPPCPPC PAPEAAGGPS VFLFPPKPKD TLYITREPEV
TCVVVDVSQE DPEVQFNWYV DGVEVHNAKT KPREEQFNST YRVVSVLTVL HQDWLNGKEY
KCKVSNKGLP SSIEKTISKA KGQPREPQVY TLPPCQEEMT KNQVSLWCLV KGFYPSDIAV
EWESNGQPEN NYKTTPPVLD SDGSFFLYSR LTVDKSRWQE GNVFSCSVMH EALHNRFTQK
SLSLSLGK**
**SEQ ID NO:204** - IL-2w C125A- IGG₄ YTE knob H435R, Y436F; ASKG222.CX12.25.3 
APTSSSTKKT QLQLEHLLLD LQMILNGINN YKNPKLTRML TFKFYMPKKA TELKHLQCLE
EELKPLEEVL NLAQSKNFHL RPRDLISNIN VIVLELKGSE TTFMCEYADE TATIVEFLNR
WITFAQSIIS TLTAESKYGP PCPPCPAPEA AGGPSVFLFP PKPKDTLYIT REPEVTCVVV
DVSQEDPEVQ FNWYVDGVEV HNAKTKPREE QFNSTYRVVS VLTVLHQDWL NGKEYKCKVS
NKGLPSSIEK TISKAKGQPR EPQVYTLPPC QEEMTKNQVS LWCLVKGFYP SDIAVEWESN
GQPENNYKTT PPVLDSDGSF FLYSRLTVDK SRWQEGNVFS CSVMHEALHN RFTQKSLSLS
LGK**
**SEQ ID NO:205** - IGG1-LALA -YTE IL-2- T3A/C125A- C7HC 4xG₄S; ASKG222.CX12.65.1 
APASSSTKKT QLQLEHLLLD LQMILNGINN YKNPKLTRML TFKFYMPKKA TELKHLQCLE
EELKPLEEVL NLAQSKNFHL RPRDLISNIN VIVLELKGSE TTFMCEYADE TATIVEFLNR
WITFAQSIIS TLTGGGGSGG GGSAAGGGGS GGGGSQVQLV QSGAEVKKPG SSVKVSCKAS
GGTFTSYAIN WVRQAPGQGL EWMGRIIPII GRVDYAQKFQ GRVTITADES TSTAYMELSS
LRSEDTAVYY CARGFVGGFD VWGQGTLVTV SSASTKGPSV FPLAPSSKST SGGTAALGCL
VKDYFPEPVT VSWNSGALTS GVHTFPAVLQ SSGLYSLSSV VTVPSSSLGT QTYICNVNHK
PSNTKVDKKA EPKSCDKTHT CPPCPAPEAA GGPSVFLFPP KPKDTLYITR EPEVTCVVVD
VSHEDPEVKF NWYVDGVEVH NAKTKPREEQ YNSTYRVVSV LTVLHQDWLN GKEYKCKVSN
KALPAPIEKT ISKAKGQPRE PQVYTLPPSR EEMTKNQVSL TCLVKGFYPS DIAVEWESNG
QPENNYKTTP PVLDSDGSFF LYSKLTVDKS RWQQGNVFSC SVMHEALHNH YTQKSLSLSP
GK**
**SEQ ID NO:206** - IGG1-LALA-YTE IL-2-T3A/C125A-C7HC 2xG₄S; ASKG222.CX12.65.2 
APASSSTKKT QLQLEHLLLD LQMILNGINN YKNPKLTRML TFKFYMPKKA TELKHLQCLE
EELKPLEEVL NLAQSKNFHL RPRDLISNIN VIVLELKGSE TTFMCEYADE TATIVEFLNR
 
WITFAQSIIS TLTGGGGSGG GGSQVQLVQS GAEVKKPGSS VKVSCKASGG TFTSYAINWV
RQAPGQGLEW MGRIIPIIGR VDYAQKFQGR VTITADESTS TAYMELSSLR SEDTAVYYCA
RGFVGGFDVW GQGTLVTVSS ASTKGPSVFP LAPSSKSTSG GTAALGCLVK DYFPEPVTVS
WNSGALTSGV HTFPAVLQSS GLYSLSSVVT VPSSSLGTQT YICNVNHKPS NTKVDKKAEP
KSCDKTHTCP PCPAPEAAGG PSVFLFPPKP KDTLYITREP EVTCVVVDVS HEDPEVKFNW
YVDGVEVHNA KTKPREEQYN STYRVVSVLT VLHQDWLNGK EYKCKVSNKA LPAPIEKTIS
KAKGQPREPQ VYTLPPSREE MTKNQVSLTC LVKGFYPSDI AVEWESNGQP ENNYKTTPPV
LDSDGSFFLY SKLTVDKSRW QQGNVFSCSV MHEALHNHYT QKSLSLSPGK**
**SEQ ID NO:207** - C7-LC ASKG222.CX12.65.3 
DIQMTQSPSS LSASVGDRVT ITCRASQDIS NYLNWYQQKP GKAPKLLIYG ASSLQSGVPS
RFSGSGSGTD FTLTISSLQP EDFATYYCQQ TYTIPLTFGQ GTKVEIKRTV AAPSVFIFPP
SDEQLKSGTA SVVCLLNNFY PREAKVQWKV DNALQSGNSQ ESVTEQDSKD STYSLSSTLT
LSKADYEKHK VYACEVTHQG LSSPVTKSFN RGEC**
**SEQ ID NO:208** - IL-2-T3A/C125A-C7LC 4xG₄S; ASKG222.CX12.65.4 
APASSSTKKT QLQLEHLLLD LQMILNGINN YKNPKLTRML TFKFYMPKKA TELKHLQCLE
EELKPLEEVL NLAQSKNFHL RPRDLISNIN VIVLELKGSE TTFMCEYADE TATIVEFLNR
WITFAQSIIS TLTGGGGSGG GGSAAGGGGS GGGGSDIQMT QSPSSLSASV GDRVTITCRA
SQDISNYLNW YQQKPGKAPK LLIYGASSLQ SGVPSRFSGS GSGTDFTLTI SSLQPEDFAT
YYCQQTYTIP LTFGQGTKVE IKRTVAAPSV FIFPPSDEQL KSGTASVVCL LNNFYPREAK
VQWKVDNALQ SGNSQESVTE QDSKDSTYSL SSTLTLSKAD YEKHKVYACE VTHQGLSSPV
TKSFNRGEC**
**SEQ ID NO:209** - IGG4 Fc-YTE-knob-IL2 T3A,C125A **H435R, Y436F** 2xG₄S; ASKG222.CX12.66.1 
AESKYGPPCP PCPAPEAAGG PSVFLFPPKP KDTLYITREP EVTCVVVDVS QEDPEVQFNW
YVDGVEVHNA KTKPREEQFN STYRVVSVLT VLHQDWLNGK EYKCKVSNKG LPSSIEKTIS
KAKGQPREPQ VYTLPPCQEE MTKNQVSLWC LVKGFYPSDI AVEWESNGQP ENNYKTTPPV
LDSDGSFFLY SRLTVDKSRW QEGNVFSCSV MHEALHNRFT QKSLSLSLGA GGGGSGGGGS
APASSSTKKT QLQLEHLLLD LQMILNGINN YKNPKLTRML TFKFYMPKKA TELKHLQCLE
EELKPLEEVL NLAQSKNFHL RPRDLISNIN VIVLELKGSE TTFMCEYADE TATIVEFLNR
WITFAQSIIS TLT**
**SEQ ID NO:210** - IGG4-Fc YTE-knob-IL2 T3A,C125A **H435R, Y436F** 0xG₄S; ASKG222.CX12.66.2 
AESKYGPPCP PCPAPEAAGG PSVFLFPPKP KDTLYITREP EVTCVVVDVS QEDPEVQFNW
YVDGVEVHNA KTKPREEQFN STYRVVSVLT VLHQDWLNGK EYKCKVSNKG LPSSIEKTIS
KAKGQPREPQ VYTLPPCQEE MTKNQVSLWC LVKGFYPSDI AVEWESNGQP ENNYKTTPPV
LDSDGSFFLY SRLTVDKSRW QEGNVFSCSV MHEALHNRFT QKSLSLSLGA APASSSTKKT
QLQLEHLLLD LQMILNGINN YKNPKLTRML TFKFYMPKKA TELKHLQCLE EELKPLEEVL
NLAQSKNFHL RPRDLISNIN VIVLELKGSE TTFMCEYADE TATIVEFLNR WITFAQSIIS
TLT**
**SEQ ID NO:211** - IL-2-C125A-C7HC 4xG₄S; ASKG222.CX12.76.1 
APTSSSTKKT QLQLEHLLLD LQMILNGINN YKNPKLTRML TFKFYMPKKA TELKHLQCLE
EELKPLEEVL NLAQSKNFHL RPRDLISNIN VIVLELKGSE TTFMCEYADE TATIVEFLNR
WITFAQSIIS TLTGGGGSGG GGSAAGGGGS GGGGSQVQLV QSGAEVKKPG SSVKVSCKAS
GGTFTSYAIN WVRQAPGQGL EWMGRIIPII GRVDYAQKFQ GRVTITADES TSTAYMELSS
LRSEDTAVYY CARGFVGGFD VWGQGTLVTV SSASTKGPSV FPLAPSSKST SGGTAALGCL
VKDYFPEPVT VSWNSGALTS GVHTFPAVLQ SSGLYSLSSV VTVPSSSLGT QTYICNVNHK
PSNTKVDKKV EPKSCDKTHT CPPCPAPEAA GAPSVFLFPP KPKDYLTIER TPEVTCVVVD
VSHEDPEVKF NWYVDGVEVH NAKTKPREEQ YNSTYRVVSV LTVLHQDWLN GKEYKCKVSN
KALPAPIEKT ISKAKGQPRE PQVYTLPPSR EEMTKNQVSL TCLVKGFYPS DIAVEWESNG
 
QPENNYKTTP PVLDSDGSFF LYSKLTVDKS RWQQGNVFSC SVMHEALHNH YTQKSLSLSP
GK**
**SEQ ID NO:212** - IGG1-LALA-YTE IL-2-N88D/C125A-C7HC 4xG₄S; ASKG222.CX12.76.2 
APTSSSTKKT QLQLEHLLLD LQMILNGINN YKNPKLTRML TFKFYMPKKA TELKHLQCLE
EELKPLEEVL NLAQSKNFHL RPRDLISDIN VIVLELKGSE TTFMCEYADE TATIVEFLNR
WITFAQSIIS TLTGGGGSGG GGSAAGGGGS GGGGSQVQLV QSGAEVKKPG SSVKVSCKAS
GGTFTSYAIN WVRQAPGQGL EWMGRIIPII GRVDYAQKFQ GRVTITADES TSTAYMELSS
LRSEDTAVYY CARGFVGGFD VWGQGTLVTV SSASTKGPSV FPLAPSSKST SGGTAALGCL
VKDYFPEPVT VSWNSGALTS GVHTFPAVLQ SSGLYSLSSV VTVPSSSLGT QTYICNVNHK
PSNTKVDKKV EPKSCDKTHT CPPCPAPEAA GAPSVFLFPP KPKDYLTIER TPEVTCVVVD
VSHEDPEVKF NWYVDGVEVH NAKTKPREEQ YNSTYRVVSV LTVLHQDWLN GKEYKCKVSN
KALPAPIEKT ISKAKGQPRE PQVYTLPPSR EEMTKNQVSL TCLVKGFYPS DIAVEWESNG
QPENNYKTTP PVLDSDGSFF LYSKLTVDKS RWQQGNVFSC SVMHEALHNH YTQKSLSLSP
GK**
**SEQ ID NO:213** - IGG1-LALA-YTE IL-2-V69A/Q74P/C125A-C7HC 4xG₄S; ASKG222.CX12.76.3 
APTSSSTKKT QLQLEHLLLD LQMILNGINN YKNPKLTRML TFKFYMPKKA TELKHLQCLE
EELKPLEEAL NLAPSKNFHL RPRDLISNIN VIVLELKGSE TTFMCEYADE TATIVEFLNR
WITFAQSIIS TLTGGGGSGG GGSAAGGGGS GGGGSQVQLV QSGAEVKKPG SSVKVSCKAS
GGTFTSYAIN WVRQAPGQGL EWMGRIIPII GRVDYAQKFQ GRVTITADES TSTAYMELSS
LRSEDTAVYY CARGFVGGFD VWGQGTLVTV SSASTKGPSV FPLAPSSKST SGGTAALGCL
VKDYFPEPVT VSWNSGALTS GVHTFPAVLQ SSGLYSLSSV VTVPSSSLGT QTYICNVNHK
PSNTKVDKKV EPKSCDKTHT CPPCPAPEAA GAPSVFLFPP KPKDYLTIER TPEVTCVVVD
VSHEDPEVKF NWYVDGVEVH NAKTKPREEQ YNSTYRVVSV LTVLHQDWLN GKEYKCKVSN
KALPAPIEKT ISKAKGQPRE PQVYTLPPSR EEMTKNQVSL TCLVKGFYPS DIAVEWESNG
QPENNYKTTP PVLDSDGSFF LYSKLTVDKS RWQQGNVFSC SVMHEALHNH YTQKSLSLSP
GK**
**SEQ ID NO:214** - IGG1-LALA-YTE IL-2-V69A/Q74P/N88D/C125A-C7HC 4xG₄S; ASKG222.CX12.76.4 
APTSSSTKKT QLQLEHLLLD LQMILNGINN YKNPKLTRML TFKFYMPKKA TELKHLQCLE
EELKPLEEAL NLAPSKNFHL RPRDLISDIN VIVLELKGSE TTFMCEYADE TATIVEFLNR
WITFAQSIIS TLTGGGGSGG GGSAAGGGGS GGGGSQVQLV QSGAEVKKPG SSVKVSCKAS
GGTFTSYAIN WVRQAPGQGL EWMGRIIPII GRVDYAQKFQ GRVTITADES TSTAYMELSS
LRSEDTAVYY CARGFVGGFD VWGQGTLVTV SSASTKGPSV FPLAPSSKST SGGTAALGCL
VKDYFPEPVT VSWNSGALTS GVHTFPAVLQ SSGLYSLSSV VTVPSSSLGT QTYICNVNHK
PSNTKVDKKV EPKSCDKTHT CPPCPAPEAA GAPSVFLFPP KPKDYLTIER TPEVTCVVVD
VSHEDPEVKF NWYVDGVEVH NAKTKPREEQ YNSTYRVVSV LTVLHQDWLN GKEYKCKVSN
KALPAPIEKT ISKAKGQPRE PQVYTLPPSR EEMTKNQVSL TCLVKGFYPS DIAVEWESNG
QPENNYKTTP PVLDSDGSFF LYSKLTVDKS RWQQGNVFSC SVMHEALHNH YTQKSLSLSP
GK**
**SEQ ID NO:215** - C7HC; ASKG222.CX12.76.5 
QVQLVQSGAE VKKPGSSVKV SCKASGGTFT SYAINWVRQA PGQGLEWMGR IIPIIGRVDY
AQKFQGRVTI TADESTSTAY MELSSLRSED TAVYYCARGF VGGFDVWGQG TLVTVSSAST
KGPSVFPLAP SSKSTSGGTA ALGCLVKDYF PEPVTVSWNS GALTSGVHTF PAVLQSSGLY
SLSSVVTVPS SSLGTQTYIC NVNHKPSNTK VDKKVEPKSC DKTHTCPPCP APEAAGAPSV
FLFPPKPKDY LTIERTPEVT CVVVDVSHED PEVKFNWYVD GVEVHNAKTK PREEQYNSTY
RVVSVLTVLH QDWLNGKEYK CKVSNKALPA PIEKTISKAK GQPREPQVYT LPPSREEMTK
NQVSLTCLVK GFYPSDIAVE WESNGQPENN YKTTPPVLDS DGSFFLYSKL TVDKSRWQQG
NVFSCSVMHE ALHNHYTQKS LSLSPGK**
**SEQ ID NO:216** - ASKG812K-PD1-IL2v H435R, Y436F YTE. 3xG₄S;ASKG812K.CX13.7.5 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNRFTQKS LSLSLGAGGG GSGGGGSGGG GSAPASSSTK KTQLQLEHLL
LDLQMILNGI NNYKNPKLTS MLTAKFAMPK KATELKHLQC LEEALKPLEE VLNLAQSKNF
HLRPRDLISN INVIVLELKG SETTFMCEYA DETATIVEFL NRWITFSQSI ISTLT**
**SEQ ID NO:217** - ASKG812K-PD1-IL2v **H435R, Y436F** YTE. 2xG₄S; ASKG812K.CX13.7.6 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNRFTQKS LSLSLGAGGG GSGGGGSAPA SSSTKKTQLQ LEHLLLDLQM
ILNGINNYKN PKLTSMLTAK FAMPKKATEL KHLQCLEEAL KPLEEVLNLA QSKNFHLRPR
DLISNINVIV LELKGSETTF MCEYADETAT IVEFLNRWIT FSQSIISTLT **
**SEQ ID NO:218** - ASKG812K-PD1-IL2v H435R, Y436F YTE. 1xG₄S; ASKG812K.CX13.7.7 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNRFTQKS LSLSLGA
GGGGSAPASSSTKKTQLQLEHLLLDLQMILNGINNYKNPKLTSMLTAKFAMPKKATELKHLQCLEEA
LKPLEEVLNLAQSKNFHLRPRDLISNINVIVLELKGSETTFMCEYADETATIVEFLNRWITFSQSIIS
TLT**
**SEQ ID NO:219** - ASKG812K-PD1-IL2v H435R, Y436F YTE. 0xG₄S; ASKG812K.CX13.7.8 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNRFTQKS LSLSLGAAPA SSSTKKTQLQ LEHLLLDLQM ILNGINNYKN
PKLTSMLTAK FAMPKKATEL KHLQCLEEAL KPLEEVLNLA QSKNFHLRPR DLISNINVIV
LELKGSETTF MCEYADETAT IVEFLNRWIT FSQSIISTLT **
**SEQ ID NO:220** - ASKG812K-PD1-knob-IL2v YTE. 3xG₄S; ASKG812K.CX13.13.1 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPCQEEMTK
NQVSLWCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSGGG GSAPASSSTK KTQLQLEHLL
LDLQMILNGI NNYKNPKLTS MLTAKFAMPK KATELKHLQC LEEALKPLEE VLNLAQSKNF
HLRPRDLISN INVIVLELKG SETTFMCEYA DETATIVEFL NRWITFSQSI ISTLT**
**SEQ ID NO:221 -** ASKG812K-HC-IL2scFv-C7 YTE cleavable 4xG₄S; ASKG812K.CX13.55.1 (CDRs bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGA GGGGSGGGGS GPLGVRGGGG SGGGGSDIQM
TQSPSSLSAS VGDRVTITCR ASQDISNYLN WYQQKPGKAP KLLIYGASSL QSGVPSRFSG
SGSGTDFTLT ISSLQPEDFA TYYCQQTYTI PLTFGQGTKV EIKAGGGGSG GGGSGGGGSQ
VQLVQSGAEV KKPGSSVKVS CKASGGTFTS YAINWVRQAP GQGLEWMGRI IPIIGRVDYA
QKFQGRVTIT ADESTSTAYM ELSSLRSEDT AVYYCARGFV GGFDVWGQGT LVTVSS**
**SEQ ID NO:222** - ASKG812K-HC-IL2scFv-B12 YTE non-cleavable 4xG₄S; ASKG812K.CX13.55.2 (CDRs bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGA GGGGSGGGGS AAGGGGSGGG GSDIQMTQSP
SSLSASVGDR VTITCRASQD ISTYLNWYQQ KPGKAPKLLI YDASSLQSGV PSRFSGSGSG
TDFTLTISSL QPEDFATYYC QQSYSAPLTF GQGTKVEIKA GGGGSGGGGS GGGGSEVQLL
ESGGGLVQPG GSLRLSCAAS GFTFSTYAMS WVRQAPGKGL EWVSTISGSG VRTYYADSVK
GRFTISRDNS KNTLYLQMNS LRAEDTAVYY CARETYIYGP FDVWGQGTLV TVSS**
**SEQ ID NO:223** - ASKG812K-HC-scFv-C7 YTE cleavable 3xG₄S; ASKG812K.CX13.60.1 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
 
NVFSCSVMHE ALHNHYTQKS LSLSLGAGGGG SGGGGSGPLG VRGGGGSDIQ MTQSPSSLSA
SVGDRVTITC RASQDISNYL NWYQQKPGKA PKLLIYGASS LQSGVPSRFS GSGSGTDFTL
TISSLQPEDF ATYYCQQTYT IPLTFGQGTK VEIKAGGGGS GGGGSGGGGS QVQLVQSGAE
VKKPGSSVKV SCKASGGTFT SYAINWVRQA PGQGLEWMGR IIPIIGRVDY AQKFQGRVTI
TADESTSTAY MELSSLRSED TAVYYCARGF VGGFDVWGQG TLVTVSS**
**SEQ ID NO:224** - ASKG812K-HC-scFv-H5 YTE cleavable 3xG₄S; ASKG812K.CX13.60.2 (CDRs are bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSGPL GVRGGGGSEI VLTQSPGTLS
LSPGERATLS CRASQSVSSS YLAWYQQKPG QAPRLLIYGA SSRATGIPDR FSGSGSGTDF
TLTISRLEPE DFAVYYCQQY SRAPGTFGQG TKVEIKAGGG GSGGGGSGGG GSQVQLVQSG
AEVKKPGSSV KVSCKASGGT FSSYGIHWVR QAPGQGLEWM GGIIPILGTA NYAQKFQGRV
TITADESTST AYMELSSLRS EDTAVYYCAV PFFDVWGQGT LVTVSS**
**SEQ ID NO:225** - ASKG812K-HC-scFv#3 K1-69-G3 YTE cleavable 3xG₄S; ASKG812K.CX13.61.1 (CDRs are bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSGPL GVRGGGGSEI VLTQSPGTLS
LSPGERATLS CRASQSVSSS YLAWYQQKPG QAPRLMIYGA SSRATGIPDR FSGSGSGTDF
TLTISRLEPE DFAVYYCQQY ESSPPITFGQ GTKVEIKAGG GGSGGGGSGG GGSQVQLVQS
GAEVKKPGSS VKVSCKASGG TFSSYGIHWV RQAPGQGLEW MGGIIPSFGI TNYAQKFQGR
VTITADESTS TAYMELSSLR SEDTAVYYCA RYPYSFDHWG QGTLVTVSS**
SEQ ID NO:226 - ASKG812K-HC-scFv#6 K3-30-F6 YTE cleavable 3xG₄S; ASKG812K.CX13.61.2 (CDRs are bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSGPL GVRGGGGSDI QMTQSPSSLS
ASVGDRVTIT CRASQSISSY LNWYQQKPGK APKLLIYAAS SLQSGVPSRF SGSGSGTDFT
LTISSLQPED FATYYCQQSY SYPLTFGQGT KVEIKAGGGG SGGGGSGGGG SEVQLLESGG
GLVQPGGSLR LSCAASGFTF SDYAMSWVRQ APGKGLELVS VISSGGGATY YADSVKGRFT
ISRDNSKNTLYLQMNSLRAED TAVYYCARGP RTYDVWGQGT LVTVSS**
**SEQ ID NO:227** - ASKG812K-HC-scFv#7 K3-30-F7 YTE cleavable 3xG₄S; ASKG812K.CX13.61.3 (CDRs are bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSGPL GVRGGGGSEI VLTQSPGTLS
LSPGERATLS CRASQSVSSS YLAWYQQKPG QAPRLLIYDA SSRATGIPDR FSGSGSGTDF
TLTISRLEPE DFAVYYCQQY GSSFPWTFGQ GTKVEIKAGG GGSGGGGSGG GGSQVQLVES
GGGVVQPGRS LRLSCAASGF TFSTYGMHWV RQAPGKGLEW VAVISHEGNN KYYADSVKGR
FTISRDNSKN TLYLQMNSLR AEDTAVYYCA RDWFYGRARF DPWGQGTLVT VSS**
**SEQ ID NO:228** - ASKG812K-HC-scFv#9 K3-30-B12 YTE cleavable 3xG₄S; ASKG812K.CX13.61.4 (CDRs are bolded and underlined 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSGPL GVRGGGGSDI QMTQSPSSLS
ASVGDRVTIT CRASQDISTY LNWYQQKPGK APKLLIYDAS SLQSGVPSRF SGSGSGTDFT
LTISSLQPED FATYYCQQSY SAPLTFGQGT KVEIKAGGGG SGGGGSGGGG SEVQLLESGG
GLVQPGGSLR LSCAASGFTF STYAMSWVRQ APGKGLEWVS TISGSGVRTY YADSVKGRFT
ISRDNSKNTL YLQMNSLRAE DTAVYYCARE TYIYGPFDVW GQGTLVTVSS**
**SEQ ID NO:229** - ASKG812K-HC-scFv#10 K1-69-A8 YTE cleavable 3xG₄S; ASKG812K.CX13.61.5 (CDRs are bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGA GGGGSGGGGS GPLGVRGGGG SEIVLTQSPG
TLSLSPGERA TLSCRASQSV SSSYLAWYQQ KPGQAPRLLI YGASSRATGI PDRFSGSGSG
TDFTLTISRL EPEDFAVYYC QQYAGSPPLT FGQGTKVEIK AGGGGSGGGG SGGGGSQVQL
VQSGAEVKKP GSSVKVSCKA SGGTFSNYAI SWVRQAPGQG LEWMGWIIPV LGIAHYAQKF
QGRVTITADE STSTAYMELS SLRSEDTAVY YCARHILVYG VSPPYYAMDS WGQGTLVTVSS**
**SEQ ID NO:230** - ASKG812K-HC-scFv#13 K3-30-G9 YTE cleavable 3xG₄S; ASKG812K.CX13.61.6 (CDRs are bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGA GGGGSGGGGS GPLGVRGGGG SEIVLTQSPG
TLSLSPGERA TLSCRASQSV SSSYLAWYQQ KPGQAPRLLI YGASSRATGI PDRFSGSGSG
 
TDFTLTISRL EPEDFAVYYC QQYSYWPVTF GQGTKVEIKA GGGGSGGGGS GGGGSQVQLV
ESGGGVVQPG RSLRLSCAAS GFTFGSYAMH WVRQAPGKGL EWVAVISYSG NIIYYADSVK
GRFTISRDNS KNTLYLQMN SLRAEDTAVYY CARGGWEFDY WGQGTLVTVS S**
**SEQ ID NO:231** - ASKG812K-HC-scFv#14 K3-30-H9 YTE cleavable 3xG₄S; ASKG812K.CX13.61.7 (CDRs are bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGA GGGGSGGGGS GPLGVRGGGG SDIQMTQSPS
SLSASVGDRV TITCRASQSI SNYLNWYQQK PGKAPKLLIY AASSLQSGVP SRFSGSGSGT
DFTLTISSLQ PEDFATYYCQ QSYSFPPTFG QGTKVEIKAG GGGSGGGGSG GGGSEVQLLE
SGGGLVQPGG SLRLSCAAS GFTFSDYAMS WVRQAPGKGL EWVSVITASG HGTYYADSVK
GRFTISRDNS KNTLYLQMNS LRAEDTAVYY CARYRSSFID IWGQGTLVTV SS**
**SEQ ID NO:232** - ASKG812K-HC-scFv#18 L4-39-B9 YTE cleavable 3xG₄S; ASKG812K.CX13.61.8 (CDRs are bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGA GGGGSGGGGS GPLGVRGGGG SQSVLTQPPS
VSGAPGQRVT ISCTGSSSNI GAGYDVHWYQ QLPGTAPKLL IYGNNNRPSG VPDRFSGSKS
GTSASLAITG LQAEDEADYY CQSYDTSLSG WVFGGGTKLT VLGGGGSGGG GSGGGGSQLQ
LQESGPGLVK PSETLSLTCT VSGGSISRSP YYWGWIRQPP GKGLEWIGSI YYSGSTYYNP
SLKSRVTISV DTSKNQFSLK LSSVTAADTA VYYCARGSHT PTIGPLFYYA FDVWGQGTLV
TVSS**
**SEQ ID NO:233** - ASKG812K-HC-IL2scFv-K3-30-F7 YTE non-cleavable 4xG₄S; 
ASKG812K.CX13.96.1 (CDRs are bolded and underlined)
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGGGGS GGGGSAAGGG GSGGGGSEIV LTQSPGTLSL
SPGERATLSC RASQSVSSSY LAWYQQKPGQ APRLLIYDAS SRATGIPDRF SGSGSGTDFT
LTISRLEPED FAVYYCQQYG SSFPWTFGQG TKVEIKSGGG GSGGGGSGGG GSQVQLVESG
GGVVQPGRSL RLSCAASGFT FSTYGMHWVR QAPGKGLEWV AVISHEGNNK YYADSVKGRF
TISRDNSKNT LYLQMNSLRA EDTAVYYCAR DWFYGRARFD PWGQGTLVTV SS**
**SEQ ID NO:234** - ASKG812K-HC-IL2scFv-K1-69-G3 YTE non-cleavable 4xG₄S; ASKG812K.CX13.96.2 (CDRs are bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGGGGS GGGGSAAGGG GSGGGGSEIV LTQSPGTLSL
SPGERATLSC RASQSVSSSY LAWYQQKPGQ APRLMIYGAS SRATGIPDRF SGSGSGTDFT
LTISRLEPED FAVYYCQQYE SSPPITFGQG TKVEIKSGGG GSGGGGSGGG GSQVQLVQSG
AEVKKPGSSV KVSCKASGGT FSSYGIHWVR QAPGQGLEWM GGIIPSFGIT NYAQKFQGRV
TITADESTST AYMELSSLRS EDTAVYYCAR YPYSFDHWGQ GTLVTVSS **
**SEQ ID NO:235** - ASKG812K-HC-IL2scFv-L-69-B9 YTE non-cleavable 4xG₄S; ASKG812K.CX13.96.3 (CDRs are bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGGGGS GGGGSAAGGG GSGGGGSQSV LTQPPSVSGA
PGQRVTISCT GSSSNIGAGY DVHWYQQLPG TAPKLLIYGN NNRPSGVPDR FSGSKSGTSA
SLAITGLQAE DEADYYCQSY DTSLSGWVFG GGTKLTVLGG GGSGGGGSGG GGSQLQLQES
GPGLVKPSET LSLTCTVSGG SISRSPYYWG WIRQPPGKGL EWIGSIYYSG STYYNPSLKS
RVTISVDTSK NQFSLKLSSV TAADTAVYYC ARGSHTPTIG PLFYYAFDVW GQGTLVTVSS**
**SEQ ID NO:236** - ASKG812K.CX13.98.1 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSGGG GSAPASSSTK KTQLQLEHLL
LDLQMILNGI NNYKNPKLTS MLTAKFAMPK KATELKHLQCL EEALKPLEE VLNLAQSKNF
HLRPRDLISN INVIVLELKG SETTFMCEYAD ETATIVEFLN RWITFSQSI ISTLT**
**SEQ ID NO:237** - ASKG812K-PD1-IL2v L36I H435R, Y436F YTE. 3xG₄S; ASKG812K.CX13.98.2 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNRFTQKS LSLSLGAGGG GSGGGGSGGG GSAPASSSTK KTQLQLEHLL
LDLQMILNGI NNYKNPKITS MLTAKFAMPK KATELKHLQC LEEALKPLEE VLNLAQSKNF
HLRPRDLISN INKIVLELKG SETTFMCEYA DETATIVEFL NRWITFSQSI ISTLT**
**SEQ ID NO:238** - ASKG812K-PD1-IL2v L36I YTE. 3xG₄S; ASKG812K.CX13.98.3 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSGGG GSAPASSSTK KTQLQLEHLL
LDLQMILNGI NNYKNPKITS MLTAKFAMPK KATELKHLQC LEEALKPLEE VLNLAQSKNF
HLRPRDLISN INKIVLELKG SETTFMCEYA DETATIVEFL NRWITFSQSI ISTLT**
**SEQ ID NO:239** - ASKG812K-PD1-IL2v YTE. 2xG₄S; ASKG812K.CX13.119.1 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSAPA SSSTKKTQLQ LEHLLLDLQM
ILNGINNYKN PKLTSMLTAK FAMPKKATEL KHLQCLEEAL KPLEEVLNLA QSKNFHLRPR
DLISNINVIV LELKGSETTF MCEYADETAT IVEFLNRWIT FSQSIISTLT **
**SEQ ID NO:240** - ASKG812K-PD1-IL2v L36I YTE. 2xG₄S; ASKG812K.CX13.119.2 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSAPA SSSTKKTQLQ LEHLLLDLQM
ILNGINNYKN PKITSMLTAK FAMPKKATEL KHLQCLEEAL KPLEEVLNLA QSKNFHLRPR
DLISNINVIV LELKGSETTF MCEYADETAT IVEFLNRWIT FSQSIISTLT **
**SEQ ID NO:241** - ASKG812K-PD1-IL2v YTE. 0xG₄S; ASKG812K.CX13.124.1 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAAPA SSSTKKTQLQ LEHLLLDLQM ILNGINNYKN
PKLTSMLTAK FAMPKKATEL KHLQCLEEAL KPLEEVLNLA QSKNFHLRPR DLISNINVIV
LELKGSETTF MCEYADETAT IVEFLNRWIT FSQSIISTLT **
**SEQ ID NO:242** - ASKG812K-PD1-IL2v L36I YTE. 0xG₄S; ASKG812K.CX13.124.2 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
 
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAAPA SSSTKKTQLQ LEHLLLDLQM ILNGINNYKN
PKITSMLTAK FAMPKKATEL KHLQCLEEAL KPLEEVLNLA QSKNFHLRPR DLISNINVIV
LELKGSETTF MCEYADETAT IVEFLNRWIT FSQSIISTLT **
**SEQ ID NO:243** - ASKG812K-HC-IL2scFv-C7 YTE cleavable 4xG₄S; ASKG812K.CX13.129.1 (CDRs are bolded and underlined) 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSPLG LAGGGGSGGG GSDIQMTQSP
SSLSASVGDR VTITCRASQD ISNYLNWYQQ KPGKAPKLLI YGASSLQSGV PSRFSGSGSG
TDFTLTISSL QPEDFATYYC QQTYTIPLTF GQGTKVEIKA GGGGSGGGGS GGGGSQVQLV
QSGAEVKKPG SSVKVSCKAS GGTFTSYAIN WVRQAPGQGL EWMGRIIPII GRVDYAQKFQ
GRVTITADES TSTAYMELSS LRSEDTAVYY CARGFVGGFD VWGQGTLVTV SS**
**SEQ ID NO:244** - ASKG812K-HC-IL2scFv-C7 YTE non-cleavable 4xG₄S; ASKG812K.CX13.129.2 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSAAG GGGSGGGGSD IQMTQSPSSL
SASVGDRVTI TCRASQDISN YLNWYQQKPG KAPKLLIYGA SSLQSGVPSR FSGSGSGTDF
TLTISSLQPE DFATYYCQQT YTIPLTFGQG TKVEIKAGGG GSGGGGSGGG GSQVQLVQSG
AEVKKPGSSV KVSCKASGGT FTSYAINWVR QAPGQGLEWM GRIIPIIGRV DYAQKFQGRV
TITADESTST AYMELSSLRS EDTAVYYCAR GFVGGFDVWG QGTLVTVSS**
**SEQ ID NO:245** - ASKG812K-PD1-IL2wt T3A, C126A YTE. 2xG4S; ASKG812K.CX13.129.3 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSAPA SSSTKKTQLQ LEHLLLDLQM
ILNGINNYKN PKLTRMLTFK FYMPKKATEL KHLQCLEEEL KPLEEVLNLA QSKNFHLRPR
DLISNINVIV LELKGSETTF MCEYADETAT IVEFLNRWIT FAQSIISTLT **
**SEQ ID NO:246** - Ref molecule >PD1-IL2v_knob (S354C/T366W) (IL2, T3A, Y45A, L72G, C125A) Fc mutation to remove effector function: L234A, L235A, P329G; ASKG812.CX13.1.1 
EVQLLESGGG LVQPGGSLRL SCAASGFSFS SYTMSWVRQA PGKGLEWVAT ISGGGRDIYY
PDSVKGRFTI SRDNSKNTLY LQMNSLRAED TAVYYCVLLT GRVYFALDSW GQGTLVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKVEP KSCDKTHTCP PCPAPEAAGG
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LGAPIEKTIS KAKGQPREPQ VYTLPPCRDE
LTKNQVSLWC LVKGFYPSDI AVEWESNGQP ENNYKTTPPV LDSDGSFFLY SKLTVDKSRW
QQGNVFSCSV MHEALHNHYT QKSLSLSPGG GGGSGGGGSG GGGSAPASSS TKKTQLQLEH
LLLDLQMILN GINNYKNPKL TRMLTAKFAM PKKATELKHL QCLEEELKPL EEVLNGAQSK
NFHLRPRDLI SNINVIVLEL KGSETTFMCE YADETATIVE FLNRWITFAQ SIISTLT**
**SEQ ID NO:247** - Ref molecule >PD1-HC_hole2 (Y349C, T366S, L368A and Y410V plus H435R, Y436F; ASKG812.CX13.1.3 
EVQLLESGGG LVQPGGSLRL SCAASGFSFS SYTMSWVRQA PGKGLEWVAT ISGGGRDIYY
PDSVKGRFTI SRDNSKNTLY LQMNSLRAED TAVYYCVLLT GRVYFALDSW GQGTLVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKVEP KSCDKTHTCP PCPAPEAAGG
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LGAPIEKTIS KAKGQPREPQ VCTLPPSRDE
LTKNQVSLSC AVKGFYPSDI AVEWESNGQP ENNYKTTPPV LDSDGSFFLV SKLTVDKSRW
QQGNVFSCSV MHEALHNRFT QKSLSLSP**
**SEQ ID NO:248** - Ref molecule >PD1-LC; ASKG812.CX13.1.4 
DIVMTQSPDS LAVSLGERAT INCKASESVD TSDNSFIHWY QQKPGQSPKL LIYRSSTLES
GVPDRFSGSG SGTDFTLTIS SLQAEDVAVY YCQQNYDVPW TFGQGTKVEI KRTVAAPSVF
IFPPSDEQLK SGTASVVCLL NNFYPREAKV QWKVDNALQS GNSQESVTEQ DSKDSTYSLS
STLTLSKADY EKHKVYACEV THQGLSSPVT KSFNRGEC**
**SEQ ID NO:249** - Fc-IL-2 ScFv2, VL-VH: positive control molecules; ASKD215.CX7.108.2 
DKTHTCPPCP APEAAGGPSV FLFPPKPKDT LMISRTPEVT CVVVDVSHED PEVKFNWYVD
GVEVHNAKTK PREEQYNSTY RVVSVLTVLH QDWLNGKEYK CKVSNKALPA PIEKTISKAK
GQPREPQVCT LPPSRDELTK NQVSLSCAVK GFYPSDIAVE WESNGQPENN YKTTPPVLDS
DGSFFLVSKL TVDKSRWQQG NVFSCSVMHE ALHNHYTQKS LSLSPGAGGG GSGGGGSGPL
GVRGGGGSGG GGSSYVLTQP PSVSVAPGKT ARITCGGNNI RSKSVHWYQQ KPGQAPVVVI
YYDSDRPSGI PERISGSNSG NTATLTISRV EAGDEADYFC QVWDSSSDHH VFGGGTKLTV
LGGGGSGGGG SGGGGSQVQL VQSGAEVKKP GSSVKVSCKA SGGTFSSYAI SWVRQAPGQG
LEWMGGIIPI FGTANYAQKF QGRVTITADE STSTAYMELS SLRSEDTAVY YCARVDRYYN
WNYFLGSFDY WGQGTLVTVS S**
**SEQ ID NO:250** - AG111-#20-K1-69-H5 
EIVLTQSPGT LSLSPGERAT LSCRASQSVS SSYLAWYQQK PGQAPRLLIY GASSRATGIP
DRFSGSGSGT DFTLTISRLE PEDFAVYYCQ QYSRAPGTFG QGTKVEIKAG GGGSGGGGSG
GGGSQVQLVQ SGAEVKKPGS SVKVSCKASG GTFSSYGIHW VRQAPGQGLE WMGGIIPILG
TANYAQKFQG RVTITADEST STAYMELSSL RSEDTAVYYC AVPFFDVWGQ GTLVTVSS**
**SEQ** ID NO:251 - AG111-T3-K1-69-A8 
EIVLTQSPGT LSLSPGERAT LSCRASQSVS SSYLAWYQQK PGQAPRLLIY GASSRATGIP
DRFSGSGSGT DFTLTISRLE PEDFAVYYCQ QYAGSPPLTF GQGTKVEIKA GGGGSGGGGS
GGGGSQVQLV QSGAEVKKPG SSVKVSCKAS GGTFSNYAIS WVRQAPGQGL EWMGWIIPVL
GIAHYAQKFQ GRVTITADES TSTAYMELSS LRSEDTAVYY CARHILVYGV SPPYYAMDSW
GQGTLVTVSS
**SEQ ID NO:252** - AG111-T12-K3-30-H9 
DIQMTQSPSS LSASVGDRVT ITCRASQSIS NYLNWYQQKP GKAPKLLIYA ASSLQSGVPS
RFSGSGSGTD FTLTISSLQP EDFATYYCQQ SYSFPPTFGQ GTKVEIKAGG GGSGGGGSGG
GGSEVQLLES GGGLVQPGGS LRLSCAASGF TFSDYAMSWV RQAPGKGLEW VSVITASGHG
TYYADSVKGR FTISRDNSKN TLYLQMNSLR AEDTAVYYCA RYRSSFIDIW GQGTLVTVSS
**SEQ ID NO:253** - AG111-#4-K3-30-G9 
EIVLTQSPGT LSLSPGERAT LSCRASQSVS SSYLAWYQQK PGQAPRLLIY GASSRATGIP
DRFSGSGSGT DFTLTISRLE PEDFAVYYCQ QYSYWPVTFG QGTKVEIKAG GGGSGGGGSG
GGGSQVQLVE SGGGVVQPGR SLRLSCAASG FTFGSYAMHW VRQAPGKGLE WVAVISYSGN
IIYYADSVKG RFTISRDNSK NTLYLQMNSL RAEDTAVYYC ARGGWEFDYW GQGTLVTVSS
**SEQ ID NO:254** - AG111-#21-L4-39-B9 
QSVLTQPPSV SGAPGQRVTI SCTGSSSNIG AGYDVHWYQQ LPGTAPKLLI YGNNNRPSGV
PDRFSGSKSG TSASLAITGL QAEDEADYYC QSYDTSLSGW VFGGGTKLTV LGGGGSGGGG
SGGGGSQLQL QESGPGLVKP SETLSLTCTV SGGSISRSPY YWGWIRQPPG KGLEWIGSIY
YSGSTYYNPS LKSRVTISVD TSKNQFSLKL SSVTAADTAV YYCARGSHTP TIGPLFYYAF
DVWGQGTLVT VSS
**SEQ ID NO:255** - AG111-T9-K3-30-B12 
DIQMTQSPSS LSASVGDRVT ITCRASQDIS TYLNWYQQKP GKAPKLLIYD ASSLQSGVPS
RFSGSGSGTD FTLTISSLQP EDFATYYCQQ SYSAPLTFGQ GTKVEIKAGG GGSGGGGSGG
GGSEVQLLES GGGLVQPGGS LRLSCAASGF TFSTYAMSWV RQAPGKGLEW VSTISGSGVR
TYYADSVKGR FTISRDNSKN TLYLQMNSLR AEDTAVYYCA RETYIYGPFD VWGQGTLVTVSS
**SEQ ID NO:256** - AG111-#3-K3-30-F6 
DIQMTQSPSS LSASVGDRVT ITCRASQSIS SYLNWYQQKP GKAPKLLIYA ASSLQSGVPS
RFSGSGSGTD FTLTISSLQP EDFATYYCQQ SYSYPLTFGQ GTKVEIKAGG GGSGGGGSGG
GGSEVQLLES GGGLVQPGGS LRLSCAASGF TFSDYAMSWV RQAPGKGLEL VSVISSGGGA
TYYADSVKGR FTISRDNSKN TLYLQMNSLR AEDTAVYYCA RGPRTYDVWG QGTLVTVSS
**SEQ ID NO:257** - AG111-#22-K3-15-F7 
DIQMTQSPSS LSASVGDRVT ITCRASQDIR RYLNWYQQKP GKAPKLLIYA ASSLQSGVPS
RFSGSGSGTD FTLTISSLQP EDFATYYCQQ AYSIPLTFGQ GTKVEIKGGG GSGGGGSGGG
GSEVQLVESG GGLVKPGGSL RLSCAASGFT FSNAWMSWVR QAPGKGLEWV GRIKSKTAGG
TTDYAAPVKG RFTISRDDSK NTLYLQMNSL KTEDTAVYYC ARDWWYLDFD YWGQGTLVTVSS
**SEQ ID NO:258** - AG111-#93-K1-69-D2 
DIQMTQSPSS LSASVGDRVT ITCRASQSIS SYLNWYQQKP GKAPKLLIYA ASSLQSGVPS
RFSGSGSGTD FTLTISSLQP EDFATYYCQQ TYSVPPTFGQ GTKVEIKGGG GSGGGGSGGG
GSQVQLVQSG AEVKKPGSSV KVSCKASGGT FSSYGIHWVR QAPGQGLEWM GGIIPLVDIV
DYAQKFQGRV TITADESTST AYMELSSLRS EDTAVYYCAR YDGRYAFDWG QGTLVTVSS
**SEQ ID NO:259** - AG111-#44-K3-30-E12 
DIQMTQSPSS LSASVGDRVT ITCRASQGIT SYLNWYQQKP GKAPKLLIYD ASSLQSGVPS
RFSGSGSGTD FTLTISSLQP EDFATYYCQQ AYSSPYTFGQ GTKVEIKGGG GSGGGGSGGG
GSEVQLLESG GGLVQPGGSL RLSCAASGFT FSDYAMSWVR QAPGKGLEWV SAISGGGHGT
YYADSVKGRF TISRDNSKNT LYLQMNSLRA EDTAVYYCAR GGWELDYWGQ GTLVTVSS
**SEQ ID NO:260** - ASKG812K-PD1-hole- YTE RF mutation; ASKG812K.CX13.13.2 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
 
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVCT LPPSQEEMTK
NQVSLSCAVK GFYPSDIAVE WESNGQPENN YKTTPPVLDS DGSFFLVSRL TVDKSRWQEG
NVFSCSVMHE ALHNRFTQKS LSLSLGK**
**SEQ ID NO:261 -** IL-2wC125A-IGG4 Fc knob [R8.48.3: CX7.106.1 & CX7.70.1]; ASKD215.CX7.106.1 
APTSSSTKKT QLQLEHLLLD LQMILNGINN YKNPKLTRML TFKFYMPKKA TELKHLQCLE
EELKPLEEVL NLAQSKNFHL RPRDLISNIN VIVLELKGSE TTFMCEYADE TATIVEFLNR
WITFAQSIIS TLTGGGGSGG GGSGGGGSAE SKYGPPCPPC PAPEAAGGPS VFLFPPKPKD
TLMISRTPEV TCVVVDVSQE DPEVQFNWYV DGVEVHNAKT KPREEQFNST YRVVSVLTVL
HQDWLNGKEY KCKVSNKGLP SSIEKTISKA KGQPREPQVY TLPPCQEEMT KNQVSLWCLV
KGFYPSDIAV EWESNGQPEN NYKTTPPVLD SDGSFFLYSR LTVDKSRWQE GNVFSCSVMH
EALHNHYTQK SLSLSLGK**
**SEQ ID NO:262** - ASKD215.CX7.70.1 
AESKYGPPCP PCPAPEAAGG PSVFLFPPKP KDTLMISRTP EVTCVVVDVS QEDPEVQFNW
YVDGVEVHNA KTKPREEQFN STYRVVSVLT VLHQDWLNGK EYKCKVSNKG LPSSIEKTIS
KAKGQPREPQ VCTLPPSQEE MTKNQVSLSC AVKGFYPSDI AVEWESNGQP ENNYKTTPPV
LDSDGSFFLV SRLTVDKSRW QEGNVFSCSV MHEALHNHYT QKSLSLSLGK**
**SEQ ID NO:263** - LC of PD-1 antibody with a new signal peptide (CX11.114.1) 
EIVLTQSPAT LSLSPGERAT LSCRASKGVS TSGYSYLHWY QQKPGQAPRL LIYLASYLES
GVPARFSGSG SGTDFTLTIS SLEPEDFAVY YCQHSRDLPL TFGGGTKVEI KRTVAAPSVF
IFPPSDEQLK SGTASVVCLL NNFYPREAKV QWKVDNALQS GNSQESVTEQ DSKDSTYSLS
STLTLSKADY EKHKVYACEV THQGLSSPVT KSFNRGEC**
**SEQ ID NO:264** - PD1-HC-IL2V KNOB; ASKB1296.CX3.58.1 
QVQLVESGGG VVQPGRSLRL DCKASGITFS NSGMHWVRQA PGKGLEWVAV IWYDGSKRYY
ADSVKGRFTI SRDNSKNTLF LQMNSLRAED TAVYYCATND DYWGQGTLVT VSSASTKGPS
VFPLAPCSRS TSESTAALGC LVKDYFPEPV TVSWNSGALT SGVHTFPAVL QSSGLYSLSS
VVTVPSSSLG TKTYTCNVDH KPSNTKVDKR VESKYGPPCP PCPAPEFLGG PSVFLFPPKP
KDTLMISRTP EVTCVVVDVS QEDPEVQFNW YVDGVEVHNA KTKPREEQFN STYRVVSVLT
VLHQDWLNGK EYKCKVSNKG LPSSIEKTIS KAKGQPREPQ VYTLPPCQEE MTKNQVSLWC
LVKGFYPSDI AVEWESNGQP ENNYKTTPPV LDSDGSFFLY SRLTVDKSRW QEGNVFSCSV
MHEALHNHYT QKSLSLSLGK GGGGSGGGGS GGGGSAPASS STKKTQLQLE HLLLDLQMIL
NGINNYKNPK LTSMLTAKFA MPKKATELKH LQCLEEALKP LEEVLNLAQS KNFHLRPRDL
ISNINVIVLE LKGSETTFMC EYADETATIV EFLNRWITFS QSIISTLT**
**SEQ ID NO:265** - PD1HC-BETA HOLE LONG LINKER; ASKB1296.CX3.58.3 
QVQLVESGGG VVQPGRSLRL DCKASGITFS NSGMHWVRQA PGKGLEWVAV IWYDGSKRYY
ADSVKGRFTI SRDNSKNTLF LQMNSLRAED TAVYYCATND DYWGQGTLVT VSSASTKGPS
VFPLAPCSRS TSESTAALGC LVKDYFPEPV TVSWNSGALT SGVHTFPAVL QSSGLYSLSS
VVTVPSSSLG TKTYTCNVDH KPSNTKVDKR VESKYGPPCP PCPAPEFLGG PSVFLFPPKP
KDTLMISRTP EVTCVVVDVS QEDPEVQFNW YVDGVEVHNA KTKPREEQFN STYRVVSVLT
VLHQDWLNGK EYKCKVSNKG LPSSIEKTIS KAKGQPREPQ VCTLPPSQEE MTKNQVSLSC
AVKGFYPSDI AVEWESNGQP ENNYKTTPPV LDSDGSFFLV SRLTVDKSRW QEGNVFSCSV
MHEALHNHYT QKSLSLSLGK GGGGSGPLGV RGGGGSGGGG SAVNGTSQFT CFYNSRANIS
CVWSQDGALQ DTSCQVHAWP DRRRWNQTCE LLPVSQASWA CNLILGAPDS QKLTTVDIVT
LRVLCREGVR WRVMAIQDFK PFENLRLMAP ISLQVVHVET HRCNISWEIS QASHYFERHL
EFEARTLSPG HTWEEAPLLT LKQKQEWICL ETLTPDTQYE FQVRVKPLQG EFTTWSPWSQ
PLAFRTKPAA LGKDT**
**SEQ** ID NO:266 - PD1 HC HOLE; ASKB1296.CX3.58.4 
QVQLVESGGG VVQPGRSLRL DCKASGITFS NSGMHWVRQA PGKGLEWVAV IWYDGSKRYY
ADSVKGRFTI SRDNSKNTLF LQMNSLRAED TAVYYCATND DYWGQGTLVT VSSASTKGPS
VFPLAPCSRS TSESTAALGC LVKDYFPEPV TVSWNSGALT SGVHTFPAVL QSSGLYSLSS
VVTVPSSSLG TKTYTCNVDH KPSNTKVDKR VESKYGPPCP PCPAPEFLGG PSVFLFPPKP
KDTLMISRTP EVTCVVVDVS QEDPEVQFNW YVDGVEVHNA KTKPREEQFN STYRVVSVLT
VLHQDWLNGK EYKCKVSNKG LPSSIEKTIS KAKGQPREPQ VCTLPPSQEE MTKNQVSLSC
AVKGFYPSDI AVEWESNGQP ENNYKTTPPV LDSDGSFFLV SRLTVDKSRW QEGNVFSCSV
MHEALHNHYT QKSLSLSLGK **
**SEQ ID NO:267 -** ASKG812K-β-ECD CX13.7.4 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPCSRSTSE STAALGCLVK DYFPEPVTVS WNSGALTSGV HTFPAVLQSS
GLYSLSSVVT VPSSSLGTKT YTCNVDHKPS NTKVDKRVES KYGPPCPPCP APEFLGGPSV
FLFPPKPKDT LYITREPEVT CVVVDVSQED PEVQFNWYVD GVEVHNAKTK PREEQFNSTY
RVVSVLTVLH QDWLNGKEYK CKVSNKGLPS SIEKTISKAK GQPREPQVYT LPPSQEEMTK
NQVSLTCLVK GFYPSDIAVE WESNGQPENN YKTTPPVLDS DGSFFLYSRL TVDKSRWQEG
NVFSCSVMHE ALHNHYTQKS LSLSLGAGGG GSGGGGSAAG GGGSGGGGSA VNGTSQFTCF
YNSRANISCV WSQDGALQDT SCQVHAWPDR RRWNQTCELL PVSQASWACN LILGAPDSQK
LTTVDIVTLR VLCREGVRWR VMAIQDFKPF ENLRLMAPIS LQVVHVETHR CNISWEISQA
SHYFERHLEF EARTLSPGHT WEEAPLLTLK SKEEWICLET LTPDTQYEFQ VRVKPLQGEF
TTWSPWSQPL AFRTKPAALG KDT**
**SEQ ID NO:268**
   GGGGSGGGGS GPLGVRGGGG SGGGGS
**SEQ ID NO:269**
   GGGGSGGGGS GPLGVRGGGG S
**SEQ ID NO:270** - mPD1-mIgG2-L234A/L2356A/P329G CX11_158_3 
EVQLVESG GGLVQPGGSL KLSCAASGFT FSNSGLAWVR QAPEKGLEWV ATITYNGTST
YYRDSVKGRF TISRDNAKNT LYLQMSSLRS EDTATYYCAR WVPGSGNFDY WGQGTLVTVS
SAKTTAPSVY PLAPVCGDTT GSSVTLGCLV KGYFPEPVTL TWNSGSLSSG VHTFPAVLQS
DLYTLSSSVT VTSSTWPSQS ITCNVAHPAS STKVDKKIEP RGPTIKPCPP CKCPAPNAAG
GPSVFIFPPK IKDVLMISLS PIVTCVVVDV SEDDPDVQIS WFVNNVEVHT AQTQTHREDY
NSTLRVVSAL PIQHQDWMSG KEFKCKVNNK DLGAPIERTI SKPKGSVRAP QVYVLPPPEE
EMTKKQVTLT CMVTDFMPED IYVEWTNNGK TELNYKNTEP VLDSDGSYFM YSKLRVEKKN
WVERNSYSCS VVHEGLHNHH TTKSFSRTPG K**
**SEQ ID NO:271** - mDX400-LC CX11_154_1 
DIVLTQSP ASLAVSLGQR ATISCRASQS VTISRYTLMH WYQQKPGQPP KLLIYRASNL
ASGIPARFSG SGSGTDFTLN IHPVEEDDAA TYYCQQSRES PWTFGGGTKL EIKRADAAPT
VSIFPPSSEQ LTSGGASVVC FLNNFYPKDI NVKWKIDGSE RQNGVLNSWT DQDSKDSTYS
MSSTLTLTKD EYERHNSYTC EATHKTSTSP IVKSFNRNEC**
**SEQ ID NO:272** - mPD1-mIgG2-L234A/L2356A/P329G, K409E/K439D IL-2vR ASKG812_CX15_154_2 
EVQLVESG GGLVQPGGSL KLSCAASGFT FSNSGLAWVR QAPEKGLEWV ATITYNGTST
YYRDSVKGRF TISRDNAKNT LYLQMSSLRS EDTATYYCAR WVPGSGNFDY WGQGTLVTVS
 
SAKTTAPSVY PLAPVCGDTT GSSVTLGCLV KGYFPEPVTL TWNSGSLSSG VHTFPAVLQS
DLYTLSSSVT VTSSTWPSQS ITCNVAHPAS STKVDKKIEP RGPTIKPCPP CKCPAPNAAG
GPSVFIFPPK IKDVLMISLS PIVTCVVVDV SEDDPDVQIS WFVNNVEVHT AQTQTHREDY
NSTLRVVSAL PIQHQDWMSG KEFKCKVNNK DLGAPIERTI SKPKGSVRAP QVYVLPPPEE
EMTKKQVTLT CMVTDFMPED IYVEWTNNGK TELNYKNTEP VLDSDGSYFM YSELRVEKKN
WVERNSYSCS VVHEGLHNHH TTDSFSRTPG GGGGSGGGGS GGGGSAPASS STKKTQLQLE
HLLLDLQMIL NGINNYKNPK LTRMLTAKFA MPKKATELKH LQCLEEELKP LEEVLNGAQS
KNFHLRPRDL ISNINVIVLE LKGSETTFMC EYADETATIV EFLNRWITFA QSIISTLT**
**SEQ ID NO:273** - mPD1-mIgG2-L234A/L2356A/P329G, E356K/D399K cx11_158_2 
EVQLVESG GGLVQPGGSL KLSCAASGFT FSNSGLAWVR QAPEKGLEWV ATITYNGTST
YYRDSVKGRF TISRDNAKNT LYLQMSSLRS EDTATYYCAR WVPGSGNFDY WGQGTLVTVS
SAKTTAPSVY PLAPVCGDTT GSSVTLGCLV KGYFPEPVTL TWNSGSLSSG VHTFPAVLQS
DLYTLSSSVT VTSSTWPSQS ITCNVAHPAS STKVDKKIEP RGPTIKPCPP CKCPAPNAAG
GPSVFIFPPK IKDVLMISLS PIVTCVVVDV SEDDPDVQIS WFVNNVEVHT AQTQTHREDY
NSTLRVVSAL PIQHQDWMSG KEFKCKVNNK DLGAPIERTI SKPKGSVRAP QVYVLPPPEK
EMTKKQVTLT CMVTDFMPED IYVEWTNNGK TELNYKNTEP VLKSDGSYFM YSKLRVEKKN
WVERNSYSCS VVHEGLHNHH TTKSFSRTP**
**SEQ ID NO:274** - mPD1-mIgG2-L234A/L2356A/P329G, K409E/K439D-F7- IL-2v-mut4 ASKG812 _CX21_132_2 
EVQLVESG GGLVQPGGSL KLSCAASGFT FSNSGLAWVR QAPEKGLEWV ATITYNGTST
YYRDSVKGRF TISRDNAKNT LYLQMSSLRS EDTATYYCAR WVPGSGNFDY WGQGTLVTVS
SAKTTAPSVY PLAPVCGDTT GSSVTLGCLV KGYFPEPVTL TWNSGSLSSG VHTFPAVLQS
DLYTLSSSVT VTSSTWPSQS ITCNVAHPAS STKVDKKIEP RGPTIKPCPP CKCPAPNAAG
GPSVFIFPPK IKDVLMISLS PIVTCVVVDV SEDDPDVQIS WFVNNVEVHT AQTQTHREDY
NSTLRVVSAL PIQHQDWMSG KEFKCKVNNK DLGAPIERTI SKPKGSVRAP QVYVLPPPEE
EMTKKQVTLT CMVTDFMPED IYVEWTNNGK TELNYKNTEP VLDSDGSYFM YSELRVEKKN
WVERNSYSCS VVHEGLHNHH TTDSFSRTPG GGGSGGGGSE IVLTQSPGTL SLSPGERATL
SCRASQSVSS SYLAWYQQKP GQAPRLLIYD ASSRATGIPD RFSGSGSGTD FTLTISRLEP
EDFAVYYCQQ YGSSFPWTFG QGTKVEIKSG GGGSGGGGSA AGGGGSGGGG SQVQLVESGG
GVVQPGRSLR LSCAASGFTF STYGMHWVRQ APGKGLEWVA VISHEGNNKY YADSVKGRFT
ISRDNSKNTL YLQMNSLRAE DTAVYYCARD WFYGRARFDP WGQGTLVTVS SGGGGSGGGG
SAAGGGGSGG GGSAPASSST KKTQLQLEHL LLDLQMILNG INNYKNPKIT SMLTFKFYMP
KKATELKHLQ CLEEELKPLE EVLNLAQSKN FHLRPRDLIS NINVIVLELK GSETTFMCEY
ADETATIVEF LNRWITFAQS IISTLT**
**SEQ ID NO:275** - ASKG812K_CX21_134_4 
QVQ LVQSGVEVKK PGASVKVSCK ASGYTFTNYY MYWVRQAPGQ GLEWMGGINP
SNGGTNFNEK FKNRVTLTTD SSTTTAYMEL KSLQFDDTAV YYCARRDYRF DMGFDYWGQG
TTVTVSSAST KGPSVFPLAP SSKSTSGGTA ALGCLVKDYF PEPVTVSWNS GALTSGVHTF
PAVLQSSGLY SLSSVVTVPS SSLGTQTYIC NVNHKPSNTK VDKKVEPKSC DKTHTCPPCP
APEAAGAPSV FLFPPKPKDT LMISRTPEVT CVVVDVSHED PEVKFNWYVD GVEVHNAKTK
PREEQYNSTY RVVSVLTVLH QDWLNGKEYK CKVSNKALPA PIEKTISKAK GQPREPQVYT
LPPCREEMTK NQVSLWCLVK GFYPSDIAVE WESNGQPENN YKTTPPVLDS DGSFFLYSKL
TVDKSRWQQG NVFSCSVMHE ALHNHYTQKS LSLSPGGGGS GGGGSEIVLT QSPGTLSLSP
GERATLSCRA SQSVSSSYLA WYQQKPGQAP RLLIYDASSR ATGIPDRFSG SGSGTDFTLT
 
ISRLEPEDFA VYYCQQYGSS FPWTFGQGTK VEIKSGGGGS GGGGSAAGGG GSGGGGSQVQ
LVESGGGVVQ PGRSLRLSCA ASGFTFSTYG MHWVRQAPGK GLEWVAVISH EGNNKYYADS
VKGRFTISRD NSKNTLYLQM NSLRAEDTAV YYCARDWFYG RARFDPWGQG TLVTVSSGGG
GSGGGGSAAG GGGSGGGGSA PASSSTKKTQ LQLEHLLLDL QMILNGINNY KNPKITSMLT
FKFYMPKKAT ELKHLQCLEE ELKPLEEVLN LAQSKNFHLR PRDLISNINV IVLELKGSET
TFMCEYADET ATIVEFLNRW ITFAQSIIST LT**
**SEQ ID NO:276** - ASKG812K_CX13_157_4 
EIVLTQSPAT LSLSPGERAT LSCRASKGVS TSGYSYLHWY QQKPGQAPRL LIYLASYLES
GVPARFSGSG SGTDFTLTIS SLEPEDFAVY YCQHSRDLPL TFGGGTKVEI KRTVAAPSVF
IFPPSDEQLK SGTASVVCLL NNFYPREAKV QWKVDNALQS GNSQESVTEQ DSKDSTYSLS
STLTLSKADY EKHKVYACEV THQGLSSPVT KSFNRGEC**
**SEQ ID NO:277** - 812K-HC L234A,L235A,G237A, hole ASKG812K_CX13_152_5 
QVQ LVQSGVEVKK PGASVKVSCK ASGYTFTNYY MYWVRQAPGQ GLEWMGGINP
SNGGTNFNEK FKNRVTLTTD SSTTTAYMEL KSLQFDDTAV YYCARRDYRF DMGFDYWGQG
TTVTVSSAST KGPSVFPLAP SSKSTSGGTA ALGCLVKDYF PEPVTVSWNS GALTSGVHTF
PAVLQSSGLY SLSSVVTVPS SSLGTQTYIC NVNHKPSNTK VDKKVEPKSC DKTHTCPPCP
APEAAGAPSV FLFPPKPKDT LMISRTPEVT CVVVDVSHED PEVKFNWYVD GVEVHNAKTK
PREEQYNSTY RVVSVLTVLH QDWLNGKEYK CKVSNKALPA PIEKTISKAK GQPREPQVCT
LPPSREEMTK NQVSLSCAVK GFYPSDIAVE WESNGQPENN YKTTPPVLDS DGSFFLVSKL
TVDKSRWQQG NVFSCSVMHE ALHNHYTQKS LSLSPGK**
**SEQ ID NO:278** - ASKG812N CX22_31_1 
QVQ LVESGGGVVQ PGRSLRLDCK ASGITFSNSG MHWVRQAPGK GLEWVAVIWY
DGSKRYYADS VKGRFTISRD NSKNTLFLQM NSLRAEDTAV YYCATNDDYW GQGTLVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKVEP KSCDKTHTCP PCPAPEAAGA
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LPAPIEKTIS KAKGQPREPQ VYTLPPCREE
MTKNQVSLWC LVKGFYPSDI AVEWESNGQP ENNYKTTPPV LDSDGSFFLY SKLTVDKSRW
QQGNVFSCSV MHEALHNHYT QKSLSLSPGG GGSGGGGSEI VLTQSPGTLS LSPGERATLS
CRASQSVSSS YLAWYQQKPG QAPRLLIYDA SSRATGIPDR FSGSGSGTDF TLTISRLEPE
DFAVYYCQQY GSSFPWTFGQ GTKVEIKSGG GGSGGGGSAA GGGGSGGGGS QVQLVESGGG
VVQPGRSLRL SCAASGFTFS TYGMHWVRQA PGKGLEWVAV ISHEGNNKYY ADSVKGRFTI
SRDNSKNTLY LQMNSLRAED TAVYYCARDW FYGRARFDPW GQGTLVTVSS GGGGSGGGGS
AAGGGGSGGG GSAPASSSTK KTQLQLEHLL LDLQMILNGI NNYKNPKITS MLTFKFYMPK
KATELKHLQC LEEELKPLEE VLNLAQSKNF HLRPRDLISN INVIVLELKG SETTFMCEYA
DETATIVEFL NRWITFAQSI ISTLT**
**SEQ ID NO:279 -** 812N-HC L234A,L235A,G237A, hole Cx22 31 2 
QVQ LVESGGGVVQ PGRSLRLDCK ASGITFSNSG MHWVRQAPGK GLEWVAVIWY
DGSKRYYADS VKGRFTISRD NSKNTLFLQM NSLRAEDTAV YYCATNDDYW GQGTLVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKVEP KSCDKTHTCP PCPAPEAAGA
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LPAPIEKTIS KAKGQPREPQ VCTLPPSREE
 
MTKNQVSLSC AVKGFYPSDI AVEWESNGQP ENNYKTTPPV LDSDGSFFLV SKLTVDKSRW
QQGNVFSCSV MHEALHNHYT QKSLSLSPGK **
**SEQ** ID **NO:280** - IL-2vL-mPD1-Fab -NX2-Fc-mIgG2 L234A/L2356A/P329G K409E/K439D ASKG678_CX18_30_1 
APASSSTK KTQLQLEHLL LDLQMILNGI NNYKNPKITS MLTAKFAMPK KATELKHLQC
LEEALKPLEE VLNLAQSKNF HLRPRDLISN INVIVLELKG SETTFMCEYA DETATIVEFL
NRWITFAQSI ISTLTGGGGS GGGGSEVQLV ESGGGLVQPG GSLKLSCAAS GFTFSNSGLA
WVRQAPEKGL EWVATITYNG TSTYYRDSVK GRFTISRDNA KNTLYLQMSS LRSEDTATYY
CARWVPGSGN FDYWGQGTLV TVSSAKTTAP SVYPLAPVCG DTTGSSVTLG CLVKGYFPEP
VTLTWNSGSL SSGVHTFPAV LQSDLYTLSS SVTVTSSTWP SQSITCNVAH PASSTKVDKK
GGGGSGPLGV RGSLSGRSDN HGGGGSEPRG PTIKPCPPCK CPAPNAAGGP SVFIFPPKIK
DVLMISLSPI VTCVVVDVSE DDPDVQISWF VNNVEVHTAQ TQTHREDYNS TLRVVSALPI
QHQDWMSGKE FKCKVNNKDL GAPIERTISK PKGSVRAPQV YVLPPPEEEM TKKQVTLTCM
VTDFMPEDIY VEWTNNGKTE LNYKNTEPVL DSDGSYFMYS ELRVEKKNWV ERNSYSCSVV
HEGLHNHHTT DSFSRTPGK**
**SEQ ID NO:281 -** C7- non clv-mIgG2-Fc-L234A/L2356A/P329G, E356K/D399K ASKG812 _CX21_32_1 
QVQLVQSG AEVKKPGSSV KVSCKASGGT FTSYAINWVR QAPGQGLEWM GRIIPIIGRV
DYAQKFQGRV TITADESTST AYMELSSLRS EDTAVYYCAR GFVGGFDVWG QGTLVTVSSG
GGGSGGGGSA AGGGGSGGGG SDIQMTQSPS SLSASVGDRV TITCRASQDI SNYLNWYQQK
PGKAPKLLIY GASSLQSGVP SRFSGSGSGT DFTLTISSLQ PEDFATYYCQ QTYTIPLTFG
QGTKVEIKSG GGGSGGGGSA AGGGGSGGGG SEPRGPTIKP CPPCKCPAPN AAGGPSVFIF
PPKIKDVLMI SLSPIVTCVV VDVSEDDPDV QISWFVNNVE VHTAQTQTHR EDYNSTLRVV
SALPIQHQDW MSGKEFKCKV NNKDLGAPIE RTISKPKGSV RAPQVYVLPP PEKEMTKKQV
TLTCMVTDFM PEDIYVEWTN NGKTELNYKN TEPVLKSDGS YFMYSKLRVE KKNWVERNSY
SCSVVHEGLH NHHTTKSFSR TPGK**
**SEQ ID NO: 282** - **Cleavable Linker Sequence**
   GGGGSGPLGV RGGGGSGGGG S
**SEQ ID NO:283** - 812NP-A IL-2vL-Nivo-Fab-NX2-Fc-Igg1-knob-YTE 
APA SSSTKKTQLQ LEHLLLDLQM ILNGINNYKN PKITSMLTAK FAMPKKATEL
KHLQCLEEAL KPLEEVLNLA QSKNFHLRPR DLISNINVIV LELKGSETTF MCEYADETAT
IVEFLNRWIT FAQSIISTLT GGGGSGGGGS QVQLVESGGG VVQPGRSLRL DCKASGITFS
NSGMHWVRQA PGKGLEWVAV IWYDGSKRYY ADSVKGRFTI SRDNSKNTLF LQMNSLRAED
TAVYYCATND DYWGQGTLVT VSSASTKGPS VFPLAPSSKS TSGGTAALGC LVKDYFPEPV
TVSWNSGALT SGVHTFPAVL QSSGLYSLSS VVTVPSSSLG TQTYICNVNH KPSNTKVDKK
GGGGSGPLGV RGSLSGRSDN HGGGGSDKTH TCPPCPAPEA AGAPSVFLFP PKPKDTLYIT
REPEVTCVVV DVSHEDPEVK FNWYVDGVEV HNAKTKPREE QYNSTYRVVS VLTVLHQDWL
NGKEYKCKVS NKALPAPIEK TISKAKGQPR EPQVYTLPPC REEMTKNQVS LWCLVKGFYP
SDIAVEWESN GQPENNYKTT PPVLDSDGSF FLYSKLTVDK SRWQQGNVFS CSVMHEALHN
HYTQKSLSLS PGK**
**SEQ ID NO:284** - C7- non clv-hIgG1 LALA G237A, YTE, hole ASKG812 _CX21_27_2 
QVQLVQSG AEVKKPGSSV KVSCKASGGT FTSYAINWVR QAPGQGLEWM RIIPIIGRVD
YAQKFQGRVT ITADESTSTA YMELSSLRSE DTAVYYCARG FVGGFDVWGQ GTLVTVSSGG
GGSGGGGSAA GGGGSGGGGS DIQMTQSPSS LSASVGDRVT ITCRASQDIS NYLNWYQQKP
GKAPKLLIYG ASSLQSGVPS RFSGSGSGTD FTLTISSLQP EDFATYYCQQ TYTIPLTFGQ
GTKVEIKSGG GGSGGGGSAA GGGGSGGGGS DKTHTCPPCP APEAAGAPSV FLFPPKPKDT
LYITREPEVT CVVVDVSHED PEVKFNWYVD GVEVHNAKTK PREEQYNSTY RVVSVLTVLH
QDWLNGKEYK CKVSNKALPA PIEKTISKAK GQPREPQVCT LPPSREEMTK NQVSLSCAVK
GFYPSDIAVE WESNGQPENN YKTTPPVLDS DGSFFLVSKL TVDKSRWQQG NVFSCSVMHE
ALHNHYTQKS LSLSPGK**
**SEQ ID NO: 285** - CX21_87_4 
EVQLVESGGG LVQPGGSLKL SCAASGFTFS NSGLAWVRQA PEKGLEWVAT ITYNGTSTYY
RDSVKGRFTI SRDNAKNTLY LQMSSLRSED TATYYCARWV PGSGNFDYWG QGTLVTVSSA
KTTAPSVYPL APVCGDTTGS SVTLGCLVKG YFPEPVTLTW NSGSLSSGVH TFPAVLQSDL
YTLSSSVTVT SSTWPSQSIT CNVAHPASST KVDKKIEPRG PTIKPCPPCK CPAPNAAGGP
SVFIFPPKIK DVLMISLSPI VTCVVVDVSE DDPDVQISWF VNNVEVHTAQ TQTHREDYNS
TLRVVSALPI QHQDWMSGKE FKCKVNNKDL GAPIERTISK PKGSVRAPQV YVLPPPEEEM
TKKQVTLTCM VTDFMPEDIY VEWTNNGKTE LNYKNTEPVL DSDGSYFMYS KLRVEKKNWV
ERNSYSCSVV HEGLHNHHTT KSFSRTPGGG GGSGGGGSEI VLTQSPGTLS LSPGERATLS
CRASQSVSSS YLAWYQQKPG QAPRLLIYDA SSRATGIPDR FSGSGSGTDF TLTISRLEPE
DFAVYYCQQY GSSFPWTFGQ GTKVEIKSGG GGSGGGGSAA GGGGSGGGGS QVQLVESGGG
VVQPGRSLRL SCAASGFTFS TYGMHWVRQA PGKGLEWVAV ISHEGNNKYY ADSVKGRFTI
SRDNSKNTLY LQMNSLRAED TAVYYCARDW FYGRARFDPW GQGTLVTVSS GGGGSGGGGS
AAGGGGSGGG GSAPASSSTK KTQLQLEHLL LDLQMILNGI NNYKNPKITS MLTFKFYMPK
KATELKHLQC LEEELKPLEE VLNLAQSKNF HLRPRDLISN INVIVLELKG SETTFMCEYA
DETATIVEFL NRWITFAQSI ISTLT**
**SEQ ID NO: 286** - 812KN-mut4 
QVQLVQSGVE VKKPGASVKV SCKASGYTFT NYYMYWVRQA PGQGLEWMGG INPSNGGTNF
NEKFKNRVTL TTDSSTTTAY MELKSLQFDD TAVYYCARRD YRFDMGFDYW GQGTTVTVSS
ASTKGPSVFP LAPSSKSTSG GTAALGCLVK DYFPEPVTVS WNSGALTSGV HTFPAVLQSS
GLYSLSSVVT VPSSSLGTQT YICNVNHKPS NTKVDKKVEP KSCDKTHTCP PCPAPEAAGA
PSVFLFPPKP KDTLMISRTP EVTCVVVDVS HEDPEVKFNW YVDGVEVHNA KTKPREEQYN
STYRVVSVLT VLHQDWLNGK EYKCKVSNKA LPAPIEKTIS KAKGQPREPQ VYTLPPSREE
MTKNQVSLTC LVKGFYPSDI AVEWESNGQP ENNYKTTPPV LDSDGSFFLY SKLTVDKSRW
QQGNVFSCSV MHEALHNHYT QKSLSLSPGG GGSGGGGSEI VLTQSPGTLS LSPGERATLS
CRASQSVSSS YLAWYQQKPG QAPRLLIYDA SSRATGIPDR FSGSGSGTDF TLTISRLEPE
DFAVYYCQQY GSSFPWTFGQ GTKVEIKSGG GGSGGGGSAA GGGGSGGGGS QVQLVESGGG
VVQPGRSLRL SCAASGFTFS TYGMHWVRQA PGKGLEWVAV ISHEGNNKYY ADSVKGRFTI
SRDNSKNTLY LQMNSLRAED TAVYYCARDW FYGRARFDPW GQGTLVTVSS GGGGSGGGGS
AAGGGGSGGG GSAPASSSTK KTQLQLEHLL LDLQMILNGI NNYKNPKITS MLTFKFYMPK
KATELKHLQC LEEELKPLEE VLNLAQSKNF HLRPRDLISN INVIVLELKG SETTFMCEYA
DETATIVEFL NRWITFAQSI ISTLT**
**SEQ ID NO: 287**
   RWITFSQSI
**SEQ ID NO: 288**
   NPKLTSMLT
**SEQ ID NO: 289**
   LISNINVIV
**SEQ ID NO: 290**
   HLRPRDLIS
**SEQ ID NO: 291**
   LNLAQSKNF
**SEQ ID NO: 292**
   LAQSKNFHL
**SEQ ID NO: 293**
   LLLDLQMIL
**SEQ ID NO: 294**
   QLQLEHLLL
**SEQ ID NO: 295**
   LEEVLNLAQ
**SEQ ID NO: 296**
   FCQSIISTL
**SEQ ID NO: 297**
   WITFCQSII
**SEQ ID NO: 298**
   LTSMLTAKF
**SEQ ID NO: 299**
   FSQSIISTL

The present invention further comprises the aspects defined in the following clauses:
1. A mutant human IL-2 polypeptide comprising an amino acid sequence that is at least 90% identical to SEQ ID NO:1 and an L36I mutation relative to SEQ ID NO: 1.
2. The mutant human IL-2 polypeptide of clause 1, further comprising one or more additional mutations that reduce the polypeptide's binding affinity for CD25.
3. The mutant human IL-2 polypeptide of clause 1, further comprising a C125A mutation relative to SEQ ID NO: 1.
4. The mutant human IL-2 polypeptide of any one of clauses 1-3, further comprising one or more additional mutations at positions selected from
   T3, optionally wherein the mutation is N3A;
   D20, optionally wherein the mutation is D20H, D20K, D20L, D20M, D20N, D20Q, D20R, D20S, D20V, or D20Y;
   R38, optionally wherein the mutation is R38A, R38K, or R38S;
   F42, optionally wherein the mutation is F42A, F42G, F42I, F42S, F42T, F42Q, F42E, F42N, F42D, F42R, or F42K;
   Y45, optionally wherein the mutation is Y45A, Y45G, Y45I, Y45S, Y45T, Y45Q, Y45E, Y45N, Y45D, Y45R, or Y45K;
   E62, optionally wherein the mutation is E62L, E62A, or E62I;
   E68, optionally wherein the mutation is E68V;
   L72, optionally wherein the mutation is L72G;
   A73, optionally wherein the mutation is A73T;
   N88, optionally wherein the mutation is N88A, N88E, N88F, N88H, N88K, N88T, N88L. N88M, N88S, N88V, N88W, or N88Y;
   N90, optionally wherein the mutation is N90T;
   V91, optionally wherein the mutation is V91K, V91A, V91H, or V91R;
   I92;
   and Q126, optionally wherein the mutation is Q126A, Q126D, Q126F, Q126G, Q126H, Q126I, Q126K, Q126L, Q126P, Q126S, Q126T, Q126W, or Q126Y (numbering according to SEQ ID NO: 1).
5. The mutant human IL-2 polypeptide of any one of clauses 1-4, wherein the mutant IL-2 polypeptide comprises an amino acid sequence selected from SEQ ID NOs:3 and 5-33, or an amino acid sequence at least 95% identical thereto.
6. A mutant human IL-2 polypeptide comprising an amino acid sequence that is at least 95% identical to SEQ ID NO:2 or 4.
7. An anti-IL-2 antibody or an antigen-binding fragment thereof, comprising heavy chain CDR1-3 of SEQ ID NOs:170-172, respectively, and light chain CDR1-3 of SEQ ID NOs:173- 175, respectively.
8. An anti-IL-2 antibody or an antigen-binding fragment thereof, comprising,
   a light chain variable domain (V_{L}) comprising SEQ ID NO:190 or an amino acid sequence at least 95% identical thereto, and a heavy chain variable domain (V_{H}) comprising SEQ ID NO:191 or an amino acid sequence at least 95% identical thereto;
   a V_{L} comprising SEQ ID NO:192 or an amino acid sequence at least 95% identical thereto, and a V_{H} comprising SEQ ID NO:193 or an amino acid sequence at least 95% identical thereto; or
   a V_{L} comprising SEQ ID NO:194 or an amino acid sequence at least 95% identical thereto, and a V_{H} comprising SEQ ID NO:195 or an amino acid sequence at least 95% identical thereto.
9. An anti-IL-2 antigen-binding fragment, comprising an amino acid sequence selected from SEQ ID NOs:34, 35, 36, and 250-258, or an amino acid sequence at least 90% identical thereto.
10. The anti-IL-2 antibody or antigen-binding fragment of clause 7, 8, or 9, wherein the antibody or antigen-binding fragment, when bound to an IL-2 polypeptide, reduces the IL-2 polypeptide's binding to IL-2Rβ (CD122) or to the complex of IL-2β and IL-2Rγ (CD132).
11. The anti-IL-2 antibody or antigen-binding fragment of clause 9 or 10, wherein the antibody or antigen-binding fragment, when in a complex with a human IL-2 polypeptide, enhances the IL-2 polypeptide's thermal stability, optionally wherein
   the complex has higher thermal stability or Tagg temperature than a complex formed by said IL-2 polypeptide and an IL-2RB; and/or
   the IL-2 polypeptide in the complex has increased Tagg temperature.
12. The anti-IL-2 antibody or antigen-binding fragment of clause 11, wherein said Tagg is increased by more than 2°C, more than 5°C, approximately 10 °C, or more than 10°C.
13. An anti-IL-2 antibody or an antigen-binding fragment thereof that competes for binding to human IL-2 with, or binds to the same epitope as, the antibody or antigen-binding fragment of any one of clauses 7-12.
14. A prodrug comprising an IL-2 cytokine moiety, a masking moiety, and optionally a carrier moiety, wherein
   the masking moiety comprises the antibody or antigen-binding fragment of any one of clauses 7-13, and
   the cytokine moiety comprises SEQ ID NO:1, or an amino acid at least 90% identical thereto.
15. The prodrug of clause 14, wherein the cytokine moiety comprises the mutant IL-2 polypeptide of any one of clauses 1-6.
16. The prodrug of clause 14, wherein the IL-2 cytokine moiety comprises an amino acid sequence selected from SEQ ID NOs:1-33.
17. The prodrug of any one of clauses 14-16, wherein the masking moiety is an antibody comprising a V_{H} of SEQ ID NO:191 or an amino acid sequence at least 95% identical thereto, and a V_{L} of SEQ ID NO:190 or an amino acid sequence at least 95% identical thereto.
18. The prodrug of clause 17, wherein the IL-2 cytokine moiety comprises SEQ ID NO:1, optionally with one or more mutations selected from T3A, L36I, V69A, Q74P, and C125A.
19. A prodrug comprising an IL-2 cytokine moiety, a masking moiety, and optionally a carrier moiety, wherein
   the masking moiety binds to the cytokine moiety and inhibits a biological activity of the cytokine moiety, and
   the IL-2 cytokine moiety comprises the mutant IL-2 polypeptide of any one of clauses 1-6.
20. The prodrug of clause 19, wherein the masking moiety comprises an extracellular domain (ECD) of IL-2Rβ or a functional fragment thereof, or
   a single chain variable fragment (scFv) or a Fab.
21. The prodrug of clause 20, wherein the masking moiety comprises an IL-2Rβ ECD comprising SEQ ID NO:37 or an amino acid sequence at least 90% identical thereto.
22. The prodrug of any one of clauses 14, 15, and 17-20, where the mutant IL-2 polypeptide comprises
   an L36I mutation,
   optionally a C125A mutation, and
   optionally wherein the masking moiety comprises SEQ ID NO:34 or an amino acid sequence at least 90% identical thereto.
23. The prodrug of any one of clauses 19-22, wherein the masking moiety comprises the anti-IL-2 antibody or antigen-binding fragment of any one of clauses 7-13.
24. The prodrug of any one of clauses 19-23, wherein the prodrug comprises a carrier moiety selected from an antigen-binding moiety, an Fc domain, albumin or a fragment thereof, and PEG.
25. The prodrug of clause 24, wherein the carrier moiety comprises an antigen-binding moiety that targets an antigen presented on an immune or a cancer cell, optionally wherein the antigen-binding moiety is a bispecific antibody, a single domain antibody, a Fab, or an scFv.
26. The prodrug of clause 25, wherein the antigen-binding moiety targets an antigen presented on a T cell, an NK cell, a macrophage, or a cell in a tumor microenvironment (TME), optionally wherein the antigen is selected from PD-1, CD3, CD4, CD8, Tim-3, LAG-3, TIGIT, HER2, signal-regulatory protein alpha (SIRPα), CTLA-4, CSF1R, NKG2A, NKG2D, CD16A, NKp30, NKp46, ILT2, ILT4, CD40, CD163, LRRC15, fibroblast activation protein (FAP), an A1 domain of tenascin C (TNC A1), an A2 domain of tenascin C (TNC A2), an extra domain B of fibronectin (EDB), fibronectin (α5β1), vitronectin (αvβ3 and αvβ5 integrins), carcinoembryonic antigen (CEA), prostate specific antigen (PSA), 5T4, BCMA, PD-L1, CD47, epidermal growth factor receptor (EGFR), c-MET, Claudin 18.2, Claudin 6, CD20, CD24, CD38, CD47, GPC3, mesothelin, ROR1, and melanoma-associated chondroitin sulfate proteoglycan (MCSP).
27. The prodrug of clause 25, wherein the antigen-binding moiety comprises an anti-PD-1 antibody or an antigen-binding fragment thereof, optionally selected from nivolumab and pembrolizumab.
28. The prodrug of clause 25, wherein the antigen-binding moiety comprises an anti-CD8 antibody or an antigen-binding fragment thereof, optionally wherein the antigen-binding moiety comprises
   OKT8 or humanized OKT8,
   the heavy and light chain CDR1-3 derived from OKT8, and/or
   a light chain variable domain comprising SEQ ID NO:52 or an amino acid sequence at least 90% identical thereto, and a heavy chain variable domain comprising SEQ ID NO:53 or 54 or an amino acid sequence at least 90% identical thereto.
29. The prodrug of any one of clause 24-28, wherein the carrier moiety comprises an IgG Fc domain or an IgG antibody comprising
   L234A and L235A ("LALA") mutations (Eu numbering), and/or
   knobs-into-holes mutations wherein the IL-2 cytokine moiety and the masking moiety are fused to different polypeptide chains of the Fc domain, to the different heavy chains of the IgG antibody, or to the light chain and the heavy chain, respectively, of the IgG antibody.
30. The prodrug of any one of clauses 14-29, wherein the prodrug comprises one or more cleavable and/or non-cleavable peptide linkers.
31. The prodrug of any one of clauses 14-30, wherein the masking moiety is fused to the carrier moiety through a cleavable or non-cleavable peptide linker, optionally through a cleavable peptide linker.
32. The prodrug of any one of clauses 14-31, wherein the cytokine moiety is fused to the carrier moiety through a cleavable or non-cleavable peptide linker, or to the masking moiety through a cleavable peptide linker.
33. The prodrug of any one of clauses 30-32, wherein the cleavable peptide linker is cleavable by one or more proteases located in a tumor microenvironment (TME), and the cleavage leads to activation of the prodrug in the TME, optionally wherein the cleavable peptide linker comprises
   a substrate sequence of urokinase-type plasminogen activator (uPA), matrix, metallopeptidase 2 (MMP2), MMP7, MMP9, MMP14, legumain, or matriptase, and/or
   substrate sequences of two, three, four, or more proteases that are preferentially expressed in the TME.
34. The prodrug of any one of clauses 30-33, wherein the cleavable peptide linker comprises an amino acid sequence selected from SEQ ID NOs:55-124, 268, and 269.
35. A prodrug comprising the mutant IL-2 polypeptide of clause 1, wherein the prodrug comprises a first heavy chain polypeptide chain, a second heavy chain polypeptide chain, and one or two light chains, wherein:
   a. the first heavy chain polypeptide chain comprises SEQ ID NO: 275 or an amino acid sequence at least 95% identical thereto, the second heavy chain polypeptide chain comprises SEQ ID NO: 277 or an amino acid sequence at least 95% identical thereto, and two identical light chains comprise SEQ ID NO: 276 or an amino acid sequence at least 95% identical thereto;
   b. the first heavy chain polypeptide chain comprises SEQ ID NO: 278 or an amino acid sequence at least 95% identical thereto, the second heavy chain polypeptide chain comprises SEQ ID NO: 279 or an amino acid sequence at least 95% identical thereto, and two identical light chains comprise SEQ ID NO: 189 or an amino acid sequence at least 95% identical thereto;
   c. the first and second heavy chain polypeptide chains comprise SEQ ID NO: 286 or an amino acid sequence at least 95% identical thereto, and two identical light chains comprise SEQ ID NO: 276 or an amino acid sequence at least 95% identical thereto;
   d. the first and second heavy chain polypeptide chains comprise SEQ ID NO: 187 or an amino acid sequence at least 95% identical thereto, and two identical light chains comprise SEQ ID NO: 189 or an amino acid sequence at least 95% identical thereto; or
   e. the first heavy chain polypeptide chain comprises SEQ ID NO: 283 or an amino acid sequence at least 95% identical thereto, the second heavy chain polypeptide chain comprises SEQ ID NO: 284 or an amino acid sequence at least 95% identical thereto, and one light chain comprising SEQ ID NO: 189 or an amino acid sequence at least 95% identical thereto.
36. An IL-2 antibody fusion molecule comprising two identical antibody light chains, a first antibody heavy chain, and a second antibody heavy chain, wherein
   a. the light chains each comprise SEQ ID NO:38 or an amino acid sequence at least 90% identical thereto, the first heavy chain comprises SEQ ID NO:40, 41, 45, 46, or an amino acid sequence at least 95% identical thereto, and the second heavy chain comprises SEQ ID NO:42, 43, 44, or 47 or an amino acid sequence at least 95% identical thereto; or
   b. the light chains each comprise SEQ ID NO:38 or an amino acid sequence at least 90% identical thereto, and the first and second heavy chains each comprise SEQ ID NO:40, 41, 48, 49, 50, or 51 or an amino acid sequence at least 95% identical thereto;
   c. the light chains each comprise SEQ ID NO:189 or an amino acid sequence at least 90% identical thereto, the first heavy chain comprises SEQ ID NO:185 or an amino acid sequence at least 95% identical thereto, and the second heavy chain comprises SEQ ID NO:186 or an amino acid sequence at least 95% identical thereto;
37. An IL-2 antibody fusion molecule comprising:
   a. two identical light chains, and two identical heavy chains, wherein the light chains and heavy chains respectively comprise
      (i) SEQ ID NO:207 or an amino acid sequence at least 95% identical thereto, and SEQ ID NO:205, 206, 211, 212, 213, or 214 or an amino acid sequence at least 95% identical thereto, or
      (ii) SEQ ID NO:208 or an amino acid sequence at least 95% identical thereto, and SEQ ID NO:215 or an amino acid sequence at least 95% identical thereto; or
   b. a first polypeptide chain and a second polypeptide chain, wherein the first and second polypeptide chains respectively comprise
      (i) SEQ ID NO:196 or 197 or an amino acid sequence at least 95% identical thereto, and SEQ ID NO:209 or 210 or an amino acid sequence at least 95% identical thereto, or
      (ii) SEQ ID NO:198 or an amino acid sequence at least 95% identical thereto, and SEQ ID NO:199, 202, 203 or 204 or an amino acid sequence at least 95% identical thereto.
38. A pharmaceutical composition comprising the mutant human IL-2 polypeptide of any one of clauses 1-6, the prodrug of any one of clauses 14-35, or the IL-2 antibody fusion molecule of clauses 36 or 37, and a pharmaceutically acceptable excipient.
39. One or more polynucleotides encoding the mutant human IL-2 polypeptide of any one of clauses 1-6, the anti-IL-2 antibody or antigen-binding fragment of any one of clauses 7-13, the prodrug of any one of clauses 14-35, or the IL-2 antibody fusion molecule of clause 36 or 37.
40. One or more expression vectors comprising the polynucleotide(s) of clause 39.
41. A host cell comprising the expression vector(s) of clause 40, optionally wherein the host cell is a mammalian cell and the gene(s) encoding matriptase, uPA, MMP-2, MMP-9, and/or MMP14 are knocked out in the host cell.
42. A method of making a protein, comprising:
   culturing the host cell of clause 41 under conditions that allow expression of the mutant IL-2 polypeptide, an anti-IL-2 antibody or antigen-binding fragment thereof, the prodrug, or the IL-2 antibody fusion molecule, wherein the host cell is a mammalian cell, and
   isolating the expressed protein from the culture.
43. A method of treating a cancer or an infectious disease or modulating the immune system in a patient in need thereof, comprising administering to the patient a therapeutically effective amount of the mutant IL-2 polypeptide of any one of clauses 1-6, the prodrug of any one of clauses 14-35, the IL-2 antibody fusion molecule of clause 36 or 37, or the pharmaceutical composition of clause 38.
44. The mutant IL-2 polypeptide of any one of clause 1-6, the prodrug of any one of clauses 14-35, the IL-2 antibody fusion molecule of clause 36 or 37, or the pharmaceutical composition of clause 38 for use in treating a cancer or an infectious disease or modulating the immune system in a patient in need thereof.
45. Use of the mutant IL-2 polypeptide of any one of clauses 1-6, the prodrug of any one of clauses 14-35, or the IL-2 antibody fusion molecule of clause 36 or 37 for the manufacture of a medicament for treating a cancer or an infectious disease or modulating the immune system in a patient in need thereof.
46. The method of clause 42, the mutant IL-2 polypeptide, prodrug, IL-2 antibody fusion molecule, or pharmaceutical composition for use of clause 44, or the use of clause 45, wherein the patient
   has HIV infection,
   has a cancer selected from the group consisting of leukemia, lymphoma, kidney cancer, bladder cancer, urinary tract cancer, cervical cancer, brain cancer, head and neck cancer, skin cancer, uterine cancer, testicular cancer, esophageal cancer, liver cancer, colorectal cancer, stomach cancer, squamous cell carcinoma, prostate cancer, pancreatic cancer, lung cancer such as non-small cell lung cancer, cholangiocarcinoma, breast cancer, and ovarian cancer, and medullary thyroid cancer, or
   an inflammatory or an autoimmune disease, optionally selected from asthma, Type I diabetes, rheumatoid arthritis, allergy, systemic lupus erythematosus, organ graft rejection, and graft-versus-host disease.

## Claims

1. An anti-IL-2 antibody or an antigen-binding fragment thereof, comprising a heavy chain variable region (V_{H}) comprising HCDR1, HCDR2, and HCDR3, and a light chain variable region (V_{L}) comprising LCDR1, LCDR2, and LCDR3, wherein
i) the HCDR1, HCDR2, HCDR3, LCDR1, LCDR2, and LCDR3 comprise SEQ ID NOs: 300, 301, 302, 303, 304, and 305, respectively, optionally wherein the V_{H} comprises an amino acid sequence that is at least 95% identical to SEQ ID NO: 193, and the V_{L} comprises an amino acid sequence that is at least 95% identical to SEQ ID NO: 192;
ii) the HCDR1, HCDR2, HCDR3, LCDR1, LCDR2, and LCDR3 comprise SEQ ID NOs: 170, 171, 172, 173, 174, and 175, respectively, optionally wherein the V_{H} comprises an amino acid sequence that is at least 95% identical to SEQ ID NO: 191, and the V_{L} comprises an amino acid sequence that is at least 95% identical to SEQ ID NO: 190,
iii) the HCDR1, HCDR2, HCDR3, LCDR1, LCDR2, and LCDR3 comprise SEQ ID NOs: 306, 307, 308, 309, 310, or 311, respectively, optionally wherein the V_{H} comprises an amino acid sequence that is at least about 90% identical to SEQ ID NO: 313, and the V_{L} comprises an amino acid sequence that is at least about 90% identical to SEQ ID NO: 312; or
iv) the HCDR1, HCDR2, HCDR3, LCDR1, LCDR2, and LCDR3 comprise SEQ ID NOs: 314, 315, 316, 317, 318, or 319, respectively, optionally wherein the V_{H} comprises an amino acid sequence that is at least about 90% identical to SEQ ID NO: 321, and the V_{L} comprises an amino acid sequence that is at least about 90% identical to SEQ ID NO: 320.

2. The anti-IL-2 antibody or the antigen-binding fragment of claim 1, wherein the V_{H} and V_{L} comprise:
SEQ ID NOs: 193 and 192, respectively;
SEQ ID NOs: 191 and 190, respectively;
SEQ ID NOs: 313 and 312, respectively; or
SEQ ID NOs: 321 and 320, respectively.

3. The anti-IL-2 antibody or the antigen-binding fragment of claim 1 or 2, wherein the V_{H} and V_{L} comprise SEQ ID NOs: 193 and 192, respectively.

4. The antigen-binding fragment of any one of claims 1-3, which is a single chain variable fragment (scFv).

5. The antigen-binding fragment of claim 4, wherein the scFv comprises SEQ ID NO: 35, 34, 36, and 257.

6. The antigen-binding fragment of claim 4 or 5, wherein the scFv comprises SEQ ID NO: 35.

7. One or more polynucleotides encoding the anti-IL-2 antibody or the antigen-binding fragment of any one of claims 1-6.

8. One or more expression vectors comprising the polynucleotide(s) of claim 7.

9. A host cell comprising the polynucleotides(s) of claim 7 or the expression vector(s) of claim 8, optionally wherein the host cell is a mammalian cell and the gene(s) encoding matriptase, uPA, MMP-2, MMP-9, and/or MMP14 are knocked out in the host cell.

10. The anti-IL-2 antibody or the antigen-binding fragment of any one of claims 1-6 for use as a medicament.

11. The anti-IL-2 antibody or the antigen-binding fragment of any one of claims 1-6 for use in inhibiting the biological activity of IL-2.
